# EUROPEAN PATENT APPLICATION

(11) **EP 3 889 175 A1**
(43) Date of publication of application: **06.10.2021**
(21) Application number: 21153405.2
(22) Date of filing: 02.05.2017
(51) Int. Cl.: C07K 16/00, C07K 16/32

(54) **THE CONTORSBODY - A SINGLE CHAIN TARGET BINDER**

(30) Priority: 02.05.2016 EP 16167920
(62) Divisional of application: 17723041.4
(71) Applicant: F. Hoffmann-La Roche AG, 4070 Basel (CH)
(72) Inventor: DENGL, Stefan, 80333 München (DE); GEORGES, Guy, 82392 Habach (DE); HESSE, Friederike, 80339 München (DE); IMHOF-JUNG, Sabine, 82152 Planegg (DE); PLATZER, Josef, 82538 Geretsried (DE)
(74) Representative: Burger, Alexander

(57) **Abstract**

Herein is reported a circular fusion polypeptide comprising a first part of a binding domain, a second part of a binding domain and a spacer domain, wherein the spacer domain is a polypeptide and comprises at least 25 amino acid residues, the first part of the binding domain is a polypeptide and is fused via a first linker to the N-terminus of the spacer domain, the second part of the binding domain is a polypeptide and is fused via a second linker to the C-terminus of the spacer domain, the first part of the binding domain and the second part of the binding domain are associated with each other and form a binding site that specifically binds to a target.

SEQUENCE LISTING

<110>  F. Hoffmann-La Roche AG

<120>  The Contorsbody - a single chain target binder

<130>  P33504-EP-3

<150>  EP16167920
<151>  2016-05-02

<160>  105

<170>  PatentIn version 3.5

<210>  1
<211>  5
<212>  PRT
<213>  Artificial Sequence

<220>
<223>  Arg-tag

<400>  1

Arg Arg Arg Arg Arg
1               5


<210>  2
<211>  6
<212>  PRT
<213>  Artificial Sequence

<220>
<223>  Arg-tag 2

<400>  2

Arg Arg Arg Arg Arg Arg
1               5


<210>  3
<211>  6
<212>  PRT
<213>  Artificial Sequence

<220>
<223>  His-tag

<400>  3

His His His His His His
1               5


<210>  4
<211>  19
<212>  PRT
<213>  Artificial Sequence

<220>

<223>  amino acid tag

<400>  4

Lys Asp His Leu Ile His Asn Val His Lys Glu Phe His Ala His Ala
1               5                   10                  15

His Asn Lys

<210>  5
<211>  8
<212>  PRT
<213>  Artificial Sequence

<220>
<223>  amino acid tag

<400>  5

Asp Tyr Lys Asp Asp Asp Asp Lys
1               5

<210>  6
<211>  22
<212>  PRT
<213>  Artificial Sequence

<220>
<223>  amino acid tag

<400>  6

Asp Tyr Lys Asp His Asp Gly Asp Tyr Lys Asp His Asp Ile Asp Tyr
1               5                   10                  15

Lys Asp Asp Asp Asp Lys
            20

<210>  7
<211>  9
<212>  PRT
<213>  Artificial Sequence

<220>
<223>  amino acid tag

<400>  7

Ala Trp Arg His Pro Gln Phe Gly Gly
1               5

<210>  8
<211>  8
<212>  PRT
<213>  Artificial Sequence

<220>
<223>  amino acid tag

<400>  8

Trp Ser His Pro Gln Phe Glu Lys
1               5


<210>  9
<211>  10
<212>  PRT
<213>  Artificial Sequence

<220>
<223>  amino acid tag

<400>  9

Met Asp Val Glu Ala Trp Leu Gly Ala Arg
1               5                   10


<210>  10
<211>  16
<212>  PRT
<213>  Artificial Sequence

<220>
<223>  amino acid tag

<400>  10

Met Asp Val Glu Ala Trp Leu Gly Ala Arg Val Pro Leu Val Glu Thr
1               5                   10                  15


<210>  11
<211>  38
<212>  PRT
<213>  Artificial Sequence

<220>
<223>  amino acid tag

<400>  11

Met Asp Glu Lys Thr Thr Gly Trp Arg Gly Gly His Val Val Glu Gly
1               5                   10                  15


Leu Ala Gly Glu Leu Glu Gln Leu Arg Ala Arg Leu Glu His His Pro
            20                  25                  30


Gln Gly Gln Arg Glu Pro
        35


<210>  12
<211>  10
<212>  PRT
<213>  Artificial Sequence

<220>
<223>  amino acid tag

<400>  12

Glu Gln Lys Leu Ile Ser Glu Glu Asp Leu
1               5                   10


<210>  13
<211>  15
<212>  PRT
<213>  Artificial Sequence

<220>
<223>  amino acid tag

<400>  13

Lys Glu Thr Ala Ala Ala Lys Phe Glu Arg Gln His Met Asp Ser
1               5                   10                  15


<210>  14
<211>  26
<212>  PRT
<213>  Artificial Sequence

<220>
<223>  amino acid tag

<400>  14

Lys Arg Arg Trp Lys Lys Asn Phe Ile Ala Val Ser Ala Ala Asn Arg
1               5                   10                  15


Phe Lys Lys Ile Ser Ser Ser Gly Ala Leu
            20                  25


<210>  15
<211>  47
<212>  PRT
<213>  Artificial Sequence

<220>
<223>  amino acid tag

<400>  15

Pro Ala Thr Thr Thr Gly Ser Ser Pro Gly Pro Thr Gln Ser His Tyr
1               5                   10                  15


Gly Gln Cys Gly Gly Ile Gly Tyr Ser Gly Pro Thr Val Cys Ala Ser
            20                  25                  30


Gly Thr Thr Cys Gln Val Leu Asn Pro Tyr Tyr Ser Gln Cys Leu
        35                  40                  45

<210>  16
<211>  32
<212>  PRT
<213>  Butyrivibrio fibrisolvens

<400>  16

Met Asp Trp Asn Ala Asn Ile Ala Pro Gly Asn Ser Val Glu Phe Gly
1               5                   10                  15

Ile Gln Gly Ala Gly Ser Val Gly Asn Val Ile Asp Ile Thr Val Glu
            20                  25                  30

<210>  17
<211>  51
<212>  PRT
<213>  Artificial Sequence

<220>
<223>  chitin-binding-domain

<400>  17

Thr Asn Pro Gly Val Ser Ala Trp Gln Val Asn Thr Ala Tyr Thr Ala
1               5                   10                  15

Gly Gln Leu Val Thr Tyr Asn Gly Lys Thr Tyr Lys Cys Leu Gln Pro
            20                  25                  30

His Thr Ser Leu Ala Gly Trp Glu Pro Ser Asn Val Pro Ala Leu Trp
        35                  40                  45

Gln Leu Gln
    50

<210>  18
<211>  209
<212>  PRT
<213>  Chondrus crispus

<400>  18

Met Pro Glu Ile Lys Leu Thr Tyr Phe Asp Met Arg Gly Arg Ala Glu
1               5                   10                  15

Ala Ser Arg Leu Ala Leu Val Val Gly Glu Ile Pro Phe Glu Asp Glu
            20                  25                  30

Arg Val Val Phe Asp His Trp Lys Glu Ala Lys Pro Lys Thr Pro Tyr
        35                  40                  45

Ala Ala Leu Pro Met Leu Thr Val Asp Gly Met Gln Val Ala Gln Ser
    50                  55                  60

Asp Ala Ile Leu Arg Tyr Cys Gly Lys Leu Ala Gly Leu Tyr Pro Ser
65                  70                  75                  80

Asp Pro Leu Glu Ala Ala Lys Val Asp Glu Val Gly Gly Val Ile Asp
                85                  90                  95

Asp Val Thr His Ala Met Tyr Arg Tyr Arg Gly Asp Asp Lys Asp Lys
            100                 105                 110

Leu Arg Glu Glu Arg Asp Lys Phe Ser Lys Val Asp Val Pro Arg Tyr
        115                 120                 125

Val Gly Ala Leu Glu Lys Arg Leu Glu Ala Phe Gly Asp Gly Pro Trp
    130                 135                 140

Ala Val Gly Gly Asn Met Thr Ile Ala Asp Leu His Ile Cys His Leu
145                 150                 155                 160

Val Thr Asn Ile Arg Cys Gly Met Leu Asp Phe Val Asp Lys Asp Leu
                165                 170                 175

Leu Glu Gly Tyr Val Arg Ile Val Lys Ser Tyr Ser Ala Val Met Glu
            180                 185                 190

His Pro Lys Val Thr Glu Trp Tyr Glu Lys Lys Pro Val Lys Met Phe
        195                 200                 205

Ser

<210>  19
<211>  396
<212>  PRT
<213>  Escherichia coli

<400>  19

Met Lys Ile Lys Thr Gly Ala Arg Ile Leu Ala Leu Ser Ala Leu Thr
1               5                   10                  15

Thr Met Met Phe Ser Ala Ser Ala Leu Ala Lys Ile Glu Glu Gly Lys
            20                  25                  30

Leu Val Ile Trp Ile Asn Gly Asp Lys Gly Tyr Asn Gly Leu Ala Glu
        35                  40                  45

Val Gly Lys Lys Phe Glu Lys Asp Thr Gly Ile Lys Val Thr Val Glu
    50                  55                  60

His Pro Asp Lys Leu Glu Glu Lys Phe Pro Gln Val Ala Ala Thr Gly
65                  70                  75                  80

Asp Gly Pro Asp Ile Ile Phe Trp Ala His Asp Arg Phe Gly Gly Tyr
                85                  90                  95

Ala Gln Ser Gly Leu Leu Ala Glu Ile Thr Pro Asp Lys Ala Phe Gln
            100                 105                 110

Asp Lys Leu Tyr Pro Phe Thr Trp Asp Ala Val Arg Tyr Asn Gly Lys
        115                 120                 125

Leu Ile Ala Tyr Pro Ile Ala Val Glu Ala Leu Ser Leu Ile Tyr Asn
    130                 135                 140

Lys Asp Leu Leu Pro Asn Pro Pro Lys Thr Trp Glu Glu Ile Pro Ala
145                 150                 155                 160

Leu Asp Lys Glu Leu Lys Ala Lys Gly Lys Ser Ala Leu Met Phe Asn
                165                 170                 175

Leu Gln Glu Pro Tyr Phe Thr Trp Pro Leu Ile Ala Ala Asp Gly Gly
            180                 185                 190

Tyr Ala Phe Lys Tyr Glu Asn Gly Lys Tyr Asp Ile Lys Asp Val Gly
        195                 200                 205

Val Asp Asn Ala Gly Ala Lys Ala Gly Leu Thr Phe Leu Val Asp Leu
    210                 215                 220

Ile Lys Asn Lys His Met Asn Ala Asp Thr Asp Tyr Ser Ile Ala Glu
225                 230                 235                 240

Ala Ala Phe Asn Lys Gly Glu Thr Ala Met Thr Ile Asn Gly Pro Trp
                245                 250                 255

Ala Trp Ser Asn Ile Asp Thr Ser Lys Val Asn Tyr Gly Val Thr Val
            260                 265                 270

Leu Pro Thr Phe Lys Gly Gln Pro Ser Lys Pro Phe Val Gly Val Leu
        275                 280                 285

Ser Ala Gly Ile Asn Ala Ala Ser Pro Asn Lys Glu Leu Ala Lys Glu
    290                 295                 300

Phe Leu Glu Asn Tyr Leu Leu Thr Asp Glu Gly Leu Glu Ala Val Asn

305                 310                 315                 320

Lys Asp Lys Pro Leu Gly Ala Val Ala Leu Lys Ser Tyr Glu Glu Glu
                325                 330                 335

Leu Ala Lys Asp Pro Arg Ile Ala Ala Thr Met Glu Asn Ala Gln Lys
            340                 345                 350

Gly Glu Ile Met Pro Asn Ile Pro Gln Met Ser Ala Phe Trp Tyr Ala
        355                 360                 365

Val Arg Thr Ala Val Ile Asn Ala Ala Ser Gly Arg Gln Thr Val Asp
    370                 375                 380

Glu Ala Leu Lys Asp Ala Gln Thr Arg Ile Thr Lys
385                 390                 395

<210>  20
<211>  98
<212>  PRT
<213>  Homo sapiens

<400>  20

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val

<210>  21
<211>  107
<212>  PRT
<213>  Homo sapiens

<400>  21

Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Trp Asp Val Ser His Glu Asp Pro Glu Val Lys Phe Asn Trp Tyr Val
        35                  40                  45

Asp Gly Val Glu Val His Asn Ala Lys Thr Lys Pro Arg Glu Glu Gln
    50                  55                  60

Glu Ser Thr Tyr Arg Trp Ser Val Leu Thr Val Leu His Gln Asp Trp
65                  70                  75                  80

Leu Asn Gly Lys Glu Tyr Lys Cys Lys Val Ser Asn Lys Ala Leu Pro
                85                  90                  95

Ala Pro Ile Glu Lys Thr Ile Ser Lys Ala Lys
            100                 105


<210>  22
<211>  106
<212>  PRT
<213>  Homo sapiens

<400>  22

<210>  23
<211>  10
<212>  PRT
<213>  Homo sapiens

<220>
<221>  MISC_FEATURE
<222>  (8)..(8)
<223>  X=S or P

<400>  23

Asp Lys Thr His Thr Cys Pro Xaa Cys Pro
1               5                   10

<210>  24
<211>  7
<212>  PRT
<213>  Homo sapiens

<220>
<221>  MISC_FEATURE
<222>  (5)..(5)
<223>  X=S or P

<400>  24

His Thr Cys Pro Xaa Cys Pro
1               5

<210>  25
<211>  5
<212>  PRT
<213>  Homo sapiens

<220>
<221>  MISC_FEATURE
<222>  (3)..(3)
<223>  X=S or P

<400>  25

Cys Pro Xaa Cys Pro
1               5

<210>  26
<211>  330
<212>  PRT
<213>  Homo sapiens

<400>  26

<210>  27
<211>  330
<212>  PRT
<213>  Homo sapiens

<400>  27

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  28
<211>  326
<212>  PRT
<213>  Homo sapiens

<400>  28

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ser Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys

290                 295                 300

Ser Val Met His Glu Ala Leu His Asn His Tyr Thr Gln Lys Ser Leu
305                 310                 315                 320

Ser Leu Ser Pro Gly Lys
                325

<210>  29
<211>  377
<212>  PRT
<213>  Homo sapiens

<400>  29

Ala Ser Thr Lys Gly Pro Ser Val Phe Pro Leu Ala Pro Cys Ser Arg
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Thr Cys Asn Val Asn His Lys Pro Ser Asn Thr Lys Val Asp Lys
                85                  90                  95

Arg Val Glu Leu Lys Thr Pro Leu Gly Asp Thr Thr His Thr Cys Pro
            100                 105                 110

Arg Cys Pro Glu Pro Lys Ser Cys Asp Thr Pro Pro Pro Cys Pro Arg
        115                 120                 125

Cys Pro Glu Pro Lys Ser Cys Asp Thr Pro Pro Pro Cys Pro Arg Cys
    130                 135                 140

Pro Glu Pro Lys Ser Cys Asp Thr Pro Pro Pro Cys Pro Arg Cys Pro
145                 150                 155                 160

Ala Pro Glu Leu Leu Gly Gly Pro Ser Val Phe Leu Phe Pro Pro Lys
                165                 170                 175

Pro Lys Asp Thr Leu Met Ile Ser Arg Thr Pro Glu Val Thr Cys Val

180                 185                 190

Val Val Asp Val Ser His Glu Asp Pro Glu Val Gln Phe Lys Trp Tyr
        195                 200                 205

Val Asp Gly Val Glu Val His Asn Ala Lys Thr Lys Pro Arg Glu Glu
    210                 215                 220

Gln Tyr Asn Ser Thr Phe Arg Val Val Ser Val Leu Thr Val Leu His
225                 230                 235                 240

Gln Asp Trp Leu Asn Gly Lys Glu Tyr Lys Cys Lys Val Ser Asn Lys
                245                 250                 255

Ala Leu Pro Ala Pro Ile Glu Lys Thr Ile Ser Lys Thr Lys Gly Gln
            260                 265                 270

Pro Arg Glu Pro Gln Val Tyr Thr Leu Pro Pro Ser Arg Glu Glu Met
        275                 280                 285

Thr Lys Asn Gln Val Ser Leu Thr Cys Leu Val Lys Gly Phe Tyr Pro
    290                 295                 300

Ser Asp Ile Ala Val Glu Trp Glu Ser Ser Gly Gln Pro Glu Asn Asn
305                 310                 315                 320

Tyr Asn Thr Thr Pro Pro Met Leu Asp Ser Asp Gly Ser Phe Phe Leu
                325                 330                 335

Tyr Ser Lys Leu Thr Val Asp Lys Ser Arg Trp Gln Gln Gly Asn Ile
            340                 345                 350

Phe Ser Cys Ser Val Met His Glu Ala Leu His Asn Arg Phe Thr Gln
        355                 360                 365

Lys Ser Leu Ser Leu Ser Pro Gly Lys
    370                 375

<210>  30
<211>  327
<212>  PRT
<213>  Homo sapiens

<400>  30

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr

20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  31
<211>  221
<212>  PRT
<213>  Homo sapiens

<220>
<221>  MISC_FEATURE
<222>  (131)..(131)
<223>  X=E or D

<220>
<221>  MISC_FEATURE
<222>  (133)..(133)
<223>  X=M or L

<400>  31

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro

115                 120                 125

Ser Arg Xaa Glu Xaa Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly
    210                 215                 220

<210>  32
<211>  226
<212>  PRT
<213>  Artificial Sequence

<220>
<223>  variant human Fc-region of the IgG1 isotype with a hole mutation

<400>  32

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  33
<211>  226
<212>  PRT
<213>  Artificial Sequence

<220>
<223>  variant human Fc-region of the IgG1 isotype with a knob mutation

<400>  33

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  34
<211>  226
<212>  PRT
<213>  Artificial Sequence

<220>
<223>  variant human Fc-region of the IgG1 isotype with the mutations
       L234A, L235A

<400>  34

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  35
<211>  226
<212>  PRT
<213>  Artificial Sequence

<220>
<223>  variant human Fc-region of the IgG1 isotype with a L234A, L235A
       and hole mutation

<400>  35

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  36
<211>  226
<212>  PRT
<213>  Artificial Sequence

<220>

<223>  variant human Fc-region of the IgG1 isotype with a L234A, L235A
       and knob mutation

<400>  36

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  37
<211>  226
<212>  PRT
<213>  Artificial Sequence

<220>
<223>  variant human Fc-region of the IgG1 isotype with a L234A, L235A
       and P329G mutation

<400>  37

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Thr Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  38
<211>  226
<212>  PRT
<213>  Artificial Sequence

<220>
<223>  variant human Fc-region of the IgG1 isotype with a L234A, L235A,
       P329G and hole mutation

<400>  38

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Ser Cys Ala Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Val Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210> 39
<211> 226
<212> PRT
<213> Artificial Sequence

<220>
<223> variant human Fc-region of the IgG1 isotype with a L234A, L235A,
P329G and knob mutation

<400> 39

Asp Lys Thr His Thr Cys Pro Pro Cys Pro Ala Pro Glu Ala Ala Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Gly Ala Pro Ile
            100                 105                 110

Glu Lys Thr Ile Ser Lys Ala Lys Gly Gln Pro Arg Glu Pro Gln Val
        115                 120                 125

Tyr Thr Leu Pro Pro Ser Arg Asp Glu Leu Thr Lys Asn Gln Val Ser
    130                 135                 140

Leu Trp Cys Leu Val Lys Gly Phe Tyr Pro Ser Asp Ile Ala Val Glu
145                 150                 155                 160

Trp Glu Ser Asn Gly Gln Pro Glu Asn Asn Tyr Lys Thr Thr Pro Pro
                165                 170                 175

Val Leu Asp Ser Asp Gly Ser Phe Phe Leu Tyr Ser Lys Leu Thr Val
            180                 185                 190

Asp Lys Ser Arg Trp Gln Gln Gly Asn Val Phe Ser Cys Ser Val Met
        195                 200                 205

His Glu Ala Leu His Asn His Tyr Thr Gln Lys Ser Leu Ser Leu Ser
    210                 215                 220

Pro Gly
225

<210>  40
<211>  221
<212>  PRT
<213>  Artificial Sequence

<220>
<223>  variant human Fc-region of the IgG1 subclass with the mutations
       L234A, L235A, P329G, Y349C, T366S, L368A and Y407V

<400>  40

Cys Pro Pro Cys Pro Ala Pro Glu Ala Ala Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Gly Ala Pro Ile Glu Lys Thr Ile Ser
            100                 105                 110

Lys Ala Lys Gly Gln Pro Arg Glu Pro Gln Val Cys Thr Leu Pro Pro
        115                 120                 125

Ser Arg Asp Glu Leu Thr Lys Asn Gln Val Ser Leu Ser Cys Ala Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Val Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly
    210                 215                 220

<210>  41
<211>  221
<212>  PRT
<213>  Artificial Sequence

<220>
<223>  variant human Fc-region of the IgG1 subclass with a L234A, L235A,
       P329G and S354C, T366W mutation

<400>  41

Cys Pro Pro Cys Pro Ala Pro Glu Ala Ala Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Gly Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Cys Arg Asp Glu Leu Thr Lys Asn Gln Val Ser Leu Trp Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly
    210                 215                 220

<210> 42
<211> 221
<212> PRT
<213> Artificial Sequence

<220>
<223> variant human Fc-region of the IgG1 subclass with a L234A, L235A,
P329G and S354C, T366S, L368A and Y407V mutation

<400> 42

Cys Pro Pro Cys Pro Ala Pro Glu Ala Ala Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Gly Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Cys Arg Asp Glu Leu Thr Lys Asn Gln Val Ser Leu Ser Cys Ala Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Val Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly
    210                 215                 220

<210>  43
<211>  221
<212>  PRT
<213>  Artificial Sequence

<220>
<223>  variant human Fc-region of the IgG1 subclass with a L234A, L235A,
       P329G and Y349C, T366W mutation

<400>  43

Cys Pro Pro Cys Pro Ala Pro Glu Ala Ala Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Gly Ala Pro Ile Glu Lys Thr Ile Ser
            100                 105                 110

Lys Ala Lys Gly Gln Pro Arg Glu Pro Gln Val Cys Thr Leu Pro Pro
        115                 120                 125

Ser Arg Asp Glu Leu Thr Lys Asn Gln Val Ser Leu Trp Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn His Tyr Thr Gln Lys Ser Leu Ser Leu Ser Pro Gly
    210                 215                 220

<210>  44
<211>  221
<212>  PRT
<213>  Artificial Sequence

<220>
<223>  variant human Fc-region of the IgG1 subclass with the mutations
       I253A, H310A and H435A

<400>  44

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ala Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu Ala Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu His
        195                 200                 205

Asn Ala Tyr Thr Gln Lys Ser Leu Ser Leu Ser Pro Gly
    210                 215                 220

<210>  45
<211>  221
<212>  PRT
<213>  Artificial Sequence

<220>
<223>  variant human Fc-region of the IgG1 subclass with the mutations
       H310A, H433A and Y436A

<400>  45

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Met Ile Ser Arg Thr Pro
            20                  25                  30

Glu Val Thr Cys Val Val Val Asp Val Ser His Glu Asp Pro Glu Val
        35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu Ala Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
            100                 105                 110

Lys Ala Lys Gly Gln Pro Arg Glu Pro Gln Val Tyr Thr Leu Pro Pro
        115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
            180                 185                 190

Gln Gln Gly Asn Val Phe Ser Cys Ser Val Met His Glu Ala Leu Ala
        195                 200                 205

Asn His Ala Thr Gln Lys Ser Leu Ser Leu Ser Pro Gly
    210                 215                 220

<210>  46
<211>  221
<212>  PRT
<213>  Artificial Sequence

<220>
<223>  variant human Fc-region of the IgG1 subclass with the mutations
       M252Y, S254T and T256E

<400>  46

Cys Pro Pro Cys Pro Ala Pro Glu Leu Leu Gly Gly Pro Ser Val Phe
1               5                   10                  15

Leu Phe Pro Pro Lys Pro Lys Asp Thr Leu Tyr Ile Thr Arg Glu Pro
                20                  25                  30

Glu Val Thr Cys Val Val Val Asp Val Ser His Glu Asp Pro Glu Val
            35                  40                  45

Lys Phe Asn Trp Tyr Val Asp Gly Val Glu Val His Asn Ala Lys Thr
    50                  55                  60

Lys Pro Arg Glu Glu Gln Tyr Asn Ser Thr Tyr Arg Val Val Ser Val
65                  70                  75                  80

Leu Thr Val Leu His Gln Asp Trp Leu Asn Gly Lys Glu Tyr Lys Cys
                85                  90                  95

Lys Val Ser Asn Lys Ala Leu Pro Ala Pro Ile Glu Lys Thr Ile Ser
                100                 105                 110

Lys Ala Lys Gly Gln Pro Arg Glu Pro Gln Val Tyr Thr Leu Pro Pro
            115                 120                 125

Ser Arg Asp Glu Leu Thr Lys Asn Gln Val Ser Leu Thr Cys Leu Val
    130                 135                 140

Lys Gly Phe Tyr Pro Ser Asp Ile Ala Val Glu Trp Glu Ser Asn Gly
145                 150                 155                 160

Gln Pro Glu Asn Asn Tyr Lys Thr Thr Pro Pro Val Leu Asp Ser Asp
                165                 170                 175

Gly Ser Phe Phe Leu Tyr Ser Lys Leu Thr Val Asp Lys Ser Arg Trp
                180                 185                 190

Gln Gln Gly Asn Val Phe Ser Cys Ser Val Met His Glu Ala Leu His
            195                 200                 205

Asn His Tyr Thr Gln Lys Ser Leu Ser Leu Ser Pro Gly
    210                 215                 220

<210>  47
<211>  228
<212>  PRT
<213>  Homo sapiens

<400>  47

Glu Ser Lys Tyr Gly Pro Pro Cys Pro Ser Cys Pro Ala Pro Glu Phe
1               5                   10                  15

Leu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly
225

<210> 48
<211> 228
<212> PRT
<213> Artificial Sequence

<220>
<223>  variant human Fc-region of the IgG4 isotype with a S228P and
      L235E mutation

<400>  48

Glu Ser Lys Tyr Gly Pro Pro Cys Pro Pro Cys Pro Ala Pro Glu Phe
1               5                   10                  15

Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Pro Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly

225

<210>  49
<211>  228
<212>  PRT
<213>  Artificial Sequence

<220>
<223>  variant human Fc-region of the IgG4 isotype with a S228P, L235E
       and P329G mutation

<400>  49

Glu Ser Lys Tyr Gly Pro Pro Cys Pro Pro Cys Pro Ala Pro Glu Phe
1               5                   10                  15

Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Gly Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Thr Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser

195                 200                 205

Val Met His Glu Ala Leu His Asn His Tyr Thr Gln Lys Ser Leu Ser
    210                 215                 220

Leu Ser Leu Gly
225

<210>  50
<211>  228
<212>  PRT
<213>  Artificial Sequence

<220>
<223>  variant human Fc-region of the IgG4 isotype with a S228P and
       L235E mutation

<400>  50

Glu Ser Lys Tyr Gly Pro Pro Cys Pro Pro Cys Pro Ala Pro Glu Phe
1               5                   10                  15

Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Gly Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln
    130                 135                 140

Val Ser Leu Ser Cys Ala Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr

165                 170                 175

Pro Pro Val Leu Asp Ser Asp Gly Ser Phe Phe Leu Val Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly
225

<210>  51
<211>  228
<212>  PRT
<213>  Artificial Sequence

<220>
<223>  variant human Fc-region of the IgG4 isotype with a S228P, L235E
       and P329G mutation

<400>  51

Glu Ser Lys Tyr Gly Pro Pro Cys Pro Pro Cys Pro Ala Pro Glu Phe
1               5                   10                  15

Glu Gly Gly Pro Ser Val Phe Leu Phe Pro Pro Lys Pro Lys Asp Thr
            20                  25                  30

Leu Met Ile Ser Arg Thr Pro Glu Val Thr Cys Val Val Val Asp Val
        35                  40                  45

Ser Gln Glu Asp Pro Glu Val Gln Phe Asn Trp Tyr Val Asp Gly Val
    50                  55                  60

Glu Val His Asn Ala Lys Thr Lys Pro Arg Glu Glu Gln Phe Asn Ser
65                  70                  75                  80

Thr Tyr Arg Val Val Ser Val Leu Thr Val Leu His Gln Asp Trp Leu
                85                  90                  95

Asn Gly Lys Glu Tyr Lys Cys Lys Val Ser Asn Lys Gly Leu Gly Ser
            100                 105                 110

Ser Ile Glu Lys Thr Ile Ser Lys Ala Lys Gly Gln Pro Arg Glu Pro
        115                 120                 125

Gln Val Tyr Thr Leu Pro Pro Ser Gln Glu Glu Met Thr Lys Asn Gln

130                 135                 140

Val Ser Leu Trp Cys Leu Val Lys Gly Phe Tyr Pro Ser Asp Ile Ala
145                 150                 155                 160

Val Glu Trp Glu Ser Asn Gly Gln Pro Glu Asn Asn Tyr Lys Thr Thr
                165                 170                 175

Pro Pro Val Leu Asp Ser Asp Gly Ser Phe Phe Leu Tyr Ser Arg Leu
            180                 185                 190

Thr Val Asp Lys Ser Arg Trp Gln Glu Gly Asn Val Phe Ser Cys Ser
        195                 200                 205

Val Met His Glu Ala Leu His Asn His Tyr Thr Gln Lys Ser Leu Ser
    210                 215                 220

Leu Ser Leu Gly
225

<210>  52
<211>  107
<212>  PRT
<213>  Homo sapiens

<400>  52

<210>  53

<211>  105
<212>  PRT
<213>  Homo sapiens

<400>  53

Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Lys Ala Gly Val Glu Thr Thr Thr Pro Ser Lys Gln Ser Asn Asn Lys
    50                  55                  60

Tyr Ala Ala Ser Ser Tyr Leu Ser Leu Thr Pro Glu Gln Trp Lys Ser
65                  70                  75                  80

His Arg Ser Tyr Ser Cys Gln Val Thr His Glu Gly Ser Thr Val Glu
                85                  90                  95

Lys Thr Val Ala Pro Thr Glu Cys Ser
            100                 105


<210>  54
<211>  4
<212>  PRT
<213>  Artificial Sequence

<220>
<223>  peptidic linker

<400>  54

Gly Gly Gly Ser
1


<210>  55
<211>  5
<212>  PRT
<213>  Artificial Sequence

<220>
<223>  peptidic linker

<400>  55

Gly Gly Gly Gly Ser
1               5


<210>  56

<211>  4
<212>  PRT
<213>  Artificial Sequence

<220>
<223>  peptidic linker

<400>  56

Gln Gln Gln Ser
1


<210>  57
<211>  5
<212>  PRT
<213>  Artificial Sequence

<220>
<223>  peptidic linker

<400>  57

Gln Gln Gln Gln Ser
1               5


<210>  58
<211>  4
<212>  PRT
<213>  Artificial Sequence

<220>
<223>  peptidic linker

<400>  58

Ser Ser Ser Gly
1


<210>  59
<211>  5
<212>  PRT
<213>  Artificial Sequence

<220>
<223>  peptidic linker

<400>  59

Ser Ser Ser Ser Gly
1               5


<210>  60
<211>  8
<212>  PRT
<213>  Artificial Sequence

<220>
<223>  peptidic linker

<400>  60

Gly Gly Gly Ser Gly Gly Gly Ser
1               5


<210>  61
<211>  12
<212>  PRT
<213>  Artificial Sequence

<220>
<223>  peptidic linker

<400>  61

Gly Gly Gly Ser Gly Gly Gly Ser Gly Gly Gly Ser
1               5                   10


<210>  62
<211>  16
<212>  PRT
<213>  Artificial Sequence

<220>
<223>  peptidic linker

<400>  62

Gly Gly Gly Ser Gly Gly Gly Ser Gly Gly Gly Ser Gly Gly Gly Ser
1               5                   10                  15


<210>  63
<211>  20
<212>  PRT
<213>  Artificial Sequence

<220>
<223>  peptidic linker

<400>  63

Gly Gly Gly Ser Gly Gly Gly Ser Gly Gly Gly Ser Gly Gly Gly Ser
1               5                   10                  15


Gly Gly Gly Ser
            20


<210>  64
<211>  10
<212>  PRT
<213>  Artificial Sequence

<220>
<223>  peptidic linker

<400>  64

Gly Gly Gly Gly Ser Gly Gly Gly Gly Ser

1                   5                   10


<210>  65
<211>  15
<212>  PRT
<213>  Artificial Sequence

<220>
<223>  peptidic linker

<400>  65

Gly Gly Gly Gly Ser Gly Gly Gly Gly Ser Gly Gly Gly Gly Ser
1               5                   10                  15


<210>  66
<211>  20
<212>  PRT
<213>  Artificial Sequence

<220>
<223>  peptidic linker

<400>  66

Gly Gly Gly Gly Ser Gly Gly Gly Gly Ser Gly Gly Gly Gly Ser Gly
1               5                   10                  15


Gly Gly Gly Ser
            20


<210>  67
<211>  17
<212>  PRT
<213>  Artificial Sequence

<220>
<223>  peptidic linker

<400>  67

Gly Ser Ser Ser Ser Ser Ser Ser Ser Ser Ser Ser Ser Ser Ser Ser
1               5                   10                  15


Gly



<210>  68
<211>  18
<212>  PRT
<213>  Artificial Sequence

<220>
<223>  peptidic linker

<400>  68

Gly Gly Ser Gly Gly Gly Gly Ser Gly Gly Gly Gly Ser Gly Gly Gly
1               5                   10                  15

Gly Ser

<210>  69
<211>  22
<212>  PRT
<213>  Artificial Sequence

<220>
<223>  peptidic linker

<400>  69

Gly Gly Gly Gly Ser Gly Gly Gly Gly Ser Gly Gly Gly Gly Ser Gly
1               5                   10                  15

Gly Gly Gly Ser Gly Gly
            20

<210>  70
<211>  30
<212>  PRT
<213>  Artificial Sequence

<220>
<223>  peptidic linker

<400>  70

Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser
            20                  25                  30

<210>  71
<211>  118
<212>  PRT
<213>  Mus musculus

<400>  71

Glu Val Gln Leu Val Glu Ser Gly Gly Gly Leu Val Gln Pro Gly Asn
1               5                   10                  15

Ser Leu Thr Leu Ser Cys Val Ala Ser Gly Phe Thr Phe Ser Asn Tyr
            20                  25                  30

Gly Met His Trp Ile Arg Gln Ala Pro Lys Lys Gly Leu Glu Trp Ile
        35                  40                  45

Ala Met Ile Tyr Tyr Asp Ser Ser Lys Met Asn Tyr Ala Asp Thr Val
    50                  55                  60

Lys Gly Arg Phe Thr Ile Ser Arg Asp Asn Ser Lys Asn Thr Leu Tyr
65                  70                  75                  80

Leu Glu Met Asn Ser Leu Arg Ser Glu Asp Thr Ala Met Tyr Tyr Cys
                85                  90                  95

Ala Val Pro Thr Ser His Tyr Val Val Asp Val Trp Gly Gln Gly Val
            100                 105                 110

Ser Val Thr Val Ser Ser
        115

<210>  72
<211>  107
<212>  PRT
<213>  Mus musculus

<400>  72

Asp Ile Gln Met Thr Gln Ser Pro Ala Ser Leu Ser Ala Ser Leu Glu
1               5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Ile
        35                  40                  45

Tyr Gly Ala Thr Ser Leu Ala Asp Gly Val Pro Ser Arg Phe Ser Gly
    50                  55                  60

Ser Arg Ser Gly Thr Gln Phe Ser Leu Lys Ile Ser Arg Val Gln Val
65                  70                  75                  80

Glu Asp Ile Gly Ile Tyr Tyr Cys Leu Gln Ala Tyr Asn Thr Pro Trp
                85                  90                  95

Thr Phe Gly Gly Gly Thr Lys Leu Glu Leu Lys
            100                 105

<210>  73
<211>  107
<212>  PRT
<213>  Artificial Sequence

<220>
<223>  anti-TfR mAb 8D3 VL variant L104V L106I

<400>  73

Asp Ile Gln Met Thr Gln Ser Pro Ala Ser Leu Ser Ala Ser Leu Glu
1               5                   10                  15

Glu Ile Val Thr Ile Thr Cys Gln Ala Ser Gln Asp Ile Gly Asn Trp
            20                  25                  30

Leu Ala Trp Tyr Gln Gln Lys Pro Gly Lys Ser Pro Gln Leu Leu Ile
        35                  40                  45

Tyr Gly Ala Thr Ser Leu Ala Asp Gly Val Pro Ser Arg Phe Ser Gly
    50                  55                  60

Ser Arg Ser Gly Thr Gln Phe Ser Leu Lys Ile Ser Arg Val Gln Val
65                  70                  75                  80

Glu Asp Ile Gly Ile Tyr Tyr Cys Leu Gln Ala Tyr Asn Thr Pro Trp
                85                  90                  95

Thr Phe Gly Gly Gly Thr Lys Val Glu Ile Lys
            100                 105


<210>  74
<211>  122
<212>  PRT
<213>  Oryctolagus cuniculus

<400>  74

Gln Ser Met Glu Glu Ser Gly Gly Arg Leu Val Thr Pro Gly Thr Pro
1               5                   10                  15

Leu Thr Leu Thr Cys Thr Val Ser Gly Phe Ser Leu Ser Ser Tyr Ala
            20                  25                  30

Met Ser Trp Val Arg Gln Ala Pro Gly Lys Gly Leu Glu Trp Ile Gly
        35                  40                  45

Tyr Ile Trp Ser Gly Gly Ser Thr Asp Tyr Ala Ser Trp Ala Lys Gly
    50                  55                  60

Arg Phe Thr Ile Ser Lys Thr Ser Thr Thr Val Asp Leu Lys Ile Thr
65                  70                  75                  80

Ser Pro Thr Thr Glu Asp Thr Ala Thr Tyr Phe Cys Ala Arg Arg Tyr
                85                  90                  95

Gly Thr Ser Tyr Pro Asp Tyr Gly Asp Ala Asn Gly Phe Asp Pro Trp
            100                 105                 110

Gly Pro Gly Thr Leu Val Thr Val Ser Ser
        115                 120

<210>  75
<211>  110
<212>  PRT
<213>  Oryctolagus cuniculus

<400>  75

Ala Tyr Asp Met Thr Gln Thr Pro Ala Ser Val Glu Val Ala Val Gly
1               5                   10                  15

Gly Thr Val Thr Ile Lys Cys Gln Ala Ser Gln Ser Ile Ser Ser Tyr
            20                  25                  30

Leu Ser Trp Tyr Gln Gln Lys Pro Gly Gln Arg Pro Lys Leu Leu Ile
        35                  40                  45

Tyr Arg Ala Ser Thr Leu Ala Ser Gly Val Ser Ser Arg Phe Lys Gly
    50                  55                  60

Ser Gly Ser Gly Thr Gln Phe Thr Leu Thr Ile Ser Gly Val Glu Cys
65                  70                  75                  80

Ala Asp Ala Ala Thr Tyr Tyr Cys Gln Gln Cys Tyr Ser Ser Ser Asn
                85                  90                  95

Val Asp Asn Thr Phe Gly Gly Gly Thr Glu Val Val Val Lys
            100                 105                 110

<210>  76
<211>  7
<212>  PRT
<213>  Oryctolagus cuniculus

<400>  76

Gly Phe Ser Leu Ser Ser Tyr
1               5

<210>  77
<211>  5
<212>  PRT
<213>  Oryctolagus cuniculus

<400>  77

Trp Ser Gly Gly Ser
1               5

<210>  78
<211>  17
<212>  PRT
<213>  Oryctolagus cuniculus

<400>  78

Arg Tyr Gly Thr Ser Tyr Pro Asp Tyr Gly Asp Ala Asn Gly Phe Asp
1               5                   10                  15

Pro

<210>  79
<211>  17
<212>  PRT
<213>  Artificial Sequence

<220>
<223>  299-000 HVR-H3 DASG

<400>  79

Arg Tyr Gly Thr Ser Tyr Pro Asp Tyr Gly Asp Ala Ser Gly Phe Asp
1               5                   10                  15

Pro

<210>  80
<211>  17
<212>  PRT
<213>  Artificial Sequence

<220>
<223>  299-000 HVR-H3 DAQG

<400>  80

Arg Tyr Gly Thr Ser Tyr Pro Asp Tyr Gly Asp Ala Gln Gly Phe Asp
1               5                   10                  15

Pro

<210>  81
<211>  11
<212>  PRT
<213>  Artificial Sequence

<220>
<223>  299-000 HVR-L1 RAA

<400>  81

Arg Ala Ser Gln Ser Ile Ser Ser Tyr Leu Ala
1               5                   10

<210>  82
<211>  7
<212>  PRT
<213>  Oryctolagus cuniculus

<400>  82

Arg Ala Ser Thr Leu Ala Ser
1               5


<210>  83
<211>  12
<212>  PRT
<213>  Artificial Sequence

<220>
<223>  299-000 HVR-L3 NYA

<400>  83

Gln Gln Asn Tyr Ala Ser Ser Asn Val Asp Asn Thr
1               5                   10


<210>  84
<211>  122
<212>  PRT
<213>  Artificial Sequence

<220>
<223>  anti-TfR mAb 567 VH (=humanized 299 VH_basic)

<400>  84

Gln Ser Met Glu Glu Ser Gly Gly Arg Leu Val Thr Pro Gly Thr Pro
1               5                   10                  15

Leu Thr Leu Thr Cys Thr Val Ser Gly Phe Ser Leu Ser Ser Tyr Ala
            20                  25                  30

Met Ser Trp Val Arg Gln Ala Pro Gly Lys Gly Leu Glu Trp Ile Gly
        35                  40                  45

Tyr Ile Trp Ser Gly Gly Ser Thr Asp Tyr Ala Ser Trp Ala Lys Gly
    50                  55                  60

Arg Phe Thr Ile Ser Lys Thr Ser Thr Thr Val Asp Leu Lys Ile Thr
65                  70                  75                  80

Ser Pro Thr Thr Glu Asp Thr Ala Thr Tyr Phe Cys Ala Arg Arg Tyr
                85                  90                  95

Gly Thr Ser Tyr Pro Asp Tyr Gly Asp Ala Asn Gly Phe Asp Pro Trp
            100                 105                 110

Gly Pro Gly Thr Leu Val Thr Val Ser Ser
        115                 120

<210>  85
<211>  110
<212>  PRT
<213>  Artificial Sequence

<220>
<223>  anti-TfR mAb 567 VL (=humanized 299 VL_basic)

<400>  85

Ala Tyr Asp Met Thr Gln Thr Pro Ala Ser Val Glu Val Ala Val Gly
1               5                   10                  15

Gly Thr Val Thr Ile Lys Cys Gln Ala Ser Gln Ser Ile Ser Ser Tyr
            20                  25                  30

Leu Ser Trp Tyr Gln Gln Lys Pro Gly Gln Arg Pro Lys Leu Leu Ile
        35                  40                  45

Tyr Arg Ala Ser Thr Leu Ala Ser Gly Val Ser Ser Arg Phe Lys Gly
    50                  55                  60

Ser Gly Ser Gly Thr Gln Phe Thr Leu Thr Ile Ser Gly Val Glu Ser
65                  70                  75                  80

Ala Asp Ala Ala Thr Tyr Tyr Cys Gln Gln Ser Tyr Ser Ser Ser Asn
                85                  90                  95

Val Asp Asn Thr Phe Gly Gly Gly Thr Glu Val Val Val Lys
            100                 105                 110

<210>  86
<211>  122
<212>  PRT
<213>  Artificial Sequence

<220>
<223>  anti-TfR mAb 932 VH (=humanized 299 VH_mutated)

<400>  86

Gln Ser Met Gln Glu Ser Gly Pro Gly Leu Val Lys Pro Ser Gln Thr
1               5                   10                  15

Leu Ser Leu Thr Cys Thr Val Ser Gly Phe Ser Leu Ser Ser Tyr Ala
            20                  25                  30

Met Ser Trp Ile Arg Gln His Pro Gly Lys Gly Leu Glu Trp Ile Gly

35                  40                  45

Tyr Ile Trp Ser Gly Gly Ser Thr Asp Tyr Ala Ser Trp Ala Lys Ser
    50                  55                  60

Arg Val Thr Ile Ser Lys Thr Ser Thr Thr Val Ser Leu Lys Leu Ser
65                  70                  75                  80

Ser Val Thr Ala Ala Asp Thr Ala Val Tyr Tyr Cys Ala Arg Arg Tyr
                85                  90                  95

Gly Thr Ser Tyr Pro Asp Tyr Gly Asp Ala Asn Gly Phe Asp Pro Trp
            100                 105                 110

Gly Gln Gly Thr Leu Val Thr Val Ser Ser
        115                 120

<210>  87
<211>  110
<212>  PRT
<213>  Artificial Sequence

<220>
<223>  anti-TfR mAb 932 VL (=humanized 299 VL_mutated)

<400>  87

Ala Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Ala Trp Tyr Gln Gln Lys Pro Gly Lys Ala Pro Lys Leu Leu Ile
        35                  40                  45

Tyr Arg Ala Ser Thr Leu Ala Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asn Tyr Ala Ser Ser Asn
                85                  90                  95

Val Asp Asn Thr Phe Gly Gly Gly Thr Lys Val Glu Ile Lys
            100                 105                 110

<210>  88
<211>  122

<212>  PRT
<213>  Artificial Sequence

<220>
<223>  299-023 VH humanization variant_DASG

<400>  88

Gln Ser Met Gln Glu Ser Gly Pro Gly Leu Val Lys Pro Ser Gln Thr
1               5                   10                  15

Leu Ser Leu Thr Cys Thr Val Ser Gly Phe Ser Leu Ser Ser Tyr Ala
            20                  25                  30

Met Ser Trp Ile Arg Gln His Pro Gly Lys Gly Leu Glu Trp Ile Gly
        35                  40                  45

Tyr Ile Trp Ser Gly Gly Ser Thr Asp Tyr Ala Ser Trp Ala Lys Ser
    50                  55                  60

Arg Val Thr Ile Ser Lys Thr Ser Thr Thr Val Ser Leu Lys Leu Ser
65                  70                  75                  80

Ser Val Thr Ala Ala Asp Thr Ala Val Tyr Tyr Cys Ala Arg Arg Tyr
                85                  90                  95

Gly Thr Ser Tyr Pro Asp Tyr Gly Asp Ala Ser Gly Phe Asp Pro Trp
            100                 105                 110

Gly Gln Gly Thr Leu Val Thr Val Ser Ser
        115                 120


<210>  89
<211>  119
<212>  PRT
<213>  Mus musculus

<400>  89

Glu Val Gln Leu Gln Gln Ser Gly Ala Val Leu Val Lys Pro Gly Ala
1               5                   10                  15

Ser Val Lys Leu Ser Cys Pro Ala Ser Gly Phe Asn Ile Lys Asp Thr
            20                  25                  30

Tyr Ile His Trp Val Ile Gln Arg Pro Glu Gln Gly Leu Glu Trp Ile
        35                  40                  45

Gly Arg Ile Asp Pro Ala Asn Gly Asp Thr Lys Cys Asp Pro Lys Phe
    50                  55                  60

Gln Val Lys Ala Thr Ile Thr Ala Asp Thr Ser Ser Asn Thr Ala Tyr
65                  70                  75                  80

Leu Gln Leu Ser Ser Leu Thr Ser Glu Asp Thr Ala Val Tyr Phe Cys
                85                  90                  95

Val Arg Asp Tyr Leu Tyr Pro Tyr Tyr Phe Asp Phe Trp Gly Gln Gly
            100                 105                 110

Thr Thr Leu Thr Val Ser Ser
        115

<210>  90
<211>  108
<212>  PRT
<213>  Mus musculus

<400>  90

Lys Ile Val Met Thr Gln Ser Pro Lys Ser Met Ser Met Ser Val Gly
1               5                   10                  15

Glu Arg Val Thr Leu Asn Cys Arg Ala Ser Glu Ser Val Asp Thr Tyr
            20                  25                  30

Val Ser Trp Tyr Gln Gln Lys Pro Glu Gln Ser Pro Glu Leu Leu Ile
        35                  40                  45

Tyr Gly Ala Ser Asn Arg Tyr Thr Gly Val Pro Asp Arg Phe Thr Gly
    50                  55                  60

Ser Gly Ser Ala Thr Asp Phe Thr Leu Thr Ile Ser Ser Val Gln Ala
65                  70                  75                  80

Glu Asp Leu Ala Asp Tyr Tyr Cys Gly Gln Thr Tyr Asn Tyr Pro Leu
                85                  90                  95

Thr Phe Gly Ala Gly Thr Lys Leu Glu Leu Lys Arg
            100                 105

<210>  91
<211>  118
<212>  PRT
<213>  Mus musculus

<400>  91

Gln Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Gly Tyr

20                  25                  30

Thr Met Asn Trp Val Arg Gln Ala Pro Gly Gln Gly Leu Glu Trp Met
        35                  40                  45

Gly Arg Ile Asn Pro His Asn Gly Gly Thr Asp Tyr Ala Gln Lys Phe
    50                  55                  60

Gln Gly Arg Val Thr Met Thr Arg Asp Thr Ser Ile Ser Thr Ala Tyr
65                  70                  75                  80

Met Glu Leu Ser Arg Leu Arg Ser Asp Asp Thr Ala Val Tyr Tyr Cys
                85                  90                  95

Ala Arg Gly Tyr Tyr Tyr Tyr Ser Leu Asp Tyr Trp Gly Gln Gly Thr
            100                 105                 110

Leu Val Thr Val Ser Ser
        115

<210>  92
<211>  106
<212>  PRT
<213>  Mus musculus

<400>  92

Asp Ile Gln Met Thr Gln Ser Pro Ser Ser Leu Ser Ala Ser Val Gly
1               5                   10                  15

Asp Arg Val Thr Ile Thr Cys Arg Ala Ser Ser Ser Ile Arg Tyr Ile
            20                  25                  30

His Trp Tyr Gln Gln Lys Pro Gly Lys Ala Pro Lys Arg Leu Ile Tyr
        35                  40                  45

Asp Thr Ser Asn Leu Ala Ser Gly Val Pro Ser Arg Phe Ser Gly Ser
    50                  55                  60

Gly Ser Gly Thr Glu Phe Thr Leu Thr Ile Ser Ser Leu Gln Pro Glu
65                  70                  75                  80

Asp Phe Ala Thr Tyr Tyr Cys His Gln Arg Asn Ser Tyr Pro Trp Thr
                85                  90                  95

Phe Gly Gln Gly Thr Arg Leu Glu Ile Lys
            100                 105

<210>  93

<211>  15
<212>  PRT
<213>  Artificial Sequence

<220>
<223>  human transferrin receptor fragment

<400>  93

Ile Gly Gln Asn Met Val Thr Ile Val Gln Ser Asn Gly Asn Leu
1               5                   10                  15


<210>  94
<211>  15
<212>  PRT
<213>  Artificial Sequence

<220>
<223>  human transferrin receptor fragment

<400>  94

Asn Met Val Thr Ile Val Gln Ser Asn Gly Asn Leu Asp Pro Val
1               5                   10                  15


<210>  95
<211>  15
<212>  PRT
<213>  Artificial Sequence

<220>
<223>  human transferrin receptor fragment

<400>  95

Gln Ser Asn Gly Asn Leu Asp Pro Val Glu Ser Pro Glu Gly Tyr
1               5                   10                  15


<210>  96
<211>  693
<212>  PRT
<213>  Artificial Sequence

<220>
<223>  anti-Her2 circular fusion polypeptide

<400>  96

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Asn Ile Lys Asp Thr
            20                  25                  30


Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Gly
    210                 215                 220

Gly Gly Gly Ser Gly Gly Gly Gly Ser Asp Lys Thr His Thr Cys Pro
225                 230                 235                 240

Pro Cys Pro Ala Pro Glu Leu Leu Gly Gly Pro Ser Val Phe Leu Phe
                245                 250                 255

Pro Pro Lys Pro Lys Asp Thr Leu Met Ile Ser Arg Thr Pro Glu Val
            260                 265                 270

Thr Cys Val Val Val Asp Val Ser His Glu Asp Pro Glu Val Lys Phe
        275                 280                 285

Asn Trp Tyr Val Asp Gly Val Glu Val His Asn Ala Lys Thr Lys Pro
    290                 295                 300

Arg Glu Glu Gln Tyr Asn Ser Thr Tyr Arg Val Val Ser Val Leu Thr
305                 310                 315                 320

Val Leu His Gln Asp Trp Leu Asn Gly Lys Glu Tyr Lys Cys Lys Val
                325                 330                 335

Ser Asn Lys Ala Leu Pro Ala Pro Ile Glu Lys Thr Ile Ser Lys Ala
            340                 345                 350

Lys Gly Gln Pro Arg Glu Pro Gln Val Tyr Thr Leu Pro Pro Ser Arg
        355                 360                 365

Asp Glu Leu Thr Lys Asn Gln Val Ser Leu Thr Cys Leu Val Lys Gly
    370                 375                 380

Phe Tyr Pro Ser Asp Ile Ala Val Glu Trp Glu Ser Asn Gly Gln Pro
385                 390                 395                 400

Glu Asn Asn Tyr Lys Thr Thr Pro Pro Val Leu Asp Ser Asp Gly Ser
                405                 410                 415

Phe Phe Leu Tyr Ser Lys Leu Thr Val Asp Lys Ser Arg Trp Gln Gln
            420                 425                 430

Gly Asn Val Phe Ser Cys Ser Val Met His Glu Ala Leu His Asn His
        435                 440                 445

Tyr Thr Gln Lys Ser Leu Ser Leu Ser Pro Gly Lys Gly Gly Gly Gly
    450                 455                 460

Ser Gly Gly Gly Gly Ser Asp Ile Gln Met Thr Gln Ser Pro Ser Ser
465                 470                 475                 480

Leu Ser Ala Ser Val Gly Asp Arg Val Thr Ile Thr Cys Arg Ala Ser
                485                 490                 495

Gln Asp Val Asn Thr Ala Val Ala Trp Tyr Gln Gln Lys Pro Gly Lys
            500                 505                 510

Ala Pro Lys Leu Leu Ile Tyr Ser Ala Ser Phe Leu Tyr Ser Gly Val
        515                 520                 525

Pro Ser Arg Phe Ser Gly Ser Arg Ser Gly Thr Asp Phe Thr Leu Thr
    530                 535                 540

Ile Ser Ser Leu Gln Pro Glu Asp Phe Ala Thr Tyr Tyr Cys Gln Gln
545                 550                 555                 560

His Tyr Thr Thr Pro Pro Thr Phe Gly Gln Gly Thr Lys Val Glu Ile
                565                 570                 575

Lys Arg Thr Val Ala Ala Pro Ser Val Phe Ile Phe Pro Pro Ser Asp
            580                 585                 590

Glu Gln Leu Lys Ser Gly Thr Ala Ser Val Val Cys Leu Leu Asn Asn
        595                 600                 605

Phe Tyr Pro Arg Glu Ala Lys Val Gln Trp Lys Val Asp Asn Ala Leu
    610                 615                 620

Gln Ser Gly Asn Ser Gln Glu Ser Val Thr Glu Gln Asp Ser Lys Asp
625                 630                 635                 640

Ser Thr Tyr Ser Leu Ser Ser Thr Leu Thr Leu Ser Lys Ala Asp Tyr
                645                 650                 655

Glu Lys His Lys Val Tyr Ala Cys Glu Val Thr His Gln Gly Leu Ser
            660                 665                 670

Ser Pro Val Thr Lys Ser Phe Asn Arg Gly Glu Cys Gly Ser Gly His
        675                 680                 685

His His His His His
    690

<210>  97
<211>  692
<212>  PRT
<213>  Artificial Sequence

<220>
<223>  anti-cMet circular fusion polypeptide

<400>  97

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Ile Tyr Ser Tyr
            20                  25                  30

Leu Ala Trp Tyr Gln Gln Lys Pro Gly Lys Ala Pro Lys Leu Leu Val
        35                  40                  45

Tyr Asn Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Thr Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Ser Gly Gly Gly Gly Ser
    210                 215                 220

Gly Gly Gly Gly Ser Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Ser Arg Asp Glu Leu Thr
        355                 360                 365

Lys Asn Gln Val Ser Leu Thr Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys Gly Gly Gly Gly Ser Gly Gly Gly
    450                 455                 460

Gly Ser Gln Val Gln Leu Gln Glu Ser Gly Pro Gly Leu Val Lys Pro
465                 470                 475                 480

Ser Glu Thr Leu Ser Leu Thr Cys Thr Val Ser Gly Tyr Ser Ile Thr
                485                 490                 495

Ser Asp Tyr Ala Trp Asn Trp Ile Arg Gln Phe Pro Gly Lys Gly Leu
            500                 505                 510

Glu Trp Ile Gly Tyr Ile Ser Tyr Ser Gly Ser Thr Ser Tyr Leu Pro
        515                 520                 525

Ser Leu Lys Ser Arg Val Thr Ile Ser Arg Asp Thr Ser Lys Asn Gln
    530                 535                 540

Phe Ser Leu Lys Leu Ser Ser Val Thr Ala Ala Asp Thr Ala Val Tyr
545                 550                 555                 560

Tyr Cys Ala Pro Ser Tyr Tyr Tyr Gly Gly Lys His Val Ala Leu Phe

565                 570                 575

Ala Tyr Trp Gly Gln Gly Thr Leu Val Thr Val Ser Ser Ala Ser Thr
            580                 585                 590

Lys Gly Pro Ser Val Phe Pro Leu Ala Pro Ser Ser Lys Ser Thr Ser
        595                 600                 605

Gly Gly Thr Ala Ala Leu Gly Cys Leu Val Lys Asp Tyr Phe Pro Glu
    610                 615                 620

Pro Val Thr Val Ser Trp Asn Ser Gly Ala Leu Thr Ser Gly Val His
625                 630                 635                 640

Thr Phe Pro Ala Val Leu Gln Ser Ser Gly Leu Tyr Ser Leu Ser Ser
                645                 650                 655

Val Val Thr Val Pro Ser Ser Ser Leu Gly Thr Gln Thr Tyr Ile Cys
            660                 665                 670

Asn Val Asn His Lys Pro Ser Asn Thr Lys Val Asp Lys Lys Val Glu
        675                 680                 685

Pro Lys Ser Cys
    690

<210>  98
<211>  693
<212>  PRT
<213>  Artificial Sequence

<220>
<223>  anti-CD20 circular fusion polypeptide (1)

<400>  98

Gln Val Gln Leu Gln Gln Pro Gly Ala Glu Leu Val Lys Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Gly Arg Gly Leu Glu Trp Ile
        35                  40                  45

Gly Ala Ile Tyr Pro Gly Asn Gly Asp Thr Ser Tyr Asn Gln Lys Phe
    50                  55                  60

Lys Gly Lys Ala Thr Leu Thr Ala Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Ser Thr Tyr Tyr Gly Gly Asp Trp Tyr Phe Asn Val Trp Gly
            100                 105                 110

Ala Gly Thr Thr Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Lys Val Glu Pro Lys Ser Cys
    210                 215                 220

Gly Gly Gly Gly Ser Gly Gly Gly Gly Ser Asp Lys Thr His Thr Cys
225                 230                 235                 240

Pro Pro Cys Pro Ala Pro Glu Leu Leu Gly Gly Pro Ser Val Phe Leu
                245                 250                 255

Phe Pro Pro Lys Pro Lys Asp Thr Leu Met Ile Ser Arg Thr Pro Glu
            260                 265                 270

Val Thr Cys Val Val Val Asp Val Ser His Glu Asp Pro Glu Val Lys
        275                 280                 285

Phe Asn Trp Tyr Val Asp Gly Val Glu Val His Asn Ala Lys Thr Lys
    290                 295                 300

Pro Arg Glu Glu Gln Tyr Asn Ser Thr Tyr Arg Val Val Ser Val Leu
305                 310                 315                 320

Thr Val Leu His Gln Asp Trp Leu Asn Gly Lys Glu Tyr Lys Cys Lys

325                 330                 335

Val Ser Asn Lys Ala Leu Pro Ala Pro Ile Glu Lys Thr Ile Ser Lys
            340                 345                 350

Ala Lys Gly Gln Pro Arg Glu Pro Gln Val Tyr Thr Leu Pro Pro Ser
        355                 360                 365

Arg Asp Glu Leu Thr Lys Asn Gln Val Ser Leu Thr Cys Leu Val Lys
    370                 375                 380

Gly Phe Tyr Pro Ser Asp Ile Ala Val Glu Trp Glu Ser Asn Gly Gln
385                 390                 395                 400

Pro Glu Asn Asn Tyr Lys Thr Thr Pro Pro Val Leu Asp Ser Asp Gly
                405                 410                 415

Ser Phe Phe Leu Tyr Ser Lys Leu Thr Val Asp Lys Ser Arg Trp Gln
            420                 425                 430

Gln Gly Asn Val Phe Ser Cys Ser Val Met His Glu Ala Leu His Asn
        435                 440                 445

His Tyr Thr Gln Lys Ser Leu Ser Leu Ser Pro Gly Lys Gly Gly Gly
    450                 455                 460

Gly Ser Gly Gly Gly Gly Ser Gln Ile Val Leu Ser Gln Ser Pro Ala
465                 470                 475                 480

Ile Leu Ser Ala Ser Pro Gly Glu Lys Val Thr Met Thr Cys Arg Ala
                485                 490                 495

Ser Ser Ser Val Ser Tyr Ile His Trp Phe Gln Gln Lys Pro Gly Ser
            500                 505                 510

Ser Pro Lys Pro Trp Ile Tyr Ala Thr Ser Asn Leu Ala Ser Gly Val
        515                 520                 525

Pro Val Arg Phe Ser Gly Ser Gly Ser Gly Thr Ser Tyr Ser Leu Thr
    530                 535                 540

Ile Ser Arg Val Glu Ala Glu Asp Ala Ala Thr Tyr Tyr Cys Gln Gln
545                 550                 555                 560

Trp Thr Ser Asn Pro Pro Thr Phe Gly Gly Gly Thr Lys Leu Glu Ile
                565                 570                 575

Lys Arg Thr Val Ala Ala Pro Ser Val Phe Ile Phe Pro Pro Ser Asp
            580                 585                 590

Glu Gln Leu Lys Ser Gly Thr Ala Ser Val Val Cys Leu Leu Asn Asn
        595                 600                 605

Phe Tyr Pro Arg Glu Ala Lys Val Gln Trp Lys Val Asp Asn Ala Leu
    610                 615                 620

Gln Ser Gly Asn Ser Gln Glu Ser Val Thr Glu Gln Asp Ser Lys Asp
625                 630                 635                 640

Ser Thr Tyr Ser Leu Ser Ser Thr Leu Thr Leu Ser Lys Ala Asp Tyr
                645                 650                 655

Glu Lys His Lys Val Tyr Ala Cys Glu Val Thr His Gln Gly Leu Ser
            660                 665                 670

Ser Pro Val Thr Lys Ser Phe Asn Arg Gly Glu Cys Gly Ser Gly His
        675                 680                 685

His His His His His
    690

<210>  99
<211>  697
<212>  PRT
<213>  Artificial Sequence

<220>
<223>  anti-CD20 circular fusion polypeptide (2)

<400>  99

Gln Val Gln Leu Val Gln Ser Gly Ala Glu Val Lys Lys Pro Gly Ser
1               5                   10                  15

Ser Val Lys Val Ser Cys Lys Ala Ser Gly Tyr Ala Phe Ser Tyr Ser
            20                  25                  30

Trp Ile Asn Trp Val Arg Gln Ala Pro Gly Gln Gly Leu Glu Trp Met
        35                  40                  45

Gly Arg Ile Phe Pro Gly Asp Gly Asp Thr Asp Tyr Asn Gly Lys Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys

                85                  90                  95

Ala Arg Asn Val Phe Asp Gly Tyr Trp Leu Val Tyr Trp Gly Gln Gly
            100                 105                 110

Thr Leu Val Thr Val Ser Ser Ala Ser Thr Lys Gly Pro Ser Val Phe
        115                 120                 125

Pro Leu Ala Pro Ser Ser Lys Ser Thr Ser Gly Gly Thr Ala Ala Leu
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Gly Gly
    210                 215                 220

Gly Gly Ser Gly Gly Gly Gly Ser Asp Lys Thr His Thr Cys Pro Pro
225                 230                 235                 240

Cys Pro Ala Pro Glu Leu Leu Gly Gly Pro Ser Val Phe Leu Phe Pro
                245                 250                 255

Pro Lys Pro Lys Asp Thr Leu Met Ile Ser Arg Thr Pro Glu Val Thr
            260                 265                 270

Cys Val Val Val Asp Val Ser His Glu Asp Pro Glu Val Lys Phe Asn
        275                 280                 285

Trp Tyr Val Asp Gly Val Glu Val His Asn Ala Lys Thr Lys Pro Arg
    290                 295                 300

Glu Glu Gln Tyr Asn Ser Thr Tyr Arg Val Val Ser Val Leu Thr Val
305                 310                 315                 320

Leu His Gln Asp Trp Leu Asn Gly Lys Glu Tyr Lys Cys Lys Val Ser
                325                 330                 335

Asn Lys Ala Leu Pro Ala Pro Ile Glu Lys Thr Ile Ser Lys Ala Lys
            340                 345                 350

Gly Gln Pro Arg Glu Pro Gln Val Tyr Thr Leu Pro Pro Ser Arg Asp
        355                 360                 365

Glu Leu Thr Lys Asn Gln Val Ser Leu Thr Cys Leu Val Lys Gly Phe
    370                 375                 380

Tyr Pro Ser Asp Ile Ala Val Glu Trp Glu Ser Asn Gly Gln Pro Glu
385                 390                 395                 400

Asn Asn Tyr Lys Thr Thr Pro Pro Val Leu Asp Ser Asp Gly Ser Phe
                405                 410                 415

Phe Leu Tyr Ser Lys Leu Thr Val Asp Lys Ser Arg Trp Gln Gln Gly
            420                 425                 430

Asn Val Phe Ser Cys Ser Val Met His Glu Ala Leu His Asn His Tyr
        435                 440                 445

Thr Gln Lys Ser Leu Ser Leu Ser Pro Gly Lys Gly Gly Gly Gly Ser
    450                 455                 460

Gly Gly Gly Gly Ser Asp Ile Val Met Thr Gln Thr Pro Leu Ser Leu
465                 470                 475                 480

Pro Val Thr Pro Gly Glu Pro Ala Ser Ile Ser Cys Arg Ser Ser Lys
                485                 490                 495

Ser Leu Leu His Ser Asn Gly Ile Thr Tyr Leu Tyr Trp Tyr Leu Gln
            500                 505                 510

Lys Pro Gly Gln Ser Pro Gln Leu Leu Ile Tyr Gln Met Ser Asn Leu
        515                 520                 525

Val Ser Gly Val Pro Asp Arg Phe Ser Gly Ser Gly Ser Gly Thr Asp
    530                 535                 540

Phe Thr Leu Lys Ile Ser Arg Val Glu Ala Glu Asp Val Gly Val Tyr
545                 550                 555                 560

Tyr Cys Ala Gln Asn Leu Glu Leu Pro Tyr Thr Phe Gly Gly Gly Thr
                565                 570                 575

Lys Val Glu Ile Lys Arg Thr Val Ala Ala Pro Ser Val Phe Ile Phe
            580                 585                 590

Pro Pro Ser Asp Glu Gln Leu Lys Ser Gly Thr Ala Ser Val Val Cys
        595                 600                 605

Leu Leu Asn Asn Phe Tyr Pro Arg Glu Ala Lys Val Gln Trp Lys Val
    610                 615                 620

Asp Asn Ala Leu Gln Ser Gly Asn Ser Gln Glu Ser Val Thr Glu Gln
625                 630                 635                 640

Asp Ser Lys Asp Ser Thr Tyr Ser Leu Ser Ser Thr Leu Thr Leu Ser
                645                 650                 655

Lys Ala Asp Tyr Glu Lys His Lys Val Tyr Ala Cys Glu Val Thr His
            660                 665                 670

Gln Gly Leu Ser Ser Pro Val Thr Lys Ser Phe Asn Arg Gly Glu Cys
        675                 680                 685

Gly Ser Gly His His His His His His
    690                 695

<210>  100
<211>  689
<212>  PRT
<213>  Artificial Sequence

<220>
<223>  anti-cMet circular fusion polypeptide VH-out-knob

<400>  100

Gln Val Gln Leu Gln Glu Ser Gly Pro Gly Leu Val Lys Pro Ser Glu
1               5                   10                  15

Thr Leu Ser Leu Thr Cys Thr Val Ser Gly Tyr Ser Ile Thr Ser Asp
            20                  25                  30

Tyr Ala Trp Asn Trp Ile Arg Gln Phe Pro Gly Lys Gly Leu Glu Trp
        35                  40                  45

Ile Gly Tyr Ile Ser Tyr Ser Gly Ser Thr Ser Tyr Leu Pro Ser Leu
    50                  55                  60

Lys Ser Arg Val Thr Ile Ser Arg Asp Thr Ser Lys Asn Gln Phe Ser
65                  70                  75                  80

Leu Lys Leu Ser Ser Val Thr Ala Ala Asp Thr Ala Val Tyr Tyr Cys
                85                  90                  95

Ala Pro Ser Tyr Tyr Tyr Gly Gly Lys His Val Ala Leu Phe Ala Tyr
            100                 105                 110

Trp Gly Gln Gly Thr Leu Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Lys Val Glu Pro Lys
    210                 215                 220

Ser Cys Gly Ser Gly Gly Gly Gly Ser Gly Gly Gly Gly Ser Asp Lys
225                 230                 235                 240

Thr His Thr Cys Pro Pro Cys Pro Ala Pro Glu Leu Leu Gly Gly Pro
                245                 250                 255

Ser Val Phe Leu Phe Pro Pro Lys Pro Lys Asp Thr Leu Met Ile Ser
            260                 265                 270

Arg Thr Pro Glu Val Thr Cys Val Val Val Asp Val Ser His Glu Asp
        275                 280                 285

Pro Glu Val Lys Phe Asn Trp Tyr Val Asp Gly Val Glu Val His Asn
    290                 295                 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Ala Leu Pro Ala Pro Ile Glu Lys
            340                 345                 350

Thr Ile Ser Lys Ala Lys Gly Gln Pro Arg Glu Pro Gln Val Tyr Thr
        355                 360                 365

Leu Pro Pro Cys Arg Asp Glu Leu Thr Lys Asn Gln Val Ser Leu Trp
    370                 375                 380

Cys Leu Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Tyr Ser Lys Leu Thr Val Asp Lys
            420                 425                 430

Ser Arg Trp Gln Gln Gly Asn Val Phe Ser Cys Ser Val Met His Glu
        435                 440                 445

Ala Leu His Asn His Tyr Thr Gln Lys Ser Leu Ser Leu Ser Pro Gly
    450                 455                 460

Lys Gly Gly Gly Gly Ser Gly Gly Gly Gly Ser Asp Ile Gln Met Thr
465                 470                 475                 480

Gln Ser Pro Ser Ser Leu Ser Ala Ser Val Gly Asp Arg Val Thr Ile
                485                 490                 495

Thr Cys Arg Thr Ser Glu Asn Ile Tyr Ser Tyr Leu Ala Trp Tyr Gln
            500                 505                 510

Gln Lys Pro Gly Lys Ala Pro Lys Leu Leu Val Tyr Asn Ala Lys Thr
        515                 520                 525

Leu Ala Glu Gly Val Pro Ser Arg Phe Ser Gly Ser Gly Ser Gly Thr
    530                 535                 540

Asp Phe Thr Leu Thr Ile Ser Ser Leu Gln Pro Glu Asp Phe Ala Thr
545                 550                 555                 560

Tyr Tyr Cys Gln His His Tyr Gly Thr Pro Phe Thr Phe Gly Gln Gly
                565                 570                 575

Thr Lys Leu Glu Ile Lys Arg Thr Val Ala Ala Pro Ser Val Phe Ile
            580                 585                 590

Phe Pro Pro Ser Asp Glu Gln Leu Lys Ser Gly Thr Ala Ser Val Val
        595                 600                 605

Cys Leu Leu Asn Asn Phe Tyr Pro Arg Glu Ala Lys Val Gln Trp Lys
    610                 615                 620

Val Asp Asn Ala Leu Gln Ser Gly Asn Ser Gln Glu Ser Val Thr Glu
625                 630                 635                 640

Gln Asp Ser Lys Asp Ser Thr Tyr Ser Leu Ser Ser Thr Leu Thr Leu
                645                 650                 655

Ser Lys Ala Asp Tyr Glu Lys His Lys Val Tyr Ala Cys Glu Val Thr
            660                 665                 670

His Gln Gly Leu Ser Ser Pro Val Thr Lys Ser Phe Asn Arg Gly Glu
        675                 680                 685

Cys

<210>  101
<211>  692
<212>  PRT
<213>  Artificial Sequence

<220>
<223>  anti-cMet circular fusion polypeptide VH-in-knob

<400>  101

Asp Ile Gln Met Thr Gln Ser Pro Ser Ser Leu Ser Ala Ser Val Gly
1               5                   10                  15

Asp Arg Val Thr Ile Thr Cys Arg Thr Ser Glu Asn Ile Tyr Ser Tyr
            20                  25                  30

Leu Ala Trp Tyr Gln Gln Lys Pro Gly Lys Ala Pro Lys Leu Leu Val
        35                  40                  45

Tyr Asn Ala Lys Thr Leu Ala Glu Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln His His Tyr Gly Thr Pro Phe
                85                  90                  95

Thr Phe Gly Gln Gly Thr Lys Leu Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys Gly Gly Gly Gly Ser Gly Gly Gly Gly Ser
    210                 215                 220

Gly Gly Gly Gly Ser Asp Lys Thr His Thr Cys Pro Pro Cys Pro Ala
225                 230                 235                 240

Pro Glu Leu Leu Gly Gly Pro Ser Val Phe Leu Phe Pro Pro Lys Pro
                245                 250                 255

Lys Asp Thr Leu Met Ile Ser Arg Thr Pro Glu Val Thr Cys Val Val
            260                 265                 270

Val Asp Val Ser His Glu Asp Pro Glu Val Lys Phe Asn Trp Tyr Val
        275                 280                 285

Asp Gly Val Glu Val His Asn Ala Lys Thr Lys Pro Arg Glu Glu Gln
    290                 295                 300

Tyr Asn Ser Thr Tyr Arg Val Val Ser Val Leu Thr Val Leu His Gln
305                 310                 315                 320

Asp Trp Leu Asn Gly Lys Glu Tyr Lys Cys Lys Val Ser Asn Lys Ala
                325                 330                 335

Leu Pro Ala Pro Ile Glu Lys Thr Ile Ser Lys Ala Lys Gly Gln Pro
            340                 345                 350

Arg Glu Pro Gln Val Tyr Thr Leu Pro Pro Cys Arg Asp Glu Leu Thr
        355                 360                 365

Lys Asn Gln Val Ser Leu Trp Cys Leu Val Lys Gly Phe Tyr Pro Ser
    370                 375                 380

Asp Ile Ala Val Glu Trp Glu Ser Asn Gly Gln Pro Glu Asn Asn Tyr
385                 390                 395                 400

Lys Thr Thr Pro Pro Val Leu Asp Ser Asp Gly Ser Phe Phe Leu Tyr
                405                 410                 415

Ser Lys Leu Thr Val Asp Lys Ser Arg Trp Gln Gln Gly Asn Val Phe
            420                 425                 430

Ser Cys Ser Val Met His Glu Ala Leu His Asn His Tyr Thr Gln Lys
        435                 440                 445

Ser Leu Ser Leu Ser Pro Gly Lys Gly Gly Gly Gly Ser Gly Gly Gly
    450                 455                 460

Gly Ser Gln Val Gln Leu Gln Glu Ser Gly Pro Gly Leu Val Lys Pro
465                 470                 475                 480

Ser Glu Thr Leu Ser Leu Thr Cys Thr Val Ser Gly Tyr Ser Ile Thr
                485                 490                 495

Ser Asp Tyr Ala Trp Asn Trp Ile Arg Gln Phe Pro Gly Lys Gly Leu
            500                 505                 510

Glu Trp Ile Gly Tyr Ile Ser Tyr Ser Gly Ser Thr Ser Tyr Leu Pro
        515                 520                 525

Ser Leu Lys Ser Arg Val Thr Ile Ser Arg Asp Thr Ser Lys Asn Gln
    530                 535                 540

Phe Ser Leu Lys Leu Ser Ser Val Thr Ala Ala Asp Thr Ala Val Tyr
545                 550                 555                 560

Tyr Cys Ala Pro Ser Tyr Tyr Tyr Gly Gly Lys His Val Ala Leu Phe
                565                 570                 575

Ala Tyr Trp Gly Gln Gly Thr Leu Val Thr Val Ser Ser Ala Ser Thr
            580                 585                 590

Lys Gly Pro Ser Val Phe Pro Leu Ala Pro Ser Ser Lys Ser Thr Ser
        595                 600                 605

Gly Gly Thr Ala Ala Leu Gly Cys Leu Val Lys Asp Tyr Phe Pro Glu

610                 615                 620

Pro Val Thr Val Ser Trp Asn Ser Gly Ala Leu Thr Ser Gly Val His
625                 630                 635                 640

Thr Phe Pro Ala Val Leu Gln Ser Ser Gly Leu Tyr Ser Leu Ser Ser
                645                 650                 655

Val Val Thr Val Pro Ser Ser Ser Leu Gly Thr Gln Thr Tyr Ile Cys
            660                 665                 670

Asn Val Asn His Lys Pro Ser Asn Thr Lys Val Asp Lys Lys Val Glu
        675                 680                 685

Pro Lys Ser Cys
    690

<210>  102
<211>  685
<212>  PRT
<213>  Artificial Sequence

<220>
<223>  anti-cMet circular fusion polypeptide VH-out-hole

<400>  102

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ala Tyr
            20                  25                  30

Phe Ile Asn Trp Val Arg Gln Met Pro Gly Lys Gly Leu Glu Trp Met
        35                  40                  45

Gly Arg Ile Tyr Pro Tyr Asn Gly Asn Thr Phe Tyr Asp Gln Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Trp Asp Tyr Asn Tyr Asp Val Trp Gly Gln Gly Thr Thr Val
            100                 105                 110

Thr Val Ser Ser Ala Ser Thr Lys Gly Pro Ser Val Phe Pro Leu Ala
        115                 120                 125

Pro Ser Ser Lys Ser Thr Ser Gly Gly Thr Ala Ala Leu Gly Cys Leu
    130                 135                 140

Val Lys Asp Tyr Phe Pro Glu Pro Val Thr Val Ser Trp Asn Ser Gly
145                 150                 155                 160

Ala Leu Thr Ser Gly Val His Thr Phe Pro Ala Val Leu Gln Ser Ser
                165                 170                 175

Gly Leu Tyr Ser Leu Ser Ser Val Val Thr Val Pro Ser Ser Ser Leu
            180                 185                 190

Gly Thr Gln Thr Tyr Ile Cys Asn Val Asn His Lys Pro Ser Asn Thr
        195                 200                 205

Lys Val Asp Lys Lys Val Glu Pro Lys Ser Cys Gly Gly Gly Gly Ser
    210                 215                 220

Gly Gly Gly Gly Ser Gly Gly Gly Gly Ser Asp Lys Thr His Thr Cys
225                 230                 235                 240

Pro Pro Cys Pro Ala Pro Glu Leu Leu Gly Gly Pro Ser Val Phe Leu
                245                 250                 255

Phe Pro Pro Lys Pro Lys Asp Thr Leu Met Ile Ser Arg Thr Pro Glu
            260                 265                 270

Val Thr Cys Val Val Val Asp Val Ser His Glu Asp Pro Glu Val Lys
        275                 280                 285

Phe Asn Trp Tyr Val Asp Gly Val Glu Val His Asn Ala Lys Thr Lys
    290                 295                 300

Pro Arg Glu Glu Gln Tyr Asn Ser Thr Tyr Arg Val Val Ser Val Leu
305                 310                 315                 320

Thr Val Leu His Gln Asp Trp Leu Asn Gly Lys Glu Tyr Lys Cys Lys
                325                 330                 335

Val Ser Asn Lys Ala Leu Pro Ala Pro Ile Glu Lys Thr Ile Ser Lys
            340                 345                 350

Ala Lys Gly Gln Pro Arg Glu Pro Gln Val Cys Thr Leu Pro Pro Ser
        355                 360                 365

Arg Asp Glu Leu Thr Lys Asn Gln Val Ser Leu Ser Cys Ala Val Lys

370                 375                 380

Gly Phe Tyr Pro Ser Asp Ile Ala Val Glu Trp Glu Ser Asn Gly Gln
385                 390                 395                 400

Pro Glu Asn Asn Tyr Lys Thr Thr Pro Pro Val Leu Asp Ser Asp Gly
                405                 410                 415

Ser Phe Phe Leu Val Ser Lys Leu Thr Val Asp Lys Ser Arg Trp Gln
            420                 425                 430

Gln Gly Asn Val Phe Ser Cys Ser Val Met His Glu Ala Leu His Asn
        435                 440                 445

His Tyr Thr Gln Lys Ser Leu Ser Leu Ser Pro Gly Lys Gly Gly Gly
    450                 455                 460

Gly Ser Gly Gly Gly Gly Ser Glu Ile Val Leu Thr Gln Ser Pro Ala
465                 470                 475                 480

Thr Leu Ser Leu Ser Pro Gly Glu Arg Ala Thr Leu Ser Cys Arg Ala
                485                 490                 495

Ser Ser Ser Val Ser His Leu Tyr Trp Tyr Gln Gln Lys Pro Gly Gln
            500                 505                 510

Ala Pro Arg Leu Trp Ile Tyr Asp Thr Ser Asn Leu Ala Ser Gly Val
        515                 520                 525

Pro Ala Arg Phe Ser Gly Ser Arg Ser Gly Thr Asp Phe Thr Leu Thr
    530                 535                 540

Ile Ser Ser Leu Glu Pro Glu Asp Phe Ala Val Tyr Phe Cys His Gln
545                 550                 555                 560

Arg Thr Asn Tyr Pro Trp Thr Phe Gly Gln Gly Thr Lys Leu Glu Ile
                565                 570                 575

Lys Arg Thr Val Ala Ala Pro Ser Val Phe Ile Phe Pro Pro Ser Asp
            580                 585                 590

Glu Gln Leu Lys Ser Gly Thr Ala Ser Val Val Cys Leu Leu Asn Asn
        595                 600                 605

Phe Tyr Pro Arg Glu Ala Lys Val Gln Trp Lys Val Asp Asn Ala Leu
    610                 615                 620

Gln Ser Gly Asn Ser Gln Glu Ser Val Thr Glu Gln Asp Ser Lys Asp
625                 630                 635                 640

Ser Thr Tyr Ser Leu Ser Ser Thr Leu Thr Ser Leu Ser Lys Ala Asp
                645                 650                 655

Tyr Glu Lys His Lys Val Tyr Ala Cys Glu Val Thr His Gln Gly Leu
            660                 665                 670

Ser Ser Pro Val Thr Lys Ser Phe Asn Arg Gly Glu Cys
        675                 680                 685

<210>  103
<211>  686
<212>  PRT
<213>  Artificial Sequence

<220>
<223>  anti-cMet circular fusion polypeptide VH-out-hole-CH-CL-crossed

<400>  103

Glu Val Gln Leu Val Gln Ser Gly Ala Glu Val Lys Lys Pro Gly Glu
1               5                   10                  15

Ser Leu Lys Ile Ser Cys Lys Gly Ser Gly Tyr Ser Phe Thr Ala Tyr
            20                  25                  30

Phe Ile Asn Trp Val Arg Gln Met Pro Gly Lys Gly Leu Glu Trp Met
        35                  40                  45

Gly Arg Ile Tyr Pro Tyr Asn Gly Asn Thr Phe Tyr Asp Gln Ser Phe
    50                  55                  60

Gln Gly Gln Val Thr Ile Ser Ala Asp Lys Ser Ile Ser Thr Ala Tyr
65                  70                  75                  80

Leu Gln Trp Ser Ser Leu Lys Ala Ser Asp Thr Ala Met Tyr Tyr Cys
                85                  90                  95

Ala Arg Trp Asp Tyr Asn Tyr Asp Val Trp Gly Gln Gly Thr Thr Val
            100                 105                 110

Thr Val Ser Ser Ser Ala Val Ala Ala Pro Ser Val Phe Ile Phe Pro
        115                 120                 125

Pro Ser Asp Glu Gln Leu Lys Ser Gly Thr Ala Ser Val Val Cys Leu
    130                 135                 140

Leu Asn Asn Phe Tyr Pro Arg Glu Ala Lys Val Gln Trp Lys Val Asp

145                 150                 155                 160

Asn Ala Leu Gln Ser Gly Asn Ser Gln Glu Ser Val Thr Glu Gln Asp
                165                 170                 175

Ser Lys Asp Ser Thr Tyr Ser Leu Ser Ser Thr Leu Thr Leu Ser Lys
            180                 185                 190

Ala Asp Tyr Glu Lys His Lys Val Tyr Ala Cys Glu Val Thr His Gln
        195                 200                 205

Gly Leu Ser Ser Pro Val Thr Lys Ser Phe Asn Arg Gly Glu Cys Gly
    210                 215                 220

Gly Gly Gly Ser Gly Gly Gly Gly Ser Gly Gly Gly Gly Ser Asp Lys
225                 230                 235                 240

Thr His Thr Cys Pro Pro Cys Pro Ala Pro Glu Leu Leu Gly Gly Pro
                245                 250                 255

Ser Val Phe Leu Phe Pro Pro Lys Pro Lys Asp Thr Leu Met Ile Ser
            260                 265                 270

Arg Thr Pro Glu Val Thr Cys Val Val Val Asp Val Ser His Glu Asp
        275                 280                 285

Pro Glu Val Lys Phe Asn Trp Tyr Val Asp Gly Val Glu Val His Asn
    290                 295                 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Ala Leu Pro Ala Pro Ile Glu Lys
            340                 345                 350

Thr Ile Ser Lys Ala Lys Gly Gln Pro Arg Glu Pro Gln Val Cys Thr
        355                 360                 365

Leu Pro Pro Ser Arg Asp Glu Leu Thr Lys Asn Gln Val Ser Leu Ser
    370                 375                 380

Cys Ala Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Val Ser Lys Leu Thr Val Asp Lys
            420                 425                 430

Ser Arg Trp Gln Gln Gly Asn Val Phe Ser Cys Ser Val Met His Glu
        435                 440                 445

Ala Leu His Asn His Tyr Thr Gln Lys Ser Leu Ser Leu Ser Pro Gly
    450                 455                 460

Lys Gly Gly Gly Gly Ser Gly Gly Gly Gly Ser Glu Ile Val Leu Thr
465                 470                 475                 480

Gln Ser Pro Ala Thr Leu Ser Leu Ser Pro Gly Glu Arg Ala Thr Leu
                485                 490                 495

Ser Cys Arg Ala Ser Ser Ser Val Ser His Leu Tyr Trp Tyr Gln Gln
            500                 505                 510

Lys Pro Gly Gln Ala Pro Arg Leu Trp Ile Tyr Asp Thr Ser Asn Leu
        515                 520                 525

Ala Ser Gly Val Pro Ala Arg Phe Ser Gly Ser Arg Ser Gly Thr Asp
    530                 535                 540

Phe Thr Leu Thr Ile Ser Ser Leu Glu Pro Glu Asp Phe Ala Val Tyr
545                 550                 555                 560

Phe Cys His Gln Arg Thr Asn Tyr Pro Trp Thr Phe Gly Gln Gly Thr
                565                 570                 575

Lys Leu Glu Ile Lys Ser Ser Ala Ser Thr Lys Gly Pro Ser Val Phe
            580                 585                 590

Pro Leu Ala Pro Ser Ser Lys Ser Thr Ser Gly Gly Thr Ala Ala Leu
        595                 600                 605

Gly Cys Leu Val Lys Asp Tyr Phe Pro Glu Pro Val Thr Val Ser Trp
    610                 615                 620

Asn Ser Gly Ala Leu Thr Ser Gly Val His Thr Phe Pro Ala Val Leu
625                 630                 635                 640

Gln Ser Ser Gly Leu Tyr Ser Leu Ser Ser Val Val Thr Val Pro Ser
                645                 650                 655

Ser Ser Leu Gly Thr Gln Thr Tyr Ile Cys Asn Val Asn His Lys Pro
            660                 665                 670

Ser Asn Thr Lys Val Asp Lys Lys Val Glu Pro Lys Ser Cys
        675                 680                 685

<210>  104
<211>  683
<212>  PRT
<213>  Artificial Sequence

<220>
<223>  anti-cMet circular fusion polypeptide VH-out-hole-VH-VL-crossed

<400>  104

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Ser Ser Val Ser His Leu
            20                  25                  30

Tyr Trp Tyr Gln Gln Lys Pro Gly Gln Ala Pro Arg Leu Trp Ile Tyr
        35                  40                  45

Asp Thr Ser Asn Leu Ala Ser Gly Val Pro Ala Arg Phe Ser Gly Ser
    50                  55                  60

Arg Ser Gly Thr Asp Phe Thr Leu Thr Ile Ser Ser Leu Glu Pro Glu
65                  70                  75                  80

Asp Phe Ala Val Tyr Phe Cys His Gln Arg Thr Asn Tyr Pro Trp Thr
                85                  90                  95

Phe Gly Gln Gly Thr Lys Leu Glu Ile Lys Ser Ser Ala Ser Thr Lys
            100                 105                 110

Gly Pro Ser Val Phe Pro Leu Ala Pro Ser Ser Lys Ser Thr Ser Gly
        115                 120                 125

Gly Thr Ala Ala Leu Gly Cys Leu Val Lys Asp Tyr Phe Pro Glu Pro
    130                 135                 140

Val Thr Val Ser Trp Asn Ser Gly Ala Leu Thr Ser Gly Val His Thr
145                 150                 155                 160

Phe Pro Ala Val Leu Gln Ser Ser Gly Leu Tyr Ser Leu Ser Ser Val
                165                 170                 175

Val Thr Val Pro Ser Ser Ser Leu Gly Thr Gln Thr Tyr Ile Cys Asn
            180                 185                 190

Val Asn His Lys Pro Ser Asn Thr Lys Val Asp Lys Lys Val Glu Pro
        195                 200                 205

Lys Ser Cys Gly Ser Gly Gly Gly Gly Ser Gly Gly Gly Gly Ser Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asn Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Cys
            340                 345                 350

Thr Leu Pro Pro Ser Arg Asp Glu Leu Thr Lys Asn Gln Val Ser Leu
        355                 360                 365

Ser Cys Ala Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Val Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly Lys Gly Gly Gly Gly Ser Gly Gly Gly Gly Ser Glu Val Gln Leu
    450                 455                 460

Val Gln Ser Gly Ala Glu Val Lys Lys Pro Gly Glu Ser Leu Lys Ile
465                 470                 475                 480

Ser Cys Lys Gly Ser Gly Tyr Ser Phe Thr Ala Tyr Phe Ile Asn Trp
                485                 490                 495

Val Arg Gln Met Pro Gly Lys Gly Leu Glu Trp Met Gly Arg Ile Tyr
            500                 505                 510

Pro Tyr Asn Gly Asn Thr Phe Tyr Asp Gln Ser Phe Gln Gly Gln Val
        515                 520                 525

Thr Ile Ser Ala Asp Lys Ser Ile Ser Thr Ala Tyr Leu Gln Trp Ser
    530                 535                 540

Ser Leu Lys Ala Ser Asp Thr Ala Met Tyr Tyr Cys Ala Arg Trp Asp
545                 550                 555                 560

Tyr Asn Tyr Asp Val Trp Gly Gln Gly Thr Thr Val Thr Val Ser Ser
                565                 570                 575

Ser Ala Val Ala Ala Pro Ser Val Phe Ile Phe Pro Pro Ser Asp Glu
            580                 585                 590

Gln Leu Lys Ser Gly Thr Ala Ser Val Val Cys Leu Leu Asn Asn Phe
        595                 600                 605

Tyr Pro Arg Glu Ala Lys Val Gln Trp Lys Val Asp Asn Ala Leu Gln
    610                 615                 620

Ser Gly Asn Ser Gln Glu Ser Val Thr Glu Gln Asp Ser Lys Asp Ser
625                 630                 635                 640

Thr Tyr Ser Leu Ser Ser Thr Leu Thr Leu Ser Lys Ala Asp Tyr Glu
                645                 650                 655

Lys His Lys Val Tyr Ala Cys Glu Val Thr His Gln Gly Leu Ser Ser
            660                 665                 670

Pro Val Thr Lys Ser Phe Asn Arg Gly Glu Cys
        675                 680

<210>  105
<211>  120
<212>  PRT
<213>  Homo sapiens

<400>  105

Asp Lys Thr His Thr Cys Pro Pro Cys Pro Ala Pro Glu Leu Leu Gly
1               5                   10                  15

Gly Pro Ser Val Phe Leu Phe Pro Pro Lys Pro Lys Asp Thr Leu Met
            20                  25                  30

Ile Ser Arg Thr Pro Glu Val Thr Cys Val Val Val Asp Val Ser His
        35                  40                  45

Glu Asp Pro Glu Val Lys Phe Asn Trp Tyr Val Asp Gly Val Glu Val
    50                  55                  60

His Asn Ala Lys Thr Lys Pro Arg Glu Glu Gln Tyr Asn Ser Thr Tyr
65                  70                  75                  80

Arg Val Val Ser Val Leu Thr Val Leu His Gln Asp Trp Leu Asn Gly
                85                  90                  95

Lys Glu Tyr Lys Cys Lys Val Ser Asn Lys Ala Leu Pro Ala Pro Ile
            100                 105                 110

Glu Lys Thr Ile Ser Lys Ala Lys
        115                 120

## Description

The current invention is in the field of target binding molecules. Herein is reported a single chain binder comprising a spacer domain which is located in between a first fragment of a binding domain and a second fragment of a binding domain.

### Background of the Invention

Since the development of the first monoclonal antibodies by Koehler and Milstein in 1974 a lot of efforts have been dedicated to the development of antibodies which are appropriate for therapy in humans. The first monoclonal antibodies which became available had been developed in mice and rats. These antibodies when used for therapy of a human being caused unwanted side effects due to anti-rodent antibodies. A lot of efforts have been dedicated to the reduction or even elimination of such unwanted side effects.

In the past years an ever growing number of human monoclonal antibodies or humanized monoclonal antibodies have reached the market. Well-known examples include for example Herceptin® and MabThera® from Hoffmann-La Roche, Basel.

Furthermore new antibody formats derived from the wild-type four chain Y-shaped antibody format have been developed. These formats are mainly bi- and multispecific formats. For a review see e.g. Kontermann, R., mAbs 4 (2012) 182-197.

In US 2009/0175867 a single-chain multivalent binding proteins with effector function is reported.

In WO 2014/131711 are reported bispecific antibodies wherein the second and the third antigen binding moiety may be fused to the Fc domain directly or through an immunoglobulin hinge region.

In EP 15176083 a novel antibody format having reduced molecular weight in comparison to a full-length antibody and use thereof is reported.

WO 2007/048022 discloses antibody-polypeptide fusion proteins and methods for producing and using same.

WO 2007/146968 discloses single-chain multivalent binding proteins with effector function.

EP 1 378 520 discloses a cyclic single strand trispecific antibody.

### Summary of the Invention

Herein is reported a single circular polypeptide as a novel target binder. In this circular polypeptide the N-terminal portion comprises a first part of a binding site and the C-terminal portion comprises the second part of the binding site. The first part of the binding site and the second part of the binding site associate with each other to form the complete or functional binding site. Thereby the polypeptide is circularized. The association can be non-covalently or covalently. In case the association is covalently it is not by a peptide bond, but, e.g. by a disulfide bond.

One aspect as reported herein is a (circular) (single chain) fusion polypeptide that (specifically) binds to a target comprising a first part of a binding domain, a second part of a binding domain and a spacer domain, wherein
- the spacer domain is a polypeptide (forming a structural domain after folding),
- the first part of the binding domain is a polypeptide and is fused (either directly or) via a first (peptidic) linker to the N-terminus of the spacer domain,
- the second part of the binding domain is a polypeptide and is fused (either directly or) via a second (peptidic) linker to the C-terminus of the spacer domain,
- the first part of the binding domain and the second part of the binding domain (of the same (single chain) fusion polypeptide) (associate/are associated with/covalently or non-covalently bind to each other and) form a (functional) binding site that specifically binds to the target.

In one embodiment the (circular) (single chain) fusion polypeptide comprises exactly one part of the binding domain N-terminal to the spacer domain and exactly one, but different, part of the same binding domain C-terminal to the spacer domain.

In one embodiment the first part of the binding domain is an antibody heavy chain variable domain and the second part of the binding domain is an antibody light chain variable domain or vice versa.

In one embodiment the first part of the binding domain and the second part of the binding domain are covalently associated with each other. In one embodiment the first part of the binding domain and the second part of the binding domain are covalently associated with each other by a bond other than a peptide bond. In one preferred embodiment the first part of the binding domain and the second part of the binding domain are covalently associated with each other by a disulfide bond.

In one embodiment the first part of the binding domain is an antibody heavy chain Fab fragment (VH+CH1) and the second part of the binding domain is an antibody light chain Fab fragment (VL+CL) or vice versa.

In one embodiment the binding site does not comprise a pair of an antibody heavy chain variable domain and an antibody light chain variable domain/is free of antibody variable domains.

In one preferred embodiment the spacer domain comprises at least 25 amino acid residues. In one embodiment the spacer domain comprises at least 50 amino acid residues. In one embodiment the spacer domain comprises at least 100 amino acid residues.

In one embodiment the (circular) (single chain) fusion polypeptide excerts effector function. In one embodiment the effector function is ADCC or/and CDC.

In one embodiment the spacer domain comprises an antibody hinge region or a fragment thereof and an antibody CH2 domain or a fragment thereof. In one embodiment the hinge region and the antibody CH2 domain or the fragments thereof are of the human IgG1 subclass. In one embodiment the hinge region and the antibody CH2 domain have the amino acid sequence of SEQ ID NO: 105.

In one embodiment the spacer domain comprises an antibody hinge region or a fragment thereof, an antibody CH2 domain, and an antibody CH3 domain or a fragment thereof. In one embodiment the hinge region, the antibody CH2 domain, and the antibody CH3 domain, or the fragments thereof are of the human IgG1 subclass. In one embodiment the hinge region, the antibody CH2 domain, and the antibody CH3 domain have an amino acid sequence selected from the group consisting of SEQ ID NO: 31 to 51. In one preferred embodiment the hinge region, the antibody CH2 domain, and the antibody CH3 domain have an amino acid sequence of SEQ ID NO: 32 or SEQ ID NO: 33 or SEQ ID NO: 38 or SEQ ID NO: 39 or SEQ ID NO: 40 or SEQ ID NO: 41.

In one embodiment the first and/or the second linker is a peptidic linker.

In one embodiment the (circular) (single chain) fusion polypeptide that (specifically) binds to a target comprises a first part of a binding domain, a second part of a binding domain and a spacer domain, wherein
- the spacer domain has an amino acid sequence selected from the group consisting of SEQ ID NO: 32, SEQ ID NO: 33, SEQ ID NO: 38, SEQ ID NO: 39, SEQ ID NO: 40, and SEQ ID NO: 41,
- the first part of the binding domain is an antibody heavy chain Fab fragment and the second part of the binding domain is an antibody light chain Fab fragment or vice versa,
- the first part of the binding domain is fused via a first peptidic linker of SEQ ID NO: 64 or SEQ ID NO: 65 to the N-terminus of the spacer domain and the second part of the binding domain is fused via a second peptidic linker of SEQ ID NO: 64 or SEQ ID NO: 65 to the C-terminus of the spacer domain, whereby the first and the second peptidic linker are selected independently of each other,
   and
- the antibody heavy chain Fab fragment and the antibody light chain Fab fragment (associate with each other to) form a (functional) binding site that (specifically) binds to a target.

In one embodiment the (circular) (single chain) fusion polypeptide comprises in N-to C-terminal direction before the spacer domain (i.e. N-terminal to the spacer domain) exactly one antibody variable domain and after the spacer domain (i.e. C-terminal to the spacer domain) exactly one antibody variable domain.

In one embodiment the target is a cell surface antigen or the soluble ligand of a cell surface receptor.

In one embodiment the binding domain is a conventional Fab, a CrossFab or a DutaFab.

In one embodiment the binding domain is a conventional Fab, wherein one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL), or vice versa.

In one embodiment one part of the binding domain comprises in N- to C-terminal direction VH-CH1 and the other part of the binding domain comprises in N- to C-terminal direction VL-CL, or vice versa.

In one embodime the binding domain is a CrossFab, wherein both parts of the binding domain comprise an antibody variable domain and at least an N-terminal fragment of a (or a complete) antibody constant domain, whereby the pairs of variable domain and constant domain are not naturally associated with each other and have a domain cross-over/exchange of a heavy chain domain and a light chain domain. In one embodiment the exchange is VH with VL or CH1 with CL.

In one embodiment one part of the binding domain comprises in N- to C-terminal direction VL-CH1 and the other part of the binding domain comprises in N- to C-terminal direction VH-CL.

In one embodiment one part of the binding domain comprises in N- to C-terminal direction VH-CL and the other part of the binding domain comprises in N- to C-terminal direction VL-CH1.

The association of the (cognate) parts of the binding domain can further be promoted beside the domain exchange in the CrossFab by the introduction of charges. In certain cases the (multicircular) (dimeric circular) fusion polypeptide comprises at least a first (circular) fusion polypeptide and a second (circular) fusion polypeptide.

In one embodiment the (multicircular) fusion polypeptide comprises
a) a first (circular) fusion polypeptide comprising as binding site a Fab (specifically) binding to a first antigen, and
b) a second (circular) fusion polypeptide comprising as binding site a Fab specifically binding to a second antigen, wherein the variable domains VL and VH in the Fab (of the second circular fusion polypeptide) are replaced by each other.

The (circular) fusion polypeptide under a) does not contain a modification as reported under b).

In the (circular) fusion polypeptide under b)
within the antibody light chain fragment
the variable light chain domain VL is replaced by the variable heavy chain domain VH of said Fab,
and
within the antibody heavy chain fragment
the variable heavy chain domain VH is replaced by the variable light chain domain VL of said Fab.

In one embodiment
i) in the constant domain CL of the first (circular) fusion polypeptide (of the multicircular fusion polypeptide) the amino acid at the position corresponding to position 124 according to Kabat is substituted by a positively charged amino acid, and wherein in the constant domain CH1 of the first (circular) fusion polypeptide (of the multicircular fusion polypeptide) the amino acid at the position corresponding to position 147 according to Kabat EU index or the amino acid at the position corresponding to position 213 according to Kabat EU index is substituted by a negatively charged amino acid,
   or
ii) in the constant domain CL of the second (circular) fusion polypeptide (of the multicircular fusion polypeptide) the amino acid at the position corresponding to position 124 according to Kabat is substituted by a positively charged amino acid, and wherein in the constant domain CH1 of the second (circular) fusion polypeptide (of the multicircular fusion polypeptide) the amino acid at the position corresponding to position 147 according to Kabat EU index or the amino acid at the position corresponding to position 213 according to Kabat EU index is substituted by a negatively charged amino acid.

In one preferred embodiment
i) in the constant domain CL of the first (circular) fusion polypeptide (of the multicircular fusion polypeptide) the amino acid at the position corresponding to position 124 according to Kabat is substituted independently by lysine (K), arginine (R) or histidine (H) (in one preferred embodiment independently by lysine (K) or arginine (R)), and wherein in the constant domain CH1 of the first (circular) fusion polypeptide (of the multicircular fusion polypeptide) the amino acid at the position corresponding to position 147 according to Kabat EU index or the amino acid at the position corresponding to position 213 according to Kabat EU index is substituted independently by glutamic acid (E) or aspartic acid (D),
   or
ii) in the constant domain CL of the second (circular) fusion polypeptide (of the multicircular fusion polypeptide) the amino acid at the position corresponding to position 124 according to Kabat is substituted independently by lysine (K), arginine (R) or histidine (H) (in one preferred embodiment independently by lysine (K) or arginine (R)), and wherein in the constant domain CH1 of the second (circular) fusion polypeptide (of the multicircular fusion polypeptide) the amino acid at the position corresponding to position 147 according to Kabat EU index or the amino acid at the position corresponding to position 213 according to Kabat EU index is substituted independently by glutamic acid (E) or aspartic acid (D).

In one embodiment in the constant domain CL of the second (circular) fusion polypeptide (of the multicircular fusion polypeptide) the amino acids at the positions corresponding to positions 124 and 123 according to the Kabat EU index are substituted by K.

In one embodiment in the constant domain CH1 of the second (circular) fusion polypeptide (of the multicircular fusion polypeptide) the amino acids at the positions corresponding to positions 147 and 213 according to the Kabat EU index are substituted by E.

In one embodiment the binding domain is a DutaFab, wherein one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL), wherein the binding domain comprises two non-overlapping paratopes in the complementary pair of a heavy chain variable domain (VH) and a light chain variable domain (VL), wherein the first paratope comprises residues from CDR1 and CDR3 of the VL domain and CDR2 of the VH domain, and the second paratope comprises residues from CDR1 and CDR3 of the VH domain and CDR2 of the VL domain.

One aspect as reported herein is a (dicircular) fusion polypeptide comprising a first (circular) fusion polypeptide as reported herein and a second (circular) fusion polypeptide as reported herein, wherein the first and the second (circular) fusion polypeptides are identical or different and wherein the spacer domain of the first (circular) fusion polypeptide is (covalently) conjugated to the spacer domain of the second (circular) fusion polypeptide.

In one embodiment the (dicircular) fusion polypeptide comprises
- a first (circular) (single chain) fusion polypeptide that (specifically) binds to a first target comprising a first part of a binding domain, a second part of a binding domain and a spacer domain, wherein
   - the spacer domain has an amino acid sequence of SEQ ID NO: 32 or SEQ ID NO: 38 or SEQ ID NO: 40,
   - the first part of the binding domain is an antibody heavy chain Fab fragment and the second part of the binding domain is an antibody light chain Fab fragment or vice versa,
   - the first part of the binding domain is fused via a first peptidic linker of SEQ ID NO: 64 or SEQ ID NO: 65 to the N-terminus of the spacer domain and the second part of the binding domain is fused via a second peptidic linker of SEQ ID NO: 64 or SEQ ID NO: 65 to the C-terminus of the spacer domain, whereby the first and the second peptidic linker are selected independently of each other,
      and
   - the antibody heavy chain Fab fragment and the antibody light chain Fab (fragment associate with each other to) form a (functional) binding site that (specifically) binds to the first target
   and
- a second (circular) (single chain) fusion polypeptide that (specifically) binds to a second target comprising a first part of a binding domain, a second part of a binding domain and a spacer domain, wherein
   - the spacer domain has an amino acid sequence of SEQ ID NO: 33 or SEQ ID NO: 39 or SEQ ID NO: 41,
   - the first part of the binding domain is an antibody heavy chain Fab fragment and the second part of the binding domain is an antibody light chain Fab fragment or vice versa,
   - the first part of the binding domain is fused via a first peptidic linker of SEQ ID NO: 64 or SEQ ID NO: 65 to the N-terminus of the spacer domain and the second part of the binding domain is fused via a second peptidic linker of SEQ ID NO: 64 or SEQ ID NO: 65 to the C-terminus of the spacer domain, whereby the first and the second peptidic linker are selected independently of each other,
      and
   - the antibody heavy chain Fab fragment and the antibody light chain Fab fragment (associate with each other to) form a (functional) binding site that (specifically) binds to the second target.

In one embodiment each of the (circular) (single chain) fusion polypeptides comprises in N- to C-terminal direction before the spacer domain (i.e. N-terminal to the spacer domain) exactly one antibody variable domain and after the spacer domain (i.e. C-terminal to the spacer domain) exactly one antibody variable domain.

In one embodiment the target is a cell surface antigen or the soluble ligand of a cell surface receptor.

In one embodiment the binding domain is a conventional Fab, a CrossFab or a DutaFab.

In one embodiment one or each of the binding domains is a conventional Fab, wherein one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL).

In one embodiment one part of the binding domain comprises in N- to C-terminal direction VH-CH1 and the other part of the binding domain comprises in N- to C-terminal direction VL-CL, or vice versa.

In one embodiment one or each of the binding domain is a CrossFab, wherein both parts of the binding domain comprise an antibody variable domain and at least an N-terminal fragment of a (or a complete) antibody constant domain, whereby the pairs of variable domain and constant domain are not naturally associated with each other and have a domain cross-over/exchange of a heavy chain domain and a light chain domain. In one embodiment the exchange is VH with VL or CH1 with CL.

In one embodiment one part of the binding domain comprises in N- to C-terminal direction VL-CH1 and the other part of the binding domain comprises in N- to C-terminal direction VH-CL.

In one embodiment one part of the binding domain comprises in N- to C-terminal direction VH-CL and the other part of the binding domain comprises in N- to C-terminal direction VL-CH1.

The association of the cognate binding domains can further be promoted beside the domain exchange in the CrossFab by the introduction of charges. In this case the (multicircular) (dimeric circular) fusion polypeptide comprises at least a first (circular) fusion polypeptide and a second (circular) fusion polypeptide.

In one embodiment the (multicircular) fusion polypeptide comprises
a) a first (circular) fusion polypeptide comprising as binding site a Fab (specifically) binding to a first antigen, and
b) a second (circular) fusion polypeptide comprising as binding site a Fab (specifically) binding to a second antigen, wherein the variable domains VL and VH in the Fab (of the second (circular) fusion polypeptide) are replaced by each other.

The (circular) fusion polypeptide under a) does not contain a modification as reported under b).

In the (circular) fusion polypeptide under b)
within the antibody light chain fragment
the variable light chain domain VL is replaced by the variable heavy chain domain VH of said Fab,
and
within the antibody heavy chain fragment
the variable heavy chain domain VH is replaced by the variable light chain domain VL of said Fab.

In one embodiment
i) in the constant domain CL of the first (circular) fusion polypeptide (of the multicircular fusion polypeptide) the amino acid at the position corresponding to position 124 according to Kabat is substituted by a positively charged amino acid, and wherein in the constant domain CH1 of the first (circular) fusion polypeptide (of the multicircular fusion polypeptide) the amino acid at the position corresponding to position 147 according to Kabat EU index or the amino acid at the position corresponding to position 213 according to Kabat EU index is substituted by a negatively charged amino acid,
   or
ii) in the constant domain CL of the second (circular) fusion polypeptide (of the multicircular fusion polypeptide) the amino acid at the position corresponding to position 124 according to Kabat is substituted by a positively charged amino acid, and wherein in the constant domain CH1 of the second (circular) fusion polypeptide (of the multicircular fusion polypeptide) the amino acid at the position corresponding to position 147 according to Kabat EU index or the amino acid at the position corresponding to position 213 according to Kabat EU index is substituted by a negatively charged amino acid.

In one preferred embodiment
i) in the constant domain CL of the first (circular) fusion polypeptide (of the multicircular fusion polypeptide) the amino acid at the position corresponding to position 124 according to Kabat is substituted independently by lysine (K), arginine (R) or histidine (H) (in one preferred embodiment independently by lysine (K) or arginine (R)), and wherein in the constant domain CH1 of the first (circular) fusion polypeptide (of the multicircular fusion polypeptide) the amino acid at the position corresponding to position 147 according to Kabat EU index or the amino acid at the position corresponding to position 213 according to Kabat EU index is substituted independently by glutamic acid (E) or aspartic acid (D),
   or
ii) in the constant domain CL of the second (circular) fusion polypeptide (of the multicircular fusion polypeptide) the amino acid at the position corresponding to position 124 according to Kabat is substituted independently by lysine (K), arginine (R) or histidine (H) (in one preferred embodiment independently by lysine (K) or arginine (R)), and wherein in the constant domain CH1 of the second (circular) fusion polypeptide (of the multicircular fusion polypeptide) the amino acid at the position corresponding to position 147 according to Kabat EU index or the amino acid at the position corresponding to position 213 according to Kabat EU index is substituted independently by glutamic acid (E) or aspartic acid (D).

In one embodiment in the constant domain CL of the second (circular) fusion polypeptide (of the multicircular fusion polypeptide) the amino acids at the positions corresponding to positions 124 and 123 according to the Kabat EU index are substituted by K.

In one embodiment in the constant domain CH1 of the second (circular) fusion polypeptide (of the multicircular fusion polypeptide) the amino acids at the positions corresponding to positions 147 and 213 according to the Kabat EU index are substituted by E.

In one embodiment one or each of the binding domain is a DutaFab, wherein one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL), wherein the binding domain comprises two non-overlapping paratopes in the complementary pair of a heavy chain variable domain (VH) and a light chain variable domain (VL), wherein the first paratope comprises residues from CDR1 and CDR3 of the VL domain and CDR2 of the VH domain, and the second paratope comprises residues from CDR1 and CDR3 of the VH domain and CDR2 of the VL domain.One aspect as reported herein is an isolated nucleic acid encoding the circular fusion polypeptide as reported herein.

One aspect as reported herein is a pair of isolated nucleic acids together encoding the dimeric (circular) fusion polypeptide as reported herein.

One aspect as reported herein is a host cell comprising the nucleic acid as reported herein or the pair of nucleic acids as reported herein.

One aspect as reported herein is a method of producing a (circular or dicircular) fusion polypeptide comprising culturing the host cell as reported herein so that the (circular or dicircular) fusion polypeptide is produced and recovering the (circular or dicircular) fusion polypeptide from the cell or the cultivation medium.

One aspect as reported herein is an immunoconjugate comprising the (circular) fusion polypeptide as reported herein and a cytotoxic agent.

One aspect as reported herein is a pharmaceutical formulation comprising the (circular) fusion polypeptide as reported herein or the (dimeric) (circular) fusion polypeptide as reported herein and a pharmaceutically acceptable carrier.

One aspect as reported herein is the (circular) fusion polypeptide as reported herein or the dimeric (circular) fusion polypeptide as reported herein for use as a medicament.

One aspect as reported herein is the use of the (circular) fusion polypeptide as reported herein or the dimeric (circular) fusion polypeptide as reported herein in the manufacture of a medicament.

### Detailed Description of the Invention

### I. Definitions

The knobs into holes dimerization modules and their use in antibody engineering are described in Carter P.; Ridgway J.B.B.; Presta L.G.: Immunotechnology, Volume 2, Number 1, February 1996 , pp. 73-73(1).

General information regarding the nucleotide sequences of human immunoglobulins light and heavy chains is given in: Kabat, E.A., et al., Sequences of Proteins of Immunological Interest, 5th ed., Public Health Service, National Institutes of Health, Bethesda, MD (1991).

As used herein, the amino acid positions of all constant regions and domains of the heavy and light chain are numbered according to the Kabat numbering system described in Kabat, et al., Sequences of Proteins of Immunological Interest, 5th ed., Public Health Service, National Institutes of Health, Bethesda, MD (1991) and is referred to as "numbering according to Kabat" herein. Specifically, the Kabat numbering system (see pages 647-660) of Kabat, et al., Sequences of Proteins of Immunological Interest, 5th ed., Public Health Service, National Institutes of Health, Bethesda, MD (1991) is used for the light chain constant domain CL of kappa and lambda isotype, and the Kabat EU index numbering system (see pages 661-723) is used for the constant heavy chain domains (CH1, Hinge, CH2 and CH3, which is herein further clarified by referring to "numbering according to Kabat EU index" in this case).

Useful methods and techniques for carrying out the current invention are described in e.g. Ausubel, F.M. (ed.), Current Protocols in Molecular Biology, Volumes I to III (1997); Glover, N.D., and Hames, B.D., ed., DNA Cloning: A Practical Approach, Volumes I and II (1985), Oxford University Press; Freshney, R.I. (ed.), Animal Cell Culture - a practical approach, IRL Press Limited (1986); Watson, J.D., et al., Recombinant DNA, Second Edition, CHSL Press (1992); Winnacker, E.L., From Genes to Clones; N.Y., VCH Publishers (1987); Celis, J., ed., Cell Biology, Second Edition, Academic Press (1998); Freshney, R.I., Culture of Animal Cells: A Manual of Basic Technique, second edition, Alan R. Liss, Inc., N.Y. (1987).

The use of recombinant DNA technology enables the generation of derivatives of a nucleic acid. Such derivatives can, for example, be modified in individual or several nucleotide positions by substitution, alteration, exchange, deletion or insertion. The modification or derivatization can, for example, be carried out by means of site directed mutagenesis. Such modifications can easily be carried out by a person skilled in the art (see e.g. Sambrook, J., et al., Molecular Cloning: A laboratory manual (1999) Cold Spring Harbor Laboratory Press, New York, USA; Hames, B.D., and Higgins, S.G., Nucleic acid hybridization - a practical approach (1985) IRL Press, Oxford, England).

It must be noted that as used herein and in the appended claims, the singular forms "a", "an", and "the" include plural reference unless the context clearly dictates otherwise. Thus, for example, reference to "a cell" includes a plurality of such cells and equivalents thereof known to those skilled in the art, and so forth. As well, the terms "a" (or "an"), "one or more" and "at least one" can be used interchangeably herein. It is also to be noted that the terms "comprising", "including", and "having" can be used interchangeably.

The term "about" denotes a range of +/- 20 % of the thereafter following numerical value. In one embodiment the term about denotes a range of +/- 10 % of the thereafter following numerical value. In one embodiment the term about denotes a range of +/-5 % of the thereafter following numerical value.

"Affinity" refers to the strength of the sum total of non-covalent interactions between a single binding site of a molecule (e.g., an antibody) and its binding partner (e.g., an antigen). Unless indicated otherwise, as used herein, "binding affinity" refers to intrinsic binding affinity which reflects a 1:1 interaction between members of a binding pair (e.g., antibody and antigen). The affinity of a molecule X for its partner Y can generally be represented by the dissociation constant (kd). Affinity can be measured by common methods known in the art, including those described herein.

The term "amino acid sequence tag" denotes a sequence of amino acid residues connected to each other via peptide bonds that has specific binding properties. In one embodiment the amino acid sequence tag is an affinity or purification tag. In one embodiment the amino acid sequence tag is selected from Arg-tag, His-tag, Flag tag, 3xFlag-tag, Strep-tag, Nano-tag, SBP-tag, c-myc-tag, S-tag, calmodulin binding-peptide, cellulose-binding-domain, chitin-binding-domain, GST-tag, or MBP-tag. In one embodiment the amino acid sequence tag is selected from SEQ ID NO: 01 (RRRRR), or SEQ ID NO: 02 (RRRRRR), or SEQ ID NO: 03 (HHHHHH), or SEQ ID NO: 04 (KDHLIHNVHK EFHAHAHNK), or SEQ ID NO: 05 (DYKDDDDK), or SEQ ID NO: 06 (DYKDHDGDYK DHDIDYKDDD DK), or SEQ ID NO: 07 (AWRHPQFGG), or SEQ ID NO: 08 (WSHPQFEK), or SEQ ID NO: 09 (MDVEAWLGAR), or SEQ ID NO: 10 (MDVEAWLGAR VPLVET), or SEQ ID NO: 11 (MDEKTTGWRG GHVVEGLAGE LEQLRARLEH HPQGQREP), or SEQ ID NO: 12 (EQKLISEEDL), or SEQ ID NO: 13 (KETAAAKFER QHMDS), or SEQ ID NO: 14 (KRRWKKNFIA VSAANRFKKI SSSGAL), or SEQ ID NO: 15 (cellulose binding domain), or SEQ ID NO: 16 (cellulose binding domain), or SEQ ID NO: 17 (TNPGVSAWQV NTAYTAGQLV TYNGKTYKCL QPHTSLAGWE PSNVPALWQL Q), or SEQ ID NO: 18 (GST-tag) or SEQ ID NO: 19 (MBP-tag).

The term "amino acid substitution" denotes the replacement of at least one amino acid residue in a predetermined parent amino acid sequence with a different "replacement" amino acid residue. The replacement residue or residues may be a "naturally occurring amino acid residue" (i.e. encoded by the genetic code) and selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). In one embodiment the replacement residue is not cysteine. Substitution with one or more non-naturally occurring amino acid residues is also encompassed by the definition of an amino acid substitution herein. A "non-naturally occurring amino acid residue" denotes a residue, other than those naturally occurring amino acid residues listed above, which is able to covalently bind adjacent amino acid residues(s) in a polypeptide chain. Examples of non-naturally occurring amino acid residues include norleucine, ornithine, norvaline, homoserine, aib and other amino acid residue analogues such as those described in Ellman, et al., Meth. Enzym. 202 (1991) 301-336. To generate such non-naturally occurring amino acid residues, the procedures of Noren, et al. (Science 244 (1989) 182) and/or Ellman, et al. (supra) can be used. Briefly, these procedures involve chemically activating a suppressor tRNA with a non-naturally occurring amino acid residue followed by in vitro transcription and translation of the RNA. Non-naturally occurring amino acids can also be incorporated into peptides via chemical peptide synthesis and subsequent fusion of these peptides with recombinantly produced polypeptides, such as antibodies or antibody fragments.

The term "antibody-dependent cellular cytotoxicity (ADCC)" is a function mediated by Fc receptor binding and refers to lysis of target cells mediated by an antibody Fc-region in the presence of effector cells. ADCC is measured in one embodiment by the treatment of a preparation of target expressing erythroid cells (e.g. K562 cells expressing recombinant target) with an Fc-region comprising multicircular fusion polypeptide as reported herein in the presence of effector cells such as freshly isolated PBMC (peripheral blood mononuclear cells) or purified effector cells from buffy coats, like monocytes or NK (natural killer) cells. Target cells are labeled with Cr-51 and subsequently incubated with the multicircular fusion polypeptide. The labeled cells are incubated with effector cells and the supernatant is analyzed for released Cr-51. Controls include the incubation of the target endothelial cells with effector cells but without the multicircular fusion polypeptide. The capacity of the multicircular fusion polypeptide to induce the initial steps mediating ADCC is investigated by measuring the binding to Fcγ receptors expressing cells, such as cells, recombinantly expressing FcγRI and/or FcγRIIA or NK cells (expressing essentially FcγRIIIA). In one preferred embodiment binding to FcγR on NK cells is measured.

The term "binding to" denotes the binding of a binding site to its target, such as e.g. of an antibody binding site comprising an antibody heavy chain variable domain and an antibody light chain variable domain to the respective antigen. This binding can be determined using, for example, a BIAcore® assay (GE Healthcare, Uppsala, Sweden).

For example, in one possible embodiment of the BIAcore® assay the antigen is bound to a surface and binding of the antibody binding site is measured by surface plasmon resonance (SPR). The affinity of the binding is defined by the terms ka (association constant: rate constant for the association to form a complex), kd (dissociation constant; rate constant for the dissociation of the complex), and KD (kd/ka). Alternatively, the binding signal of a SPR sensorgram can be compared directly to the response signal of a reference, with respect to the resonance signal height and the dissociation behaviors.

The term "CH1 domain" denotes the part of an antibody heavy chain polypeptide that extends approximately from EU position 118 to EU position 215 (EU numbering system). In one embodiment a CH1 domain has the amino acid sequence of ASTKGPSVFP LAPSSKSTSG GTAALGCLVK DYFPEPVTVS WNSGALTSGV HTFPAVLQSS GLYSLSSVVT VPSSSLGTQT YICNVNHKPS NTKVDKKV(SEQ ID NO: 20).

The term "CH2 domain" denotes the part of an antibody heavy chain polypeptide that extends approximately from EU position 231 to EU position 340 (EU numbering system according to Kabat). In one embodiment a CH2 domain has the amino acid sequence of APELLGGPSV FLFPPKPKDT LMISRTPEVT CVWDVSHEDP EVKFNWYVDG VEVHNAKTKP REEQESTYRW SVLTVLHQDW LNGKEYKCKV SNKALPAPIE KTISKAK (SEQ ID NO: 21). The CH2 domain is unique in that it is not closely paired with another domain. Rather, two N-linked branched carbohydrate chains are interposed between the two CH2 domains of an intact native Fc-region. It has been speculated that the carbohydrate may provide a substitute for the domain-domain pairing and help stabilize the CH2 domain. Burton, Mol. Immunol. 22 (1985) 161-206.

The term "CH3 domain" denotes the part of an antibody heavy chain polypeptide that extends approximately from EU position 341 to EU position 446. In one embodiment the CH3 domain has the amino acid sequence of GQPREPQVYT LPPSRDELTK NQVSLTCLVK GFYPSDIAVE WESNGQPENN YKTTPPVLDS DGSFFLYSKL TVDKSRWQQG NVFSCSVMHE ALHNHYTQKS LSLSPG(SEQID NO: 22).

The "class" of an antibody or an Fc-region refers to the type of constant domain or constant region possessed by the heavy chains or fragments thereof. There are five major classes: IgA, IgD, IgE, IgG, and IgM, and several of these may be further divided into subclasses (isotypes), e.g., IgG1, IgG2, IgG3, IgG4, IgA1, and IgA2. The heavy chain constant domains that correspond to the different classes of immunoglobulins are called α, δ, ε, γ, and µ, respectively.

The term "cytotoxic agent" as used herein refers to a substance that inhibits or prevents a cellular function and/or causes cell death or destruction. Cytotoxic agents include, but are not limited to, radioactive isotopes (e.g., At-211, 1-131, 1-125, Y-90, Re-186, Re-188, Sm-153, Bi-212, P-32, Pb-212 and radioactive isotopes of Lu); chemotherapeutic agents or drugs (e.g., methotrexate, adriamicin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "complement-dependent cytotoxicity (CDC)" refers to lysis of cells induced by the Fc-region of an antibody as reported herein in the presence of complement. CDC is measured in one embodiment by the treatment of target expressing human endothelial cells with a multicircular fusion polypeptide as reported herein in the presence of complement. The cells are in one embodiment labeled with calcein. CDC is found if the multicircular fusion polypeptide induces lysis of 20 % or more of the target cells at a concentration of 30 µg/ml. Binding to the complement factor C1q can be measured in an ELISA. In such an assay in principle an ELISA plate is coated with concentration ranges of the multicircular fusion polypeptide, to which purified human C1q or human serum is added. C1q binding is detected by an antibody directed against C1q followed by a peroxidase-labeled conjugate. Detection of binding (maximal binding Bmax) is measured as optical density at 405 nm (OD405) for peroxidase substrate ABTS® (2,2'-azino-di-[3-ethylbenzthiazoline-6-sulfonate]).

The term "domain crossover" as used herein denotes that in a pair of an antibody heavy chain VH-CH1 fragment and its corresponding cognate antibody light chain, i.e. in an antibody binding arm (i.e. in the Fab fragment), the domain sequence deviates from the natural sequence in that at least one heavy chain domain is substituted by its corresponding light chain domain and vice versa. There are three general types of domain crossovers, (i) the crossover of the CH1 and the CL domains, which leads to domain crossover light chain with a VL-CH1 domain sequence and a domain crossover heavy chain fragment with a VH-CL domain sequence (or a full length antibody heavy chain with a VH-CL-hinge-CH2-CH3 domain sequence), (ii) the domain crossover of the VH and the VL domains, which leads to domain crossover light chain with a VH-CL domain sequence and a domain crossover heavy chain fragment with a VL-CH1 domain sequence, and (iii) the domain crossover of the complete light chain (VL-CL) and the complete VH-CH1 heavy chain fragment ("Fab crossover"), which leads to a domain crossover light chain with a VH-CH1 domain sequence and a domain crossover heavy chain fragment with a VL-CL domain sequence (all aforementioned domain sequences are indicated in N-terminal to C-terminal direction).

As used herein the term "replaced by each other" with respect to corresponding heavy and light chain domains refers to the aforementioned domain crossovers. As such, when CH1 and CL domains are "replaced by each other" it is referred to the domain crossover mentioned under item (i) and the resulting heavy and light chain domain sequence. Accordingly, when VH and VL are "replaced by each other" it is referred to the domain crossover mentioned under item (ii); and when the CH1 and CL domains are "replaced by each other" and the VH1 and VL domains are "replaced by each other" it is referred to the domain crossover mentioned under item (iii). Bispecific antibodies including domain crossovers are reported, e.g. in WO 2009/080251, WO 2009/080252, WO 2009/080253, WO 2009/080254 and Schaefer, W. et al, Proc. Natl. Acad. Sci USA 108 (2011) 11187-11192.

The multispecific antibody produced with a method as reported herein essentially comprises Fab fragments including a domain crossover of the CH1 and the CL domains as mentioned under item (i) above, or a domain crossover of the VH and the VL domains as mentioned under item (ii) above. The Fab fragments specifically binding to the same antigen(s) are constructed to be of the same domain sequence. Hence, in case more than one Fab fragment with a domain crossover is contained in the multispecific antibody, said Fab fragment(s) specifically bind to the same antigen.

"Effector functions" refer to those biological activities attributable to the Fc-region of an antibody, which vary with the antibody class from which it is derived. Examples of antibody effector functions include: C1q binding and complement dependent cytotoxicity (CDC); Fc receptor binding; antibody-dependent cell-mediated cytotoxicity (ADCC); phagocytosis; down regulation of cell surface receptors (e.g. B-cell receptor); and B-cell activation.

Fc receptor binding dependent effector functions can be mediated by the interaction of the Fc-region of an antibody with Fc receptors (FcRs), which are specialized cell surface receptors on hematopoietic cells. Fc receptors belong to the immunoglobulin superfamily, and have been shown to mediate both the removal of antibody-coated pathogens by phagocytosis of immune complexes, and the lysis of erythrocytes and various other cellular targets (e.g. tumor cells) presenting the Fc-region, via antibody dependent cell mediated cytotoxicity (ADCC) (see e.g. Van de Winkel, J.G. and Anderson, C.L., J. Leukoc. Biol. 49 (1991) 511-524). FcRs are defined by their specificity for immunoglobulin isotypes: Fc receptors for IgG type Fc-regions are referred to as FcyR. Fc receptor binding is described e.g. in Ravetch, J.V. and Kinet, J.P., Annu. Rev. Immunol. 9 (1991) 457-492; Capel, P.J., et al., Immunomethods 4 (1994) 25-34; de Haas, M., et al., J. Lab. Clin. Med. 126 (1995) 330-341; Gessner, J.E., et al., Ann. Hematol. 76 (1998) 231-248.

Cross-linking of receptors for the Fc-region of IgG type antibodies (FcyR) triggers a wide variety of effector functions including phagocytosis, antibody-dependent cellular cytotoxicity, and release of inflammatory mediators, as well as immune complex clearance and regulation of antibody production. In humans, three classes of FcyR have been characterized, which are:
- FcγRI (CD64) binds monomeric IgG with high affinity and is expressed on macrophages, monocytes, neutrophils and eosinophils. Modification in the Fc-region IgG at least at one of the amino acid residues E233-G236, P238, D265, N297, A327 and P329 (numbering according to EU index of Kabat) reduce binding to FcyRI. IgG2 residues at positions 233-236, substituted into IgG1 and IgG4, reduced binding to FcyRI by 10³-fold and eliminated the human monocyte response to antibody-sensitized red blood cells (Armour, K.L., et al., Eur. J. Immunol. 29 (1999) 2613-2624).
- FcγRII (CD32) binds complexed IgG with medium to low affinity and is widely expressed. This receptor can be divided into two sub-types, FcγRIIA and FcγRIIB. FcγRIIA is found on many cells involved in killing (e.g. macrophages, monocytes, neutrophils) and seems able to activate the killing process. FcγRIIB seems to play a role in inhibitory processes and is found on B cells, macrophages and on mast cells and eosinophils. On B-cells it seems to function to suppress further immunoglobulin production and isotype switching to, for example, the IgE class. On macrophages, FcγRIIB acts to inhibit phagocytosis as mediated through FcγRIIA. On eosinophils and mast cells the B-form may help to suppress activation of these cells through IgE binding to its separate receptor. Reduced binding for FcγRIIA is found e.g. for antibodies comprising an IgG Fc-region with mutations at least at one of the amino acid residues E233-G236, P238, D265, N297, A327, P329, D270, Q295, A327, R292, and K414 (numbering according to EU index of Kabat).
- FcγRIII (CD16) binds IgG with medium to low affinity and exists as two types. FcγRIIIA is found on NK cells, macrophages, eosinophils and some monocytes and T cells and mediates ADCC. FcγRIIIB is highly expressed on neutrophils. Reduced binding to FcγRIIIA is found e.g. for antibodies comprising an IgG Fc-region with mutation at least at one of the amino acid residues E233-G236, P238, D265, N297, A327, P329, D270, Q295, A327, S239, E269, E293, Y296, V303, A327, K338 and D376 (numbering according to EU index of Kabat).

Mapping of the binding sites on human IgG1 for Fc receptors, the above mentioned mutation sites and methods for measuring binding to FcγRI and FcγRIIA are described in Shields, R.L., et al. J. Biol. Chem. 276 (2001) 6591-6604.

An "effective amount" of an agent, e.g., a pharmaceutical formulation, refers to an amount effective, at dosages and for periods of time necessary, to achieve the desired therapeutic or prophylactic result.

The term "epitope" refers to that part of a given target that is required for specific binding between the target and a binding site. An epitope may be continuous, i.e. formed by adjacent structural elements present in the target, or discontinuous, i.e. formed by structural elements that are at different positions in the primary sequence of the target, such as in the amino acid sequence of a protein as target, but in close proximity in the three-dimensional structure, which the target adopts in a native environment, such as in a bodily fluid.

The term "Fc receptor" as used herein refers to activation receptors characterized by the presence of a cytoplasmatic ITAM sequence associated with the receptor (see e.g. Ravetch, J.V. and Bolland, S., Annu. Rev. Immunol. 19 (2001) 275-290). Such receptors are FcγRI, FcγRIIA and FcγRIIIA. The term "no binding of FcγR" denotes that at an antibody concentration of 10 µg/ml the binding of an antibody as reported herein to NK cells is 10 % or less of the binding found for anti-OX40L antibody LC.001 as reported in WO 2006/029879.

While IgG4 shows reduced FcR binding, antibodies of other IgG subclasses show strong binding. However Pro238, Asp265, Asp270, Asn297 (loss of Fc carbohydrate), Pro329, Leu234, Leu235, Gly236, Gly237, Ile253, Ser254, Lys288 , Thr307, Gln311, Asn434, and His435 are residues which provide if altered also reduce FcR binding (Shields, R.L., et al. J. Biol. Chem. 276 (2001) 6591-6604; Lund, J., et al., FASEB J. 9 (1995) 115-119; Morgan, A., et al., Immunology 86 (1995) 319-324; and EP 0 307 434).

In one embodiment the spacer domain of the circular single chain fusion polypeptide as reported herein is an antibody Fc-region.

In one embodiment the Fc-region in the circular fusion polypeptide as reported herein is of IgG1 or IgG2 subclass and comprises the mutation PVA236, GLPSS331, and/or L234A/L235A/P329G.

In one embodiment the Fc-region in the circular fusion polypeptide as reported herein is of IgG1 subclass and comprises the mutations L234A/L235A. In one embodiment the Fc-region in the circular fusion polypeptide further comprises the mutation P329G. In one embodiment the Fc-region in the circular fusion polypeptide as reported herein is of IgG1 subclass and comprises the mutations L234A/L235A/P329G (all numbering according to EU index of Kabat).

In one embodiment the Fc-region in the circular fusion polypeptide as reported herein is of IgG4 subclass and comprises the mutation L235E. In one embodiment the Fc-region in the circular fusion polypeptide further comprises the mutation S228P. In one embodiment the Fc-region in the circular fusion polypeptide further comprises the mutation P329G. In one embodiment the Fc-region in the circular fusion polypeptide as reported herein is of IgG4 subclass and comprises the mutations S228P/L235E/P329G (all numbering according to EU index of Kabat).

The term "Fc-region" herein is used to define a C-terminal region of an immunoglobulin heavy chain that contains at least a portion of the constant region. The term includes native sequence Fc-regions and variant Fc-regions. In one embodiment, a human IgG heavy chain Fc-region extends from Cys226, or from Pro230, or from Ala231 to the carboxyl-terminus of the heavy chain. However, the C-terminal lysine (Lys447) of the Fc-region may or may not be present.

The circular fusion polypeptides as reported herein may comprise an Fc-region, in one embodiment an Fc-region derived from human origin. In one embodiment the Fc-region comprises all parts of the human constant region. The Fc-region is directly involved in complement activation, C1q binding, C3 activation and Fc receptor binding. Binding to C1q is caused by defined binding sites in the Fc-region. Such binding sites are known in the state of the art and described e.g. by Lukas, T.J., et al., J. Immunol. 127 (1981) 2555-2560; Brunhouse, R., and Cebra, J.J., Mol. Immunol. 16 (1979) 907-917; Burton, D.R., et al., Nature 288 (1980) 338-344; Thommesen, J.E., et al., Mol. Immunol. 37 (2000) 995-1004; Idusogie, E.E., et al., J. Immunol. 164 (2000) 4178-4184; Hezareh, M., et al., J. Virol. 75 (2001) 12161-12168; Morgan, A., et al., Immunology 86 (1995) 319-324; and EP 0 307 434. Such binding sites are e.g. L234, L235, D270, N297, E318, K320, K322, P331 and P329 (numbering according to EU index of Kabat). Antibodies of subclass IgG1, IgG2 and IgG3 usually show complement activation, C1q binding and C3 activation, whereas IgG4 do not activate the complement system, do not bind C1q and do not activate C3. An "Fc-region of an antibody" is a term well known to the skilled artisan and defined on the basis of papain cleavage of antibodies. In one embodiment the Fc-region is a human Fc-region. In one embodiment the Fc-region is of the human IgG4 subclass comprising the mutations S228P and/or L235E and/or P329G (numbering according to EU index of Kabat). In one embodiment the Fc-region is of the human IgG1 subclass comprising the mutations L234A and L235A and optionally P329G (numbering according to EU index of Kabat).

The term "hinge region" denotes the part of an antibody heavy chain polypeptide that joins in a wild-type antibody heavy chain the CH1 domain and the CH2 domain, e. g. from about position 216 to about position 230 according to the EU number system of Kabat, or from about position 226 to about position 230 according to the EU number system of Kabat. The hinge regions of other IgG subclasses can be determined by aligning with the hinge-region cysteine residues of the IgG1 subclass sequence.

The hinge region is normally a dimeric molecule consisting of two polypeptides with identical amino acid sequence. The hinge region generally comprises about 25 amino acid residues and is flexible allowing the associated target binding sites to move independently. The hinge region can be subdivided into three domains: the upper, the middle, and the lower hinge domain (see e.g. Roux, et al., J. Immunol. 161 (1998) 4083).

In one embodiment the hinge region has the amino acid sequence DKTHTCPXCP (SEQ ID NO: 23), wherein X is either S or P. In one embodiment the hinge region has the amino acid sequence HTCPXCP (SEQ ID NO: 24), wherein X is either S or P. In one embodiment the hinge region has the amino acid sequence CPXCP (SEQ ID NO: 25), wherein X is either S or P.

The term "wild-type Fc-region" denotes an amino acid sequence identical to the amino acid sequence of an Fc-region found in nature. Wild-type human Fc-regions include a native human IgG1 Fc-region (non-A and A allotypes), native human IgG2 Fc-region, native human IgG3 Fc-region, and native human IgG4 Fc-region as well as naturally occurring variants thereof. Wild-type Fc-regions are denoted in SEQ ID NO: 26 (IgG1, caucasian allotype), SEQ ID NO: 27 (IgG1, afroamerican allotype), SEQ ID NO: 28 (IgG2), SEQ ID NO: 29 (IgG3) and SEQ ID NO: 30 (IgG4).

The term "variant (human) Fc-region" denotes an amino acid sequence which differs from that of a "wild-type" (human) Fc-region amino acid sequence by virtue of at least one "amino acid mutation". In one embodiment the variant Fc-region has at least one amino acid mutation compared to a native Fc-region, e.g. from about one to about ten amino acid mutations, and in one embodiment from about one to about five amino acid mutations in a native Fc-region. In one embodiment the (variant) Fc-region has at least about 80 % homology with a wild-type Fc-region, and in one embodiment the variant Fc-region has least about 90 % homology, in one embodiment the variant Fc-region has at least about 95 % homology.

Variant (human) Fc-regions are defined by the amino acid mutations that are contained. Thus, for example, the term P329G denotes a variant Fc-region with the mutation of proline to glycine at amino acid position 329 relative to the parent (wild-type) Fc-region (numbering according to EU index of Kabat). The identity of the wild-type amino acid may be unspecified, in which case the aforementioned variant is referred to as 329G. The term "mutation" denotes a change to naturally occurring amino acids as well as a change to non-naturally occurring amino acids (see e.g. US 6,586,207, WO 98/48032, WO 03/073238, US 2004/0214988, WO 2005/35727, WO 2005/74524, Chin, J.W., et al., J. Am. Chem. Soc. 124 (2002) 9026-9027; Chin, J.W. and Schultz, P.G., ChemBioChem 11 (2002) 1135-1137; Chin, J.W., et al., PICAS United States of America 99 (2002) 11020-11024; Wang, L. and Schultz, P.G., Chem. (2002) 1-10).

A polypeptide chain of a wild-type human Fc-region of the IgG1 subclass has the following amino acid sequence starting with a cysteine residue at position 227 and ending with a glycine residue at position 446:

CPPCPAPELL GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR (E/D)E(M/L)TKNQVSL
TCLVKGFYPS DIAVEWESNG QPENNYKTTP PVLDSDGSFF LYSKLTVDKS
RWQQGNVFSC SVMHEALHNH YTQKSLSLSP G(SEQ ID NO: 31).

A polypeptide chain of a variant human Fc-region of the IgG1 subclass with the mutations T366S, L368A and Y407V has the following amino acid sequence:

CPPCPAPELL GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR DELTKNQVSL SCAVKGFYPS
DIAVEWESNG QPENNYKTTP PVLDSDGSFF LVSKLTVDKS RWQQGNVFSC
SVMHEALHNH YTQKSLSLSP G (SEQ ID NO: 32).

A polypeptide chain of a variant human Fc-region of the IgG1 subclass with the mutation T366W has the following amino acid sequence:

CPPCPAPELL GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR DELTKNQVSL WCLVKGFYPS
DIAVEWESNG QPENNYKTTP PVLDSDGSFF LYSKLTVDKS RWQQGNVFSC
SVMHEALHNH YTQKSLSLSP G (SEQ ID NO: 33).

A polypeptide chain of a variant human Fc-region of the IgG1 subclass with the mutations L234A and L235A has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR DELTKNQVSL TCLVKGFYPS
DIAVEWESNG QPENNYKTTP PVLDSDGSFF LYSKLTVDKS RWQQGNVFSC
SVMHEALHNH YTQKSLSLSP G (SEQ ID NO: 34).

A polypeptide chain of a variant human Fc-region of the IgG1 subclass with the mutations L234A, L235A, T366S, L368A and Y407V has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR DELTKNQVSL SCAVKGFYPS
DIAVEWESNG QPENNYKTTP PVLDSDGSFF LVSKLTVDKS RWQQGNVFSC
SVMHEALHNH YTQKSLSLSP G (SEQ ID NO: 35).

A polypeptide chain of a variant human Fc-region of the IgG1 subclass with the mutations L234A, L235A and T366W has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR DELTKNQVSL WCLVKGFYPS
DIAVEWESNG QPENNYKTTP PVLDSDGSFF LYSKLTVDKS RWQQGNVFSC
SVMHEALHNH YTQKSLSLSP G (SEQ ID NO: 36).

A polypeptide chain of a variant human Fc-region of the IgG1 subclass with the mutations L234A, L235A and P329G has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVYTLPPSR DELTKNQVSL TCLVKGFYPS

DIAVEWESNG QPENNYKTTP PVLDSDGSFF LYSKLTVDKS RWQQGNVFSC
SVMHEALHNH YTQKSLSLSP G (SEQ ID NO: 37).

A polypeptide chain of a variant human Fc-region of the IgG1 subclass with the mutations L234A, L235A, P329G, T366S, L368A and Y407V has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVYTLPPSR DELTKNQVSL SCAVKGFYPS
DIAVEWESNG QPENNYKTTP PVLDSDGSFF LVSKLTVDKS RWQQGNVFSC
SVMHEALHNH YTQKSLSLSP G (SEQ ID NO: 38).

A polypeptide chain of a variant human Fc-region of the IgG1 subclass with the mutations L234A, L235A, P329G and T366W has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVYTLPPSR DELTKNQVSL WCLVKGFYPS
DIAVEWESNG QPENNYKTTP PVLDSDGSFF LYSKLTVDKS RWQQGNVFSC
SVMHEALHNH YTQKSLSLSP G (SEQ ID NO: 39).

A polypeptide chain of a variant human Fc-region of the IgG1 subclass with the mutations L234A, L235A, P329G, Y349C, T366S, L368A and Y407V has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVCTLPPSR DELTKNQVSL SCAVKGFYPS
DIAVEWESNG QPENNYKTTP PVLDSDGSFF LVSKLTVDKS RWQQGNVFSC
SVMHEALHNH YTQKSLSLSP G (SEQ ID NO: 40).

A polypeptide chain of a variant human Fc-region of the IgG1 subclass with the mutations L234A, L235A, P329G, S354C and T366W has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVYTLPPCR DELTKNQVSL WCLVKGFYPS

DIAVEWESNG QPENNYKTTP PVLDSDGSFF LYSKLTVDKS RWQQGNVFSC
SVMHEALHNH YTQKSLSLSP G (SEQ ID NO: 41).

A polypeptide chain of a variant human Fc-region of the IgG1 subclass with the mutations L234A, L235A, P329G, S354C, T366S, L368A and Y407V has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVYTLPPCR DELTKNQVSL SCAVKGFYPS
DIAVEWESNG QPENNYKTTP PVLDSDGSFF LVSKLTVDKS RWQQGNVFSC
SVMHEALHNH YTQKSLSLSP G (SEQ ID NO: 42).

A polypeptide chain of a variant human Fc-region of the IgG1 subclass with the mutations L234A, L235A, P329G, Y349C and T366W has the following amino acid sequence:

CPPCPAPEAA GGPSVFLFPP KPKDTLMISR TPEVTCVVVD VSHEDPEVKF
NWYVDGVEVH NAKTKPREEQ YNSTYRVVSV LTVLHQDWLN GKEYKCKVSN
KALGAPIEKT ISKAKGQPRE PQVCTLPPSR DELTKNQVSL WCLVKGFYPS
DIAVEWESNG QPENNYKTTP PVLDSDGSFF LYSKLTVDKS RWQQGNVFSC
SVMHEALHNH YTQKSLSLSP G (SEQ ID NO: 43).

A polypeptide chain of a variant human Fc-region of the IgG1 subclass with the mutations I253A, H310A and H435A has the following amino acid sequence:

CPPCPAPELL GGPSVFLFPP KPKDTLMASR TPEVTCVVVD VSHEDPEVKF
NWYVDGVEVH NAKTKPREEQ YNSTYRVVSV LTVLAQDWLN GKEYKCKVSN
KALPAPIEKT ISKAKGQPRE PQVYTLPPSR DELTKNQVSL TCLVKGFYPS
DIAVEWESNG QPENNYKTTP PVLDSDGSFF LYSKLTVDKS RWQQGNVFSC
SVMHEALHNA YTQKSLSLSP G (SEQ ID NO: 44).

A polypeptide chain of a variant human Fc-region of the IgG1 subclass with the mutations H310A, H433A and Y436A has the following amino acid sequence:

CPPCPAPELL GGPSVFLFPP KPKDTLMISR TPEVTCVVVD VSHEDPEVKF
NWYVDGVEVH NAKTKPREEQ YNSTYRVVSV LTVLAQDWLN GKEYKCKVSN
KALPAPIEKT ISKAKGQPRE PQVYTLPPSR DELTKNQVSL TCLVKGFYPS
DIAVEWESNG QPENNYKTTP PVLDSDGSFF LYSKLTVDKS RWQQGNVFSC
SVMHEALANH ATQKSLSLSP G (SEQ ID NO: 45).

A polypeptide chain of a variant human Fc-region of the IgG1 subclass with the mutations M252Y, S254T and T256E has the following amino acid sequence:

CPPCPAPELL GGPSVFLFPP KPKDTLYITR EPEVTCVVVD VSHEDPEVKF
NWYVDGVEVH NAKTKPREEQ YNSTYRVVSV LTVLHQDWLN GKEYKCKVSN
KALPAPIEKT ISKAKGQPRE PQVYTLPPSR DELTKNQVSL TCLVKGFYPS
DIAVEWESNG QPENNYKTTP PVLDSDGSFF LYSKLTVDKS RWQQGNVFSC
SVMHEALHNH YTQKSLSLSP G (SEQ ID NO: 46).

A polypeptide chain of a wild-type human Fc-region of the IgG4 subclass has the following amino acid sequence:

CPSCPAPEFL GGPSVFLFPP KPKDTLMISR TPEVTCVVVD VSQEDPEVQF
NWYVDGVEVH NAKTKPREEQ FNSTYRVVSV LTVLHQDWLN GKEYKCKVSN
KGLPSSIEKT ISKAKGQPRE PQVYTLPPSQ EEMTKNQVSL TCLVKGFYPS
DIAVEWESNG QPENNYKTTP PVLDSDGSFF LYSRLTVDKS RWQEGNVFSC
SVMHEALHNH YTQKSLSLSL G (SEQ ID NO: 47).

A polypeptide chain of a variant human Fc-region of the IgG4 subclass with the mutations S228P and L235E has the following amino acid sequence:

CPPCPAPEFE GGPSVFLFPP KPKDTLMISR TPEVTCVVVD VSQEDPEVQF
NWYVDGVEVH NAKTKPREEQ FNSTYRVVSV LTVLHQDWLN GKEYKCKVSN
KGLPSSIEKT ISKAKGQPRE PQVYTLPPSQ EEMTKNQVSL TCLVKGFYPS
DIAVEWESNG QPENNYKTTP PVLDSDGSFF LYSRLTVDKS RWQEGNVFSC
SVMHEALHNH YTQKSLSLSL G (SEQ ID NO: 48).

A polypeptide chain of a variant human Fc-region of the IgG4 subclass with the mutations S228P, L235E and P329G has the following amino acid sequence:

CPPCPAPEFE GGPSVFLFPP KPKDTLMISR TPEVTCVVVD VSQEDPEVQF
NWYVDGVEVH NAKTKPREEQ FNSTYRVVSV LTVLHQDWLN GKEYKCKVSN
KGLGSSIEKT ISKAKGQPRE PQVYTLPPSQ EEMTKNQVSL TCLVKGFYPS
DIAVEWESNG QPENNYKTTP PVLDSDGSFF LYSRLTVDKS RWQEGNVFSC
SVMHEALHNH YTQKSLSLSL G (SEQ ID NO: 49).

A polypeptide chain of a variant human Fc-region of the IgG4 subclass with the mutations S228P, L235E, P329G, T366S, L368A and Y407V has the following amino acid sequence:

ESKYGPPCPP CPAPEFEGGP SVFLFPPKPK DTLMISRTPE VTCVVVDVSQ
EDPEVQFNWY VDGVEVHNAK TKPREEQFNS TYRVVSVLTV LHQDWLNGKE
YKCKVSNKGL GSSIEKTISK AKGQPREPQV YTLPPSQEEM TKNQVSLSCA
VKGFYPSDIA VEWESNGQPE NNYKTTPPVL DSDGSFFLVS RLTVDKSRWQ
EGNVFSCSVM HEALHNHYTQ KSLSLSLG (SEQ ID NO: 50).

A polypeptide chain of a variant human Fc-region of the IgG4 subclass with the mutations S228P, L235E, P329G and T366W has the following amino acid sequence:

ESKYGPPCPP CPAPEFEGGP SVFLFPPKPK DTLMISRTPE VTCVVVDVSQ
EDPEVQFNWY VDGVEVHNAK TKPREEQFNS TYRVVSVLTV LHQDWLNGKE
YKCKVSNKGL GSSIEKTISK AKGQPREPQV YTLPPSQEEM TKNQVSLWCL
VKGFYPSDIA VEWESNGQPE NNYKTTPPVL DSDGSFFLYS RLTVDKSRWQ
EGNVFSCSVM HEALHNHYTQ KSLSLSLG (SEQ ID NO: 51).

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The terms "full length antibody", "intact antibody", and "whole antibody" are used herein interchangeably to refer to an antibody having a structure substantially similar to a native antibody structure.

The terms "host cell", "host cell line", and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

A "humanized" antibody refers to a chimeric antibody comprising amino acid residues from non-human HVRs and amino acid residues from human FRs. In certain embodiments, a humanized antibody will comprise substantially all of at least one, and typically two, variable domains, in which all or substantially all of the HVRs (e.g., CDRs) correspond to those of a non-human antibody, and all or substantially all of the FRs correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "hypervariable region" or "HVR", as used herein, refers to each of the regions of an antibody variable domain comprising the amino acid residue stretches which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3).

### HVRs include

(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

An "immunoconjugate" is a circular fusion polypeptide as reported herein conjugated to one or more molecule(s), including but not limited to a cytotoxic agent.

An "individual" or "subject" is a mammal. Mammals include, but are not limited to, domesticated animals (e.g. cows, sheep, cats, dogs, and horses), primates (e.g., humans and non-human primates such as monkeys), rabbits, and rodents (e.g., mice and rats). In certain embodiments, the individual or subject is a human.

An "isolated" circular fusion polypeptide, i.e. dicircular fusion polypeptide or multicircular fusion polypeptide is one which has been separated from a component of its natural environment. In some embodiments, a circular fusion polypeptide is purified to greater than 95% or 99% purity as determined by, for example, electrophoretic (e.g., SDS-PAGE, isoelectric focusing (IEF), capillary electrophoresis) or chromatographic (e.g., ion exchange or reverse phase HPLC). For review of methods for assessment of purity, see, e.g., Flatman, S. et al., J. Chromatogr. B 848 (2007) 79-87.

An "isolated" nucleic acid refers to a nucleic acid molecule that has been separated from a component of its natural environment. An isolated nucleic acid includes a nucleic acid molecule contained in cells that ordinarily contain the nucleic acid molecule, but the nucleic acid molecule is present extrachromosomally or at a chromosomal location that is different from its natural chromosomal location.

"Isolated nucleic acid encoding a (multi)circular fusion polypeptide" refers to one (homomultimeric (multi) circular fusion polypeptide) or more (heteromultimeric (multi)circular fusion polypeptide) nucleic acid molecules each encoding a single chain polypeptide of the circular fusion polypeptide, including such nucleic acid molecule(s) in a single vector or separate vectors, and such nucleic acid molecule(s) present at one or more locations in a host cell.

The term "light chain" denotes the shorter polypeptide chains of native IgG antibodies. The light chain of an antibody may be assigned to one of two types, called kappa (κ) and lambda (λ), based on the amino acid sequence of its constant domain, see SEQ ID NO: 52 for a human kappa light chain constant domain and SEQ ID NO: 53 for a human lambda light chain constant domain.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "naked circular fusion polypeptide" refers to a circular fusion polypeptide that is not conjugated to a moiety (e.g., a cytotoxic moiety) or radiolabel. The naked circular fusion polypeptide may be present in a pharmaceutical formulation.

"Native antibodies" refer to naturally occurring immunoglobulin molecules with varying structures. For example, native IgG antibodies are heterotetrameric glycoproteins of about 150,000 daltons, composed of two identical light chains and two identical heavy chains that are disulfide-bonded. From N- to C-terminus, each heavy chain has a variable region (VH), also called a variable heavy domain or a heavy chain variable domain, followed by three constant domains (CH1, CH2, and CH3), whereby between the first and the second constant domain a hinge region is located. Similarly, from N- to C-terminus, each light chain has a variable region (VL), also called a variable light domain or a light chain variable domain, followed by a constant light (CL) domain. The light chain of an antibody may be assigned to one of two types, called kappa (κ) and lambda (λ), based on the amino acid sequence of its constant domain.

The term "package insert" is used to refer to instructions customarily included in commercial packages of therapeutic products, that contain information about the indications, usage, dosage, administration, combination therapy, contraindications and/or warnings concerning the use of such therapeutic products.

The term "paratope" refers to that part of a given antibody molecule that is required for specific binding between a target and a binding site. A paratope may be continuous, i.e. formed by adjacent amino acid residues present in the binding site, or discontinuous, i.e. formed by amino acid residues that are at different positions in the primary sequence of the amino acid residues, such as in the amino acid sequence of the CDRs of the amino acid residues, but in close proximity in the three-dimensional structure, which the binding site adopts.

The term "peptidic linker" denotes a linker of natural and/or synthetic origin. A peptidic linker consists of a linear chain of amino acids wherein the 20 naturally occurring amino acids are the monomeric building blocks which are connected by peptide bonds. The chain has a length of from 1 to 50 amino acid residues, preferred between 1 and 28 amino acid residues, especially preferred between 3 and 25 amino acid residues. The peptidic linker may contain repetitive amino acid sequences or sequences of naturally occurring polypeptides. The peptidic linker has the function to ensure that the domains of a circular fusion polypeptide can perform their biological activity by allowing the domains to fold correctly and to be presented properly. Preferably the peptidic linker is a "synthetic peptidic linker" that is designated to be rich in glycine, glutamine, and/or serine residues. These residues are arranged e.g. in small repetitive units of up to five amino acids, such as GGGS (SEQ ID NO: 54), GGGGS (SEQ ID NO: 55), QQQG (SEQ ID NO: 56), QQQQG (SEQ ID NO: 57), SSSG (SEQ ID NO: 58) or SSSSG (SEQ ID NO: 59). This small repetitive unit may be repeated for two to five times to form a multimeric unit, such as e.g. (GGGS)2 (SEQ ID NO: 60), (GGGS)3 (SEQ ID NO: 61), (GGGS)4 (SEQ ID NO: 62), (GGGS)5 (SEQ ID NO: 63), (GGGGS)2 (SEQ ID NO: 64), (GGGGS)3 (SEQ ID NO: 65), or (GGGGS)4 (SEQ ID NO: 66). At the amino- and/or carboxy-terminal ends of the multimeric unit up to six additional arbitrary, naturally occurring amino acids may be added. Other synthetic peptidic linkers are composed of a single amino acid, that is repeated between 10 to 20 times and may comprise at the amino- and/or carboxy-terminal end up to six additional arbitrary, naturally occurring amino acids, such as e.g. serine in the linker GSSSSSSSSSSSSSSSG (SEQ ID NO: 67). All peptidic linkers can be encoded by a nucleic acid molecule and therefore can be recombinantly expressed. As the linkers are themselves peptides, the antifusogenic peptide is connected to the linker via a peptide bond that is formed between two amino acids.

"Percent (%) amino acid sequence identity" with respect to a reference polypeptide sequence is defined as the percentage of amino acid residues in a candidate sequence that are identical with the amino acid residues in the reference polypeptide sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN or Megalign (DNASTAR) software. Those skilled in the art can determine appropriate parameters for aligning sequences, including any algorithms needed to achieve maximal alignment over the full length of the sequences being compared. For purposes herein, however, % amino acid sequence identity values are generated using the sequence comparison computer program ALIGN-2. The ALIGN-2 sequence comparison computer program was authored by Genentech, Inc., and the source code has been filed with user documentation in the U.S. Copyright Office, Washington D.C., 20559, where it is registered under U.S. Copyright Registration No. TXU510087. The ALIGN-2 program is publicly available from Genentech, Inc., South San Francisco, California, or may be compiled from the source code. The ALIGN-2 program should be compiled for use on a UNIX operating system, including digital UNIX V4.0D. All sequence comparison parameters are set by the ALIGN-2 program and do not vary.

In situations where ALIGN-2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:
100 times the fraction X/Y where X is the number of amino acid residues scored as identical matches by the sequence alignment program ALIGN-2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. Unless specifically stated otherwise, all % amino acid sequence identity values used herein are obtained as described in the immediately preceding paragraph using the ALIGN-2 computer program.

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies of the invention are used to delay development of a disease or to slow the progression of a disease.

The term "variable region" or "variable domain" refers to the domain of an antibody heavy or light chain that is involved in binding the antibody to antigen. The variable domains of the heavy chain and light chain (VH and VL, respectively) of a native antibody generally have similar structures, with each domain comprising four conserved framework regions (FRs) and three hypervariable regions (HVRs) (see, e.g., Kindt, T.J. et al. Kuby Immunology, 6th ed., W.H. Freeman and Co., N.Y. (2007), page 91) A single VH or VL domain may be sufficient to confer antigen-binding specificity. Furthermore, antibodies that bind a particular antigen may be isolated using a VH or VL domain from an antibody that binds the antigen to screen a library of complementary VL or VH domains, respectively (see, e.g., Portolano, S., et al., J. Immunol. 150 (1993) 880-887; Clackson, T., et al., Nature 352 (1991) 624-628).

The term "vector", as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors".

The invention is exemplified in the following using circular fusion polypeptides of different structure and specificity. These are presented only in order to exemplify the invention. This has not to be construed as a limitation. The true scope is set forth in the claims.

### II. The circular fusion polypeptide as reported herein

The invention is based at least in part on the finding that the fusion of the light chain variable domain to the C-terminus of a (full length) heavy chain, or vice versa, results in the formation of a functional binding site of the respective VH and VL domains, i.e. the pair of variable light chain domain and variable heavy chain domain within a single polypeptide chain form a functional VH/VL-pair and thereby a functional binding site by intrachain circularization.

The invention is based at least in part on the finding that a target binder can be provided comprising a single (circular) polypeptide. In this (circular) polypeptide the N-terminal portion comprises a first part of a binding domain and the C-terminal portion comprises a second part of a binding domain. The first part of the binding domain and the second part of the binding domain (associate with each other to) form a complete or functional binding site. Thereby the polypeptide is circularized.

The invention is based at least in part on the finding that it is possible, by modifying the length of the peptidic linker connecting individual antibody variable domains to the respective N- and C-termini of the central spacer domain such as e.g. Fc-region polypeptides, to adjust the geometry/distance of the two binding sites in a resulting (bicircular) dimeric fusion polypeptide.

The invention is based at least in part on the finding that the binding geometry of a dimeric, i.e. dicircular, fusion polypeptide can be changed depending on the lengths of the first linker and the second linker (i.e. the linker length ratio). Thereby it is possible to fix the geometry of the two Fab-like binding arms with respect to each other.

Herein is disclosed a circular fusion polypeptide comprising a first part of a binding domain, a second part of a binding domain and a spacer domain, wherein
- the spacer domain is a polypeptide, e.g. forming a structural domain after folding,
- the first part of the binding domain is a polypeptide and is fused via a first (peptidic) linker to the N-terminus of the spacer domain,
- the second part of the binding domain is a polypeptide and is fused via a second (peptidic) linker to the C-terminus of the spacer domain,
- the first part of the binding domain and the second part of the binding domain (of the same fusion polypeptide) associated with each other and form a (functional) binding site that specifically binds to a target.

The first part of the binding domain and the second part of the binding domain can be non-covalently or covalently associated with each other. If the association is covalently it is by a bond other than a peptide bond, such as e.g. by a disulfide bond.

The spacer domain is a polypeptide forming a structural domain after folding. Thus, the spacer domain can be smaller than 100 amino acid residues, but needs to be structurally confined to fix the binding motifs. Exemplary spacer domains are pentameric coil-coils, antibody hinge regions or antibody Fc-regions or fragments thereof.

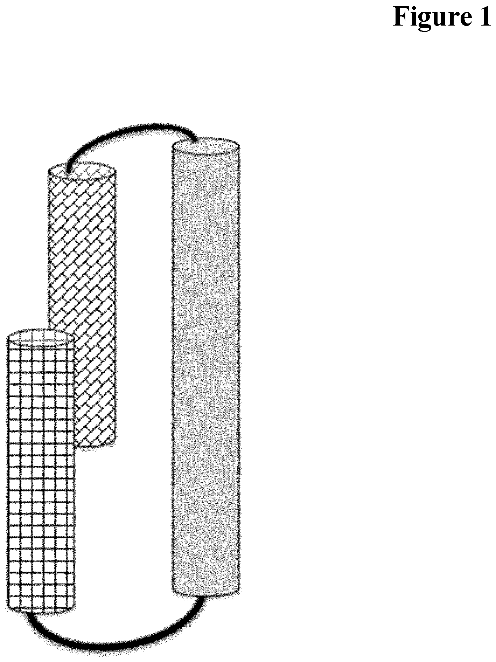

The circular fusion polypeptide as reported herein is a single chain polypeptide. The general structure of the circular fusion polypeptide is shown in Figure 1.

Also reported herein is a dimeric, i.e. dicircular, fusion polypeptide comprising a first circular fusion polypeptide as reported herein and a second circular fusion polypeptide as reported herein, wherein the first and the second circular fusion polypeptide are identical or different and wherein the spacer domain of the first circular fusion polypeptide is conjugated to the spacer domain of the second circular fusion polypeptide by at least one non-peptide bond, in one embodiment by at least one disulfide bond.

In this case the spacer domain is a dimerization domain, i.e. the structural property of the spacer domain is the provision of a dimerization functionality.

Likewise trimeric, i.e. tricircular, fusion polypeptides and tetrameric, i.e. tetracircular, fusion polypeptides can be obtained if a trimerization domain or a tetramerization domain is used as spacer domain respectively.

The multicircular fusion polypeptides as reported herein are also termed Contorsbody. The general structure of the dicircular fusion polypeptide/Contorsbody is shown in Figure 2A (with dimerization spacer domain) and 2B (without dimerization spacer domain), of the tricircular fusion polypeptide/Contorsbody is shown in Figure 3, and of the tetracircular fusion polypeptide/Contorsbody is shown in Figure 4A (with tetramerization spacer domain) and 4B (without tetramerization spacer domain).

If the spacer domain is an antibody Fc-region, as an example of a dimerization domain, and the binding domains are antibody Fab heavy and light chain fragments this specific Contorsbody is an antibody format. Compared to normal IgG antibodies, the Contorsbody is using only one chain and not a heavy and a light chain. The particularity of the Contorsbody is that the Fc-region coding sequence is located in between the heavy chain Fab fragment and the light chain Fab fragment.

This specific Contorsbody is used in the following to show the specific properties of the dicircular fusion polypeptide as reported herein. Beside the Fab fragments also isolated variable domains could be used as parts of a binding site.

Single chain fusion polypeptides as reported herein with a "Hinge-CH2-CH3" spacer domain as dimerization domain in between the heavy chain Fab fragment and the light chain Fab fragment dimerize via their Fc-regions, which are thereby forming a complete Fc portion of an antibody presenting two Fabs, wherein the two Fabs are fixed in their orientation to each other. In contrast, in a normal IgG type antibody the two Fabs are more flexible and much differently oriented.

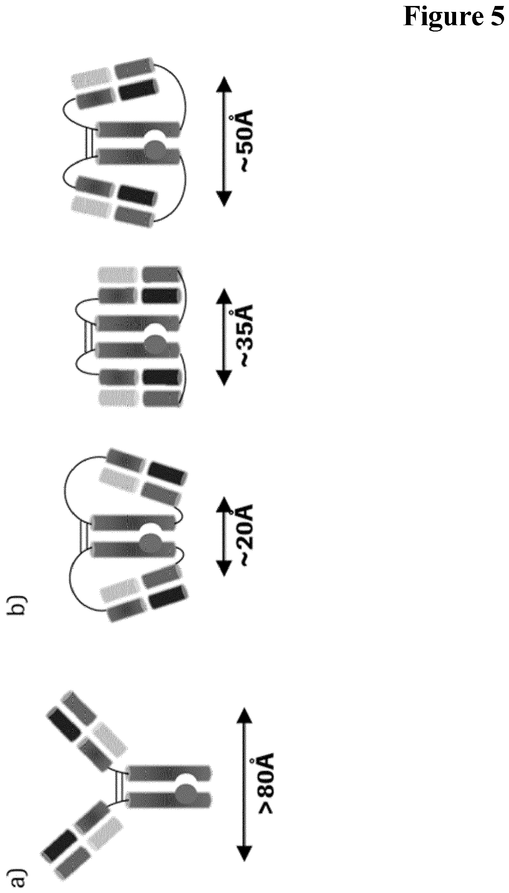

The geometry of the two binding sites relative to each other can be modulated in a Contorsbody by the length of the employed linkers. The orientation and spatial distance between the binding sites of a normal antibody of the IgG type and a dicircular fusion polypeptide as reported herein in exemplary embodiments are shown in Figure 5.

The spatial distance between the binding sites of a conventional antibody of the IgG type is about 80 angstrom or more. The spatial distance between the binding sites of a dicircular fusion polypeptide as reported herein can be between about 20 angstrom to about 50 angstrom depending on the length and length ratio of the peptidic linker in the individual circular fusion polypeptides forming the dicircular fusion polypeptide. Depending on the intended geometry the peptidic linker is selected. In one embodiment the peptidic linker are independently of each other selected from the group of peptidic linker consisting of SEQ ID NO: 54 to SEQ ID NO: 70.

### II.1. monospecific multicircular fusion polypeptides as reported herein

A monospecific multicircular fusion polypeptide comprises two or more circular fusion polypeptides as reported herein wherein each of the circular fusion polypeptides specifically binds to the same epitope on the same target, i.e. comprises the same binding site.

An exemplary monospecific multicircular fusion polypeptide is an anti-Her2 Contorsbody (comprising the circular fusion polypeptide of SEQ ID NO: 96). It was produced by transfecting HEK293 cells with a vector containing a nucleotide sequence encoding the circular fusion polypeptide.

It has been found that a conventional protein A affinity chromatography is suitable to extract the Fc-region containing part of the cultivation supernatant. Alternatively a hexahistidine C-terminal tag (SEQ ID NO: 03) connected via a GSG peptidic linker for purification purpose can be used.

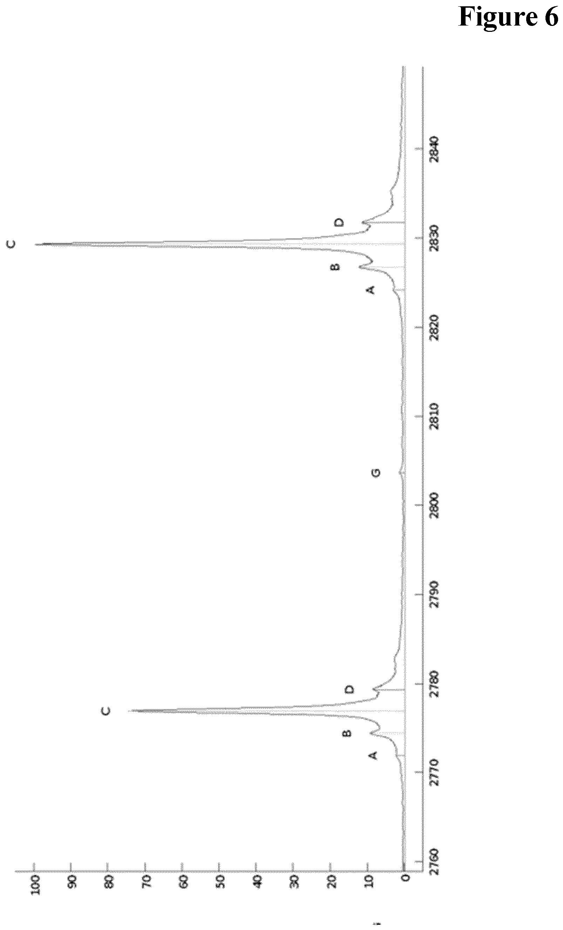

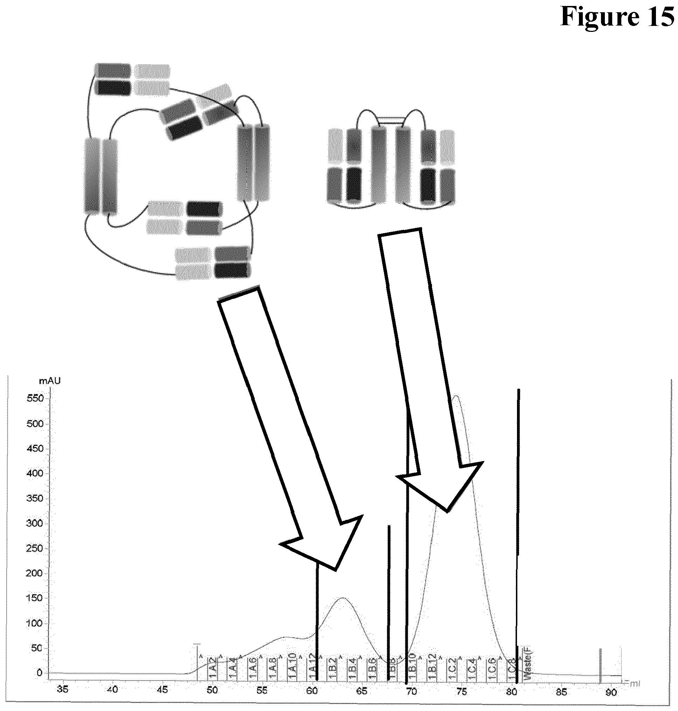

Preparative size exclusion chromatography was used in the second purification step to separate the circular fusion polypeptide from product related impurities, mostly higher order structures of the circular fusion polypeptide (a small portion of aggregates is also seen in the chromatogram as for an antibody of the IgG type) (see e.g. Figure 15). Typical yield for such constructs is averaging 10 mg/liter. The anti-Her2 Contorsbody has been expressed in several batches (from 0.5 liter to 2 liter shake flask scale). Product quality has been analyzed by mass spectrometry (see Figure 6). The identity of the product was confirmed with a purity grade above 95%.

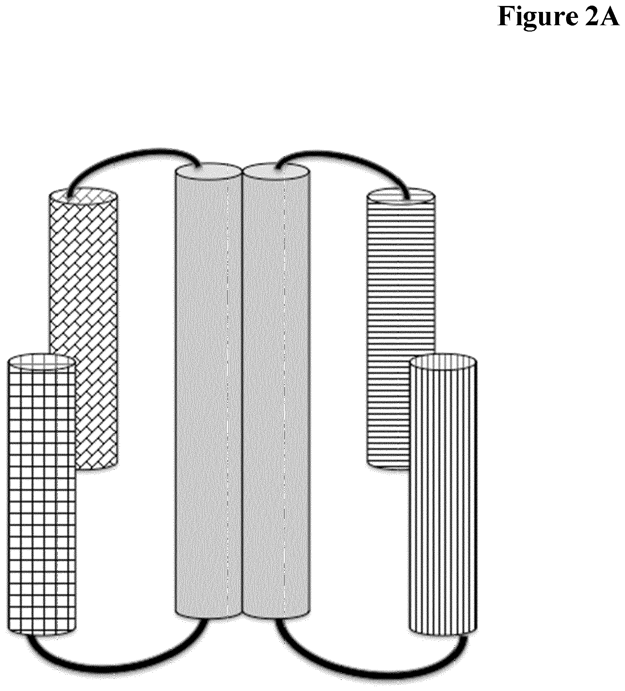

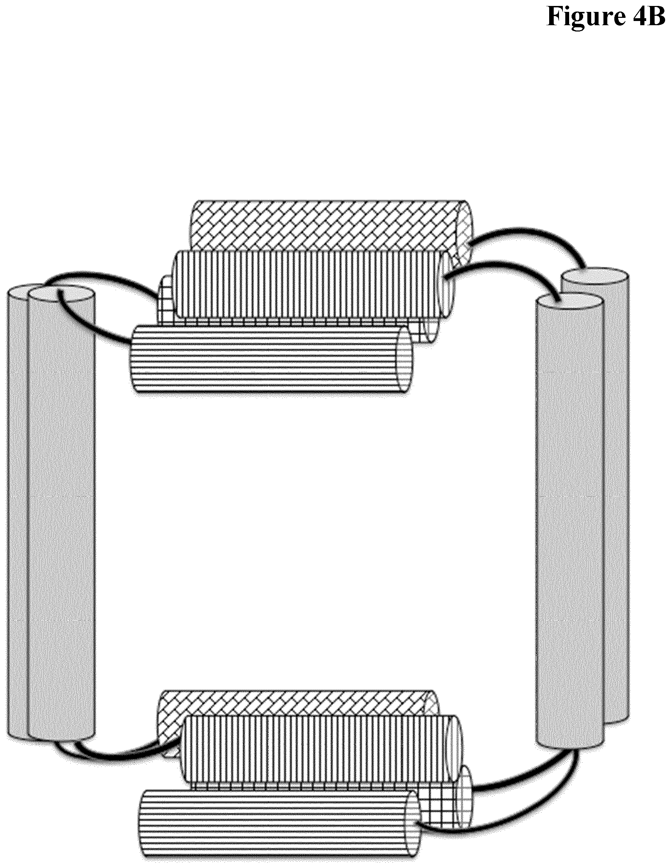

The binding of the anti-Her2 Contorsbody in its dicircular form (cf. Figure 2A) and in its tetracircular form (cf. Figure 4B) has been determined using surface plasmon resonance (SPR, e.g. BIAcore) in two different settings in order to assess the affinity and the avidity of the molecules compared to a conventional anti-Her2 antibody of the IgG type.

In the first setting (1 in the following Table; for affinity) the respective anti-Her2 Contorsbody was captured by an anti-human Fc-region antibody conjugated to the SPR chip surface. As analyte the Her2 extracellular domain (ECD) was used. In the second setting (2 in the following Table; for avidity) the anti-Her2 antibody pertuzumab (marketed as Perjeta(R)) was immobilized on the chip surface and the ECD of Her2 was captured thereby. As analyte the respective Contorsbody was used. The avidity of the IgG type reference antibody trastuzumab, the bivalent anti-Her2 Contorsbody and the tetravalent anti-Her2 Contorsbody was measured using concentration series of the analyte. As reference in both setting the anti-Her2 antibody trastuzumab (marketed as Herceptin(R)) has been used.

| **setting** | **molecule** | **kₐ** | **k_{d}** | **t(1/2)** | **K_{D}** | **ratio Rₘₐₓ exp./theor.** |
|---|---|---|---|---|---|---|
| | | **[M⁻¹ s⁻¹]** | **[s⁻¹]** | **[min]** | **[M]** | |
| 1 | trastuzumab | 6.8E+05 | 5.2E-04 | 22.2 | 7.7E-10 | 114 |
| 1 | bicircular Contorsbody | 2.1E+05 | 1.9E-03 | 6.1 | 9.1E-09 | 41 |
| 1 | tetracircular Contorsbody | 3.5E+04 | 1.2E-04 | 96.1 | 3.1E-09 | 84 |
| 2 | trastuzumab | 1.03E+06 | 6.36E-05 | 181.7 | 6.2E-11 | 101.5 |
| 2 | bicircular Contorsbody | 1.11E+06 | 6.59E-05 | 175.4 | 5.9E-11 | 102.3 |
| 2 | tetracircular Contorsbody | 6.25E+05 | 5.93E-05 | 194.9 | 9.6E-11 | 123.3 |

The Contorsbody is much more compact compared to a conventioinal antibody of the IgG type.

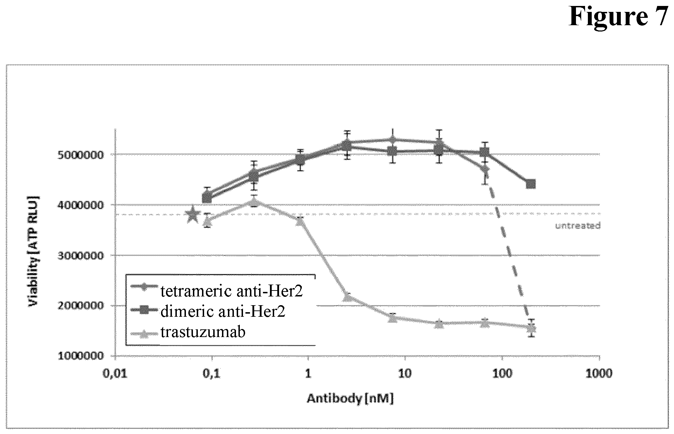

The anti-Her2 Contorsbodies have been tested in a proliferation assay. Similarly to Scheer et al. (Scheer et al., PLoS One 7 (2012) e51817) who used chemically crosslinked trastuzumab Fabs, the anti-Her2 Contorsbodies recruited receptors on the cell surface and promoted an activation signal. Trastuzumab, a binder for an Her2 epitope located on the receptor stalk domain, is keeping the receptors away from each other and, and consequently is antagonizing the proliferation. Figure 7 shows the differential effects of trastuzumab and both the dicircular and the tetracircular anti-Her2 Contorsbody, respectively, to be anti-proliferative and pro-proliferative.

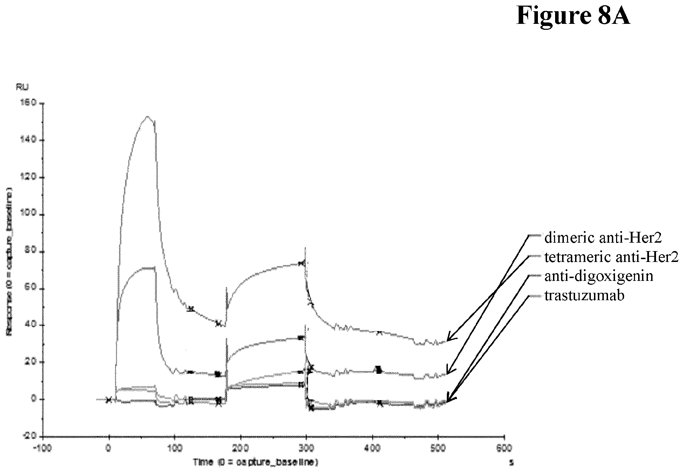

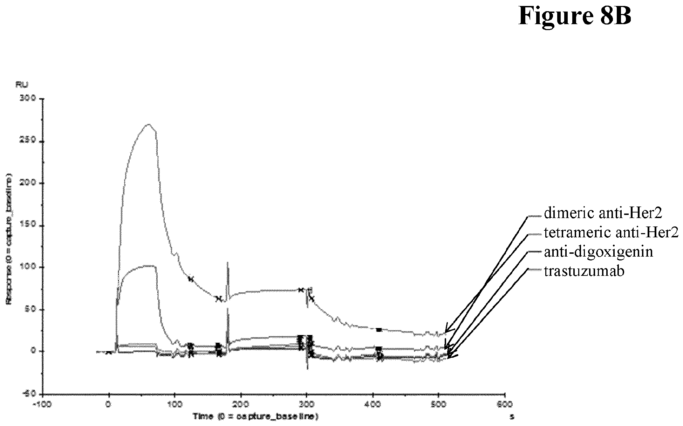

The ability of the anti-Her2 Contorsbodies to bind FcRn receptor has been determined. Compared to trastuzumab, an antibody of the IgG type, IgG1 subclass, the binding of both anti-Her2 Contorsbodies to human FcRn receptor is surprisingly higher, i.e. around 10x and 30x for the bicircular Contorsbody and the tetracircular Contorsbody, respectively (see Figure 8A). Against cynomolgus FcRn trastuzumab and the Contorsbodies behave similarly (see Figure 8B), the dimeric Contorsbody being 4.5 times more affine than trastuzumab and the tetrameric Contorsbody being 61 times more affine.

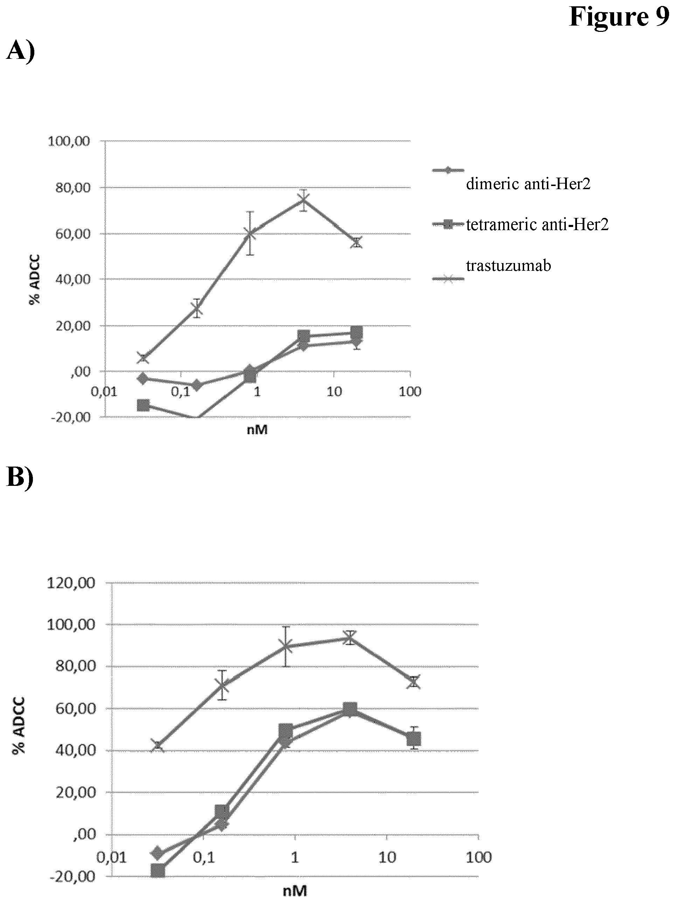

The ability of the anti-Her2 Contorsbodies to elicit antibody dependent cell mediated cytotoxicity (ADCC) has been evaluated. Through binding to FcyR, an IgG can trigger ADCC. In Figure 9 the ADCC kinetic of the anti-Her2 Contorsbodies and trastuzumab is shown.

Using tryptophan autofluorescence, the first thermal denaturation temperature Tₘ1 is approx. 63 °C for the Contorsbody. Using static light scattering, an aggregation onset temp. of approx. 66 °C was determined for the Contorsbody. Using dynamic light scattering, an aggregation onset temp. of approx. 66°C was determined for the Contorsbody. In all experiments the formulation was 1 mg/mL Contorsbody in 20 mM His/His^{∗}HCl, 140 mM NaCl.

It could be confirmed by mass spectrometry that no mixed Fabs are formed.

Other examples of a dicircular mono-specific Contorsbodies are anti-cMET Contorsbody (circular fusion polypeptide of SEQ ID NO: 97), and anti-CD20 Contorsbodies with different variable domains ((1) circular fusion polypeptide of SEQ ID NO: 98; (2) circular fusion polypeptide of SEQ ID NO: 99). In the following Table the expression rate, the yield and the quality of these Contorsbodies are given.

| | anti-cMET Contors-body | anti-CD20 Contors-body (1) | anti-CD20 Contors-body (2) |
|---|---|---|---|
| expression volume [ml] | 250 | 500 | 500 |
| concentration [mg/ml] | 0.60 | 1.06 | 1.0 |
| amount (mg) | 0.78 | 3.1 | 5.7 |
| % monomer (analyt. SEC) | 100.00 | 97.9 | 98.4 |
| % main peak (CE-SDS) | 96.30 | 99.3 | 99.6 |
| yield [mg/l] | 3.12 | 6.2 | 11.4 |

All Contorsbodies have been expressed transiently in HEK-293 cells with yields ranging from 2 to 15 mg/L. The product after protein A column is above 85 % and is purified from side-products by SEC column chromatography up to a purity above 96 % in general.

### II.2. multispecific multicircular fusion polypeptides

A multispecific multicircular fusion polypeptide comprises two or more circular fusion polypeptides as reported herein wherein each of the circular fusion polypeptides specifically binds to a different target and/or to a different epitope on the same target, i.e. comprises at least two binding sites of different sopecificity.

Without being bound by this theory it is postulated that the self-assembly of the corresponding domains of the binding sites in the single chain polypeptide is prevalent to inter-chain association, i.e. in other words, after expression of a single circular fusion polypeptide the individual, non-functional domains of the binding site associate and form a functional binding site. For example, if the functional binding site is a Fab and the spacer domain is an Fc-region, the Fab portions, i.e. the heavy chain fragment (VH-CH1) and the light chain fragment (VK-CK), form a constitutive, binding competent Fab moiety and the orphan half Fc-portion of this first assembled circular fusion polypeptide associates consecutively with another circular fusion polypeptide to form a bicircular fusion polypeptide (Contorsbody comprising a dimer of two single circular fusion polypeptides). In order to obtain a multispecific multicircular fusion polypeptide the heterodimerization of the isolated circular fusion polypeptides has to be promoted. One exemplary heterodimerization promoting element are the mutations according to the knob-into-hole technology.

It is possible to modify the length of the linker at the respective N- and C-termini of the Fc-region spacer domain in order to vary the geometry/distance of the two binding sites of the resulting bispecific Contorsbody; the same is also true for monospecific Contorsbodies.

It is possible to modify the geometry/distance of the binding sites by changing the relative position(s) of the binding site(s) to each other.

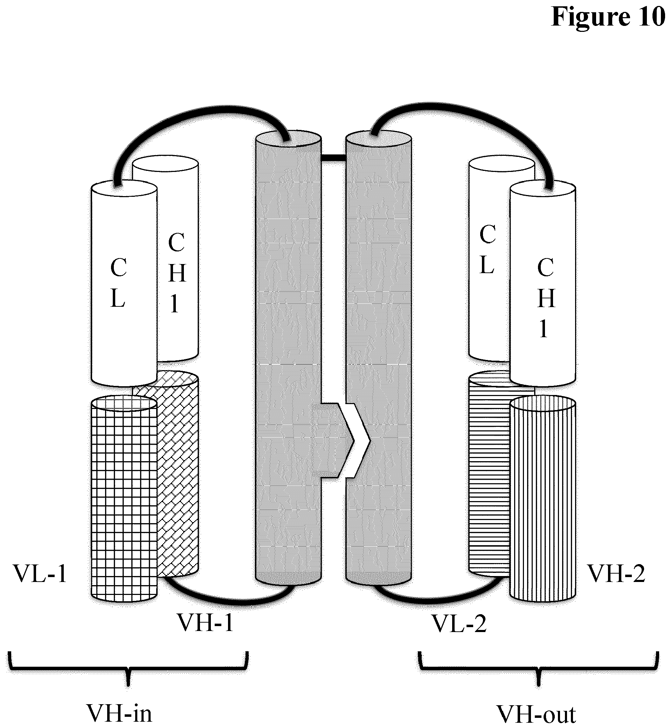

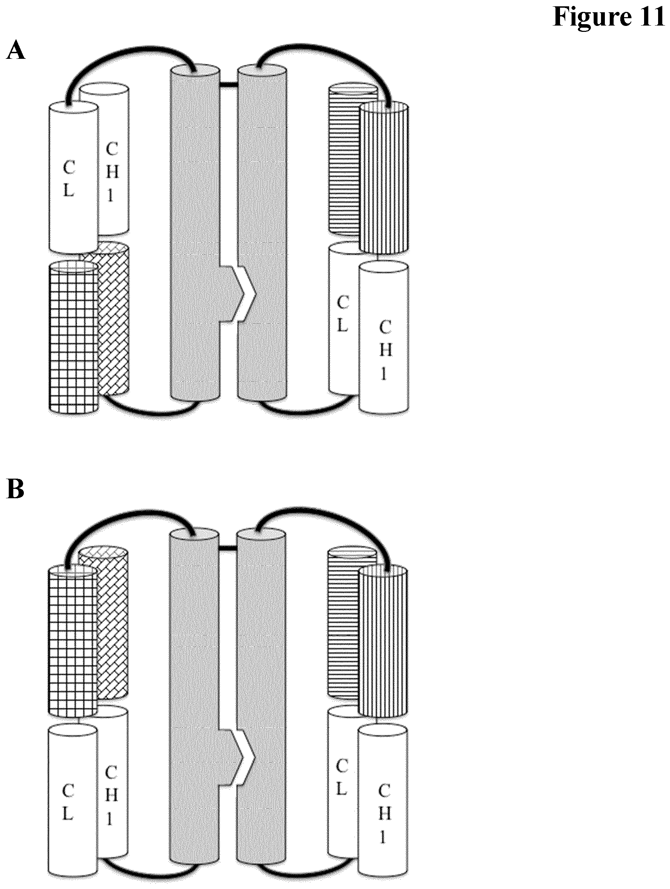

For example, in case of a Fab as binding site, the sequence of the domains of the heavy chain Fab fragment and the light chain Fab fragment can be inverted, i.e., e.g., the heavy chain Fab fragment can have the domain sequence VH-CH1 or CH1-VH (from N- to C-terminus), respectively, and likewise the light chain Fab fragment can have the domain sequence VL-CL or CL-VL (from the N- to C-terminus) or mixed. Assuming that a linker is present before and after the spacer domain, e.g. before and after the Fc-region (in N- to C-terminal direction), the Fab fragment is limited in its orientations with regard to the spacer domain. Consequently, the relative position of the VH domain is either close to the Fc-region, called here "VH-in", or more apart from the Fc-region, called here "VH-out" (see Figures 10 and 11).

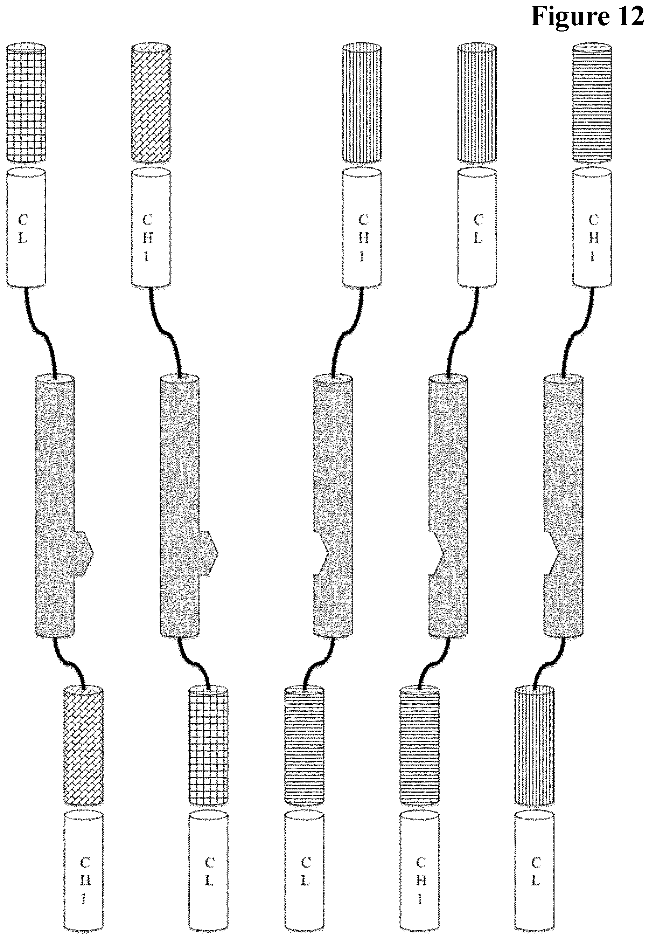

It is possible to also modify the assembly behavior in using the CrossMab technology, i.e. a domain exchange in one arm. This can further be combined with charge variants in the exchanged or non-exchanged arm. Exemplary chains of anti-cMET circular fusion polypeptides with the respective orientation used for the production of bispecific bicircular fusion polypeptides are shown in Figure 12 (VH-out, VH-in, and the respective expression rate, yield and quality of these different chain combinations are given in the following Table.

| | VH-out-knob/V H-out-hole | VH-in-knob/V H-out-hole | VH-out-knob/V H-out-hole-CH-CL-crossed | VH-in-knob/V H-out-hole-CH-CL-crossed | VH-out-knob/V H-in-hole-VH-VL-crossed | VH-in-knob/V H-in-hole-VH-VL-crossed |
|---|---|---|---|---|---|---|
| expression volume [ml] | 250 | 250 | 250 | 250 | 250 | 250 |
| concentratio n [mg/ml] | 1.80 | 0.60 | 0.48 | 1.17 | 0.36 | 0.65 |
| amount [mg] | 2.88 | 0.60 | 0.81 | 1.17 | 0.43 | 0.65 |
| monomer (analyt. SEC) [%] | 98.10 | 93.80 | 97.34 | 97.90 | 98.74 | 98.88 |
| main peak (CE-SDS) [%] | 96.80 | 98.80 | 95.73 | 100.00 | 100.00 | 100.00 |
| yield [mg/l] | 11.52 | 2.40 | 3.24 | 4.68 | 1.72 | 2.60 |

| | | | | | | |
|---|---|---|---|---|---|---|
| VH-out-knob = SEQ ID NO: 100, VH-in-knob = SEQ ID NO: 101, VH-out-hole = SEQ ID NO: 102, VH-out-hole-CH-CL-crossed = SEQ ID NO: 103, VH-in-hole-VH-VL-crossed = SEQ ID NO: 104. | | | | | | |

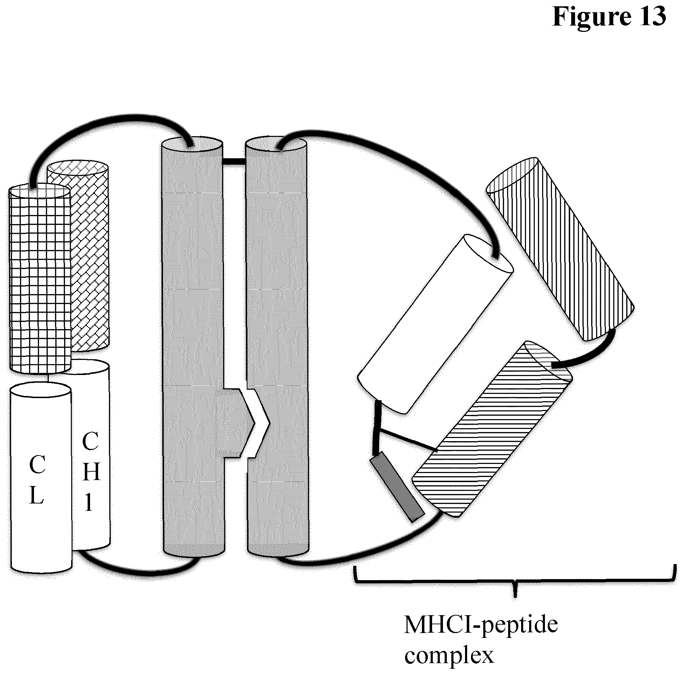

The circular fusion polypeptides as reported herein may comprise any type of two- or multi-component complex as long as a multimerization domain, such as e.g. an half Fc-region, can be inserted in the sequence of the resulting single chain polypeptide. An example of such a multi-component complex is a peptide-loaded MHC-I complex. The respective Contorsbody is depicted in Figure 13.

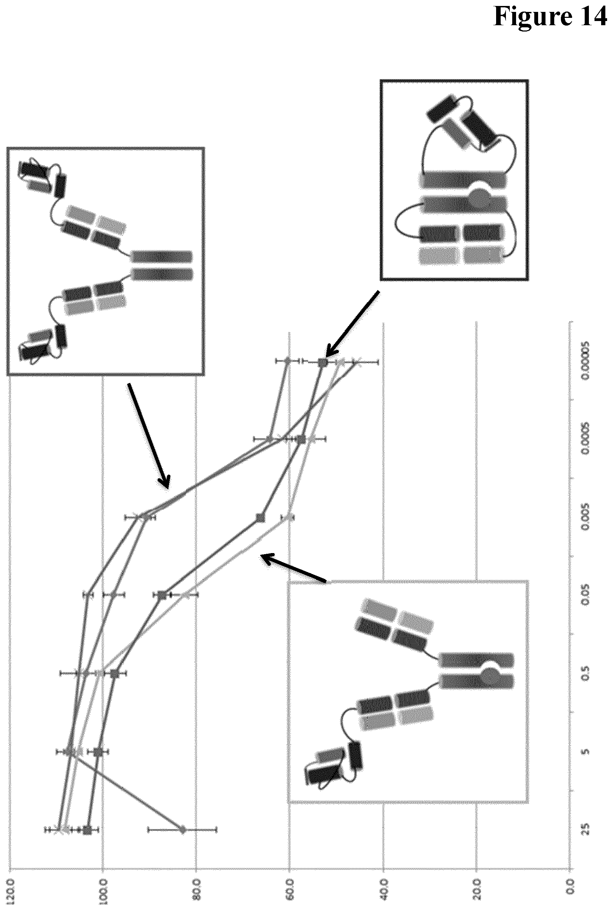

The effect based on MHC-I mediated killer cell recruiting and cell removal is comparable between the MHC-I IgG-type antibody fusions and the bicircular fusion polypeptide as reported herein (Figure 14).

The generally applicable techniques for making conventional multispecific antibodies can also be used and adopted to make multispecific multicircular fusion polypeptides as reported herein.

For example, techniques for making multispecific antibodies include, but are not limited to, recombinant co-expression of two immunoglobulin heavy chain-light chain pairs having different specificities (see Milstein, C. and Cuello, A.C., Nature 305 (1983) 537-540, WO 93/08829, and Traunecker, A. et al., EMBO J. 10 (1991) 3655-3659), and "knob-in-hole" engineering (see, e.g., US 5,731,168). Multispecific antibodies may also be made by engineering electrostatic steering effects for making antibody Fc-heterodimeric molecules (WO 2009/089004); cross-linking two or more antibodies or fragments (see, e.g., US 4,676,980, and Brennan, M. et al., Science 229 (1985) 81-83); using leucine zippers to produce bi-specific antibodies (see, e.g., Kostelny, S.A. et al., J. Immunol. 148 (1992) 1547-1553; using "diabody" technology for making bispecific antibody fragments (see, e.g., Holliger, P. et al., Proc. Natl. Acad. Sci. USA 90 (1993) 6444-6448); and using single-chain Fv (sFv) dimers (see, e.g. Gruber, M et al., J. Immunol. 152 (1994) 5368-5374); and preparing trispecific antibodies as described, e.g., in Tutt, A. et al., J. Immunol. 147 (1991) 60-69).

In one embodiment of all aspects the circular fusion polypeptide as reported herein is a multispecific multicircular fusion polypeptide, which requires heterodimerization of at least two circular fusion polypeptides.

Likewise, one aspects as reported herein is a multimeric, preferably dimeric, circular fusion polypeptide as reported herein, wherein a first circular fusion polypeptide specifically binds to a first target and a second circular fusion polypeptide specifically binds to a second target, each comprising as spacer domain a heterodimerization domain.

The Fc-region contained in the Contorsbody can be with (wild-type; SEQ ID NO: 31)) or without FcR effector function (without FcgammaIII effector function with the mutations L234A, L235A, P329G (SEQ ID NO: 37; without FcRn effector function with the mutations I253A, H310A, H435A (SEQ ID NO: 44) or H310A, H433A, Y436A (SEQ ID NO: 45); numbering according to Kabat EU index).

The binding site can be selected from an antibody binding site comprising a pair of an antibody heavy chain variable domain and an antibody light chain variable domain, an FcRn comprising the MHC-like domain and beta-2-microglobulin, or a pair of DARPINS (designed ankyrin repeat domains), a tandem scFv, and two anticalins in a row.

Generally, each time a geometrical constraint is required to fix the orientation of two binding sites, a Contorsbody can be used.

In one embodiment the spacer domain comprises a tag. In one embodiment a tag is conjugated to the C-terminus of the circular fusion polypeptide.

In one embodiment the spacer domain comprises a multimerization domain. In one embodiment the multimerization domain is selected from the group consisting of an antibody Fc-region and variants thereof, and a tetranectin domain and variants thereof.

The spacer domain can be a single coil domain that forms multimers, or a functional natural protein known to multimerize and, as a multimeric assembly, bring its own function to the Contorsbody. For example, Myc/Max/Mad family dimers or Leucine Zipper make dimeric coil-coil, COMP (cartilage oligomeric matrix protein) makes pentameric coil-coil like system with disulfide bridge in between and a head to head orientation. There are several other systems with head to tail orientation like SARAH (human MST1 C-terminal dimerization domain (residues 431-487)), a dimerization domain, coil-coil with a disulfide bridge useful to have Fabs fixed at 180°. Also the tenascin-C (TNC) trimerization domain could be used.

### III. Binding sites

### III.1. Antibody fragment derived binding site

In certain embodiments, the binding site in the circular fusion polypeptide as reported herein is composed of an antibody heavy chain variable domain (VH) and an antibody light chain variable domain (VL).

In certain embodiments, the binding site of the circular fusion polypeptide is an antibody fragment. Antibody fragments include, but are not limited to, Fab, Fab', Fab'-SH, and Fvfragments. For a review of certain antibody fragments, see Hudson, P.J. et al., Nat. Med. 9 (2003) 129-134. For a review of scFv fragments, see, e.g., Plueckthun, A., In; The Pharmacology of Monoclonal Antibodies, Vol. 113, Rosenburg and Moore (eds.), Springer-Verlag, New York (1994), pp. 269-315; see also WO 93/16185; US 5,571,894 and US 5,587,458. For discussion of Fab fragments comprising salvage receptor binding epitope residues and having increased in vivo half-life, see US 5,869,046.

The antibody fragment can be also a "Dual Acting Fab" or "DAF" (see, US 2008/0069820, for example).

Single-domain antibodies are antibody fragments comprising all or a portion of the heavy chain variable domain or all or a portion of the light chain variable domain of an antibody. In certain embodiments, a single-domain antibody is a human single-domain antibody (Domantis, Inc., Waltham, MA; see, e.g., US 6,248,516).

Antibody fragments can be made by various techniques, including but not limited to proteolytic digestion of an intact antibody as well as production by recombinant host cells (e.g. E. coli or phage), as described herein.

If the binding site is a Fab then the Fab can be a conventional Fab, a CrossFab or a DutaFab.

In case of a conventional Fab one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL). The order of these domains may be any as long as association thereof and forming of a (functional) binding site is possible (i.e. not prevented).

In one embodiment one part of the binding domain comprises in N- to C-terminal direction VH-CH1 and the other part of the binding domain comprises in N- to C-terminal direction VL-CL.

In case of a CrossFab both parts of the binding domain comprises an antibody variable domain and at least an N-terminal fragment of a (or a complete) antibody constant domain whereby the pairs of variable domain and constant domain are not naturally associated with each other and are obtained by a domain crossover/exchange of a heavy chain domain and a light chain domain. This can be the exchange of VH with VL or CH1 with CL. The order of these domains may be any as long as association thereof and forming of a (functional) binding site is possible (i.e. not prevented).

In one embodiment one part of the binding domain comprises in N- to C-terminal direction VL-CH1 and the other part of the binding domain comprises in N- to C-terminal direction VH-CL.

In one embodiment one part of the binding domain comprises in N- to C-terminal direction VH-CL and the other part of the binding domain comprises in N- to C-terminal direction VL-CH1.

In case of a multicircular fusion polypeptide the association of the cognate binding domains can further be promoted beside the domain exchange in the CrossFab by the introduction of charges. In this case the multicircular fusion polypeptide comprises at least a first circular fusion polypeptide and a second circular fusion polypeptide.

In one embodiment the multicircular fusion polypeptide comprises
a) a first circular fusion polypeptide comprising as binding site a Fab specifically binding to a first antigen, and
b) a second circular fusion polypeptide comprising as binding site a Fab specifically binding to a second antigen, wherein the variable domains VL
and VH in the Fab (of the second circular fusion polypeptide) are replaced by each other.

The circular fusion polypeptide under a) does not contain a modification as reported under b).

In the circular fusion polypeptide under b)
within the antibody light chain fragment
the variable light chain domain VL is replaced by the variable heavy chain domain VH of said Fab,
and
within the antibody heavy chain fragment
the variable heavy chain domain VH is replaced by the variable light chain domain VL of said Fab.

In one embodiment
i) in the constant domain CL of the first circular fusion polypeptide of the multicircular fusion polypeptide the amino acid at the position corresponding to position 124 according to Kabat is substituted by a positively charged amino acid, and wherein in the constant domain CH1 of the first circular fusion polypeptide of the multicircular fusion polypeptide the amino acid at the position corresponding to position 147 according to Kabat EU index or the amino acid at the position corresponding to position 213 according to Kabat EU index is substituted by a negatively charged amino acid,
   or
ii) in the constant domain CL of the second circular fusion polypeptide of the multicircular fusion polypeptide the amino acid at the position corresponding to position 124 according to Kabat is substituted by a positively charged amino acid, and wherein in the constant domain CH1 of the second circular fusion polypeptide of the multicircular fusion polypeptide the amino acid at the position corresponding to position 147 according to Kabat EU index or the amino acid at the position corresponding to position 213 according to Kabat EU index is substituted by a negatively charged amino acid.

In one preferred embodiment
i) in the constant domain CL of the first circular fusion polypeptide of the multicircular fusion polypeptide the amino acid at the position corresponding to position 124 according to Kabat is substituted independently by lysine (K), arginine (R) or histidine (H) (in one preferred embodiment independently by lysine (K) or arginine (R)), and wherein in the constant domain CH1 of the first circular fusion polypeptide of the multicircular fusion polypeptide the amino acid at the position corresponding to position 147 according to Kabat EU index or the amino acid at the position corresponding to position 213 according to Kabat EU index is substituted independently by glutamic acid (E) or aspartic acid (D),
   or
ii) in the constant domain CL of the second circular fusion polypeptide of the multicircular fusion polypeptide the amino acid at the position corresponding to position 124 according to Kabat is substituted independently by lysine (K), arginine (R) or histidine (H) (in one preferred embodiment independently by lysine (K) or arginine (R)), and wherein in the constant domain CH1 of the second circular fusion polypeptide of the multicircular fusion polypeptide the amino acid at the position corresponding to position 147 according to Kabat EU index or the amino acid at the position corresponding to position 213 according to Kabat EU index is substituted independently by glutamic acid (E) or aspartic acid (D).

In one embodiment in the constant domain CL of the second circular fusion polypeptide of the multicircular fusion polypeptide the amino acids at the positions corresponding to positions 124 and 123 according to the Kabat EU index are substituted by K.

In one embodiment in the constant domain CH1 of the second circular fusion polypeptide of the multicircular fusion polypeptide the amino acids at the positions corresponding to positions 147 and 213 according to the Kabat EU index are substituted by E.

In one preferred embodiment in the constant domain CL of the first circular fusion polypeptide of the multicircular fusion polypeptide the amino acids at the positions corresponding to positions 124 and 123 according to the Kabat EU index are substituted by K, and in the constant domain CH1 of the first circular fusion polypeptide of the multicircular fusion polypeptide the amino acids at the positions corresponding to positions 147 and 213 according to the Kabat EU index are substituted by E.

In one embodiment in the constant domain CL of the second circular fusion polypeptide of the multicircular fusion polypeptide the amino acids at the positions corresponding to positions 124 and 123 according to Kabat EU index are substituted by K, and wherein in the constant domain CH1 of the second circular fusion polypeptide of the multicircular fusion polypeptide the amino acids at the positions corresponding to positions 147 and 213 according to Kabat EU index are substituted by E, and in the variable domain VL of the first circular fusion polypeptide of the multicircular fusion polypeptide the amino acid at the position corresponding to position 38 according to Kabat is substituted by K, in the variable domain VH of the first circular fusion polypeptide of the multicircular fusion polypeptide the amino acid at the position corresponding to position 39 according to Kabat is substituted by

E, in the variable domain VL of the second circular fusion polypeptide of the multicircular fusion polypeptide the amino acid at the position corresponding to position 38 according to Kabat is substituted by K, and in the variable domain VH of the second circular fusion polypeptide of the multicircular fusion polypeptide the amino acid at the position corresponding to position 39 according to Kabat is substituted by E.

In case of a DutaFab one part of the binding domain comprises an antibody heavy chain variable domain (VH) and at least an N-terminal fragment of a (or a complete) first antibody heavy chain constant domain (CH1) and the respective other binding domain comprises an antibody light chain variable domain (VL) and at least an N-terminal fragment of a (or a complete) antibody light chain constant domain (CL), wherein herein said binding domain comprises two non-overlapping paratopes in the complementary pair of a heavy chain variable domain (VH) and a light chain variable domain (VL), wherein the first paratope comprises residues from CDR1 and CDR3 of the VL domain and CDR2 of the VH domain, and the second paratope comprises residues from CDR1 and CDR3 of the VH domain and CDR2 of the VL domain.

In one embodiment the first paratope comprises residues from CDR1 and CDR3 of the VL domain and CDR2 of the VH domain, and the second paratope comprises residues from CDR1 and CDR3 of the VH domain and CDR2 of the VL domain.

In one embodiment the heavy chain variable domain of the binding site is based on a human VH3 family heavy chain sequence and the light chain variable domain of the binding site is based on a human Vkappa1 family light chain sequence.

In one embodiment the heavy chain variable domain of the binding site is based on a human VH3 family heavy chain sequence and the light chain variable domain of the binding site is based on a human Vlambdal family light chain sequence.

### III.2. Chimeric and humanized antibody derived binding site

In certain embodiments, the binding site in the circular fusion polypeptide as reported herein is composed of an antibody heavy chain variable domain (VH) and an antibody light chain variable domain (VL). In certain embodiments, the variable domains are chimeric domains derived from a chimeric antibody, e.g. a humanized antibody.

"Framework" or "FR" refers to variable domain residues other than hypervariable region (HVR) residues. The FR of a variable domain generally consists of four FR domains: FR1, FR2, FR3, and FR4. Accordingly, the HVR and FR sequences generally appear in the following sequence in VH (or VL): FR1-H1(L1)-FR2-H2(L2)-FR3-H3(L3)-FR4.

The term "hypervariable region" or "HVR", as used herein, refers to each of the regions of an antibody variable domain comprising the amino acid residue stretches which are hypervariable in sequence ("complementarity determining regions" or "CDRs") and/or form structurally defined loops ("hypervariable loops"), and/or contain the antigen-contacting residues ("antigen contacts"). Generally, antibodies comprise six HVRs; three in the VH (H1, H2, H3), and three in the VL (L1, L2, L3).

### HVRs include

(a) hypervariable loops occurring at amino acid residues 26-32 (L1), 50-52 (L2), 91-96 (L3), 26-32 (H1), 53-55 (H2), and 96-101 (H3) (Chothia, C. and Lesk, A.M., J. Mol. Biol. 196 (1987) 901-917);
(b) CDRs occurring at amino acid residues 24-34 (L1), 50-56 (L2), 89-97 (L3), 31-35b (H1), 50-65 (H2), and 95-102 (H3) (Kabat, E.A. et al., Sequences of Proteins of Immunological Interest, 5th ed. Public Health Service, National Institutes of Health, Bethesda, MD (1991), NIH Publication 91-3242.);
(c) antigen contacts occurring at amino acid residues 27c-36 (L1), 46-55 (L2), 89-96 (L3), 30-35b (H1), 47-58 (H2), and 93-101 (H3) (MacCallum et al. J. Mol. Biol. 262: 732-745 (1996)); and
(d) combinations of (a), (b), and/or (c), including amino acid residues 46-56 (L2), 47-56 (L2), 48-56 (L2), 49-56 (L2), 26-35 (H1), 26-35b (H1), 49-65 (H2), 93-102 (H3), and 94-102 (H3).

Unless otherwise indicated, HVR residues and other residues in the variable domain (e.g., FR residues) are numbered herein according to Kabat et al., *supra.*

In certain embodiments, a chimeric antibody is a humanized antibody. Typically, a non-human antibody is humanized to reduce immunogenicity to humans, while retaining the specificity and affinity of the parental non-human antibody. Generally, a humanized antibody comprises one or more variable domains in which HVRs, e.g., CDRs, (or portions thereof) are derived from a non-human antibody, and FRs (or portions thereof) are derived from human antibody sequences. In some embodiments, some FR residues in a humanized antibody are substituted with corresponding residues from a non-human antibody (e.g., the antibody from which the HVR residues are derived), e.g., to restore or improve antibody specificity or affinity.

Humanized antibodies and methods of making them are reviewed, e.g., in Almagro, J.C. and Fransson, J., Front. Biosci. 13 (2008) 1619-1633, and are further described, e.g., in Riechmann, I. et al., Nature 332 (1988) 323-329; Queen, C. et al., Proc. Natl. Acad. Sci. USA 86 (1989) 10029-10033; US 5, 821,337, US 7,527,791, US 6,982,321, and US 7,087,409; Kashmiri, S.V. et al., Methods 36 (2005) 25-34 (describing specificity determining region (SDR) grafting); Padlan, E.A., Mol. Immunol. 28 (1991) 489-498 (describing "resurfacing"); Dall'Acqua, W.F. et al., Methods 36 (2005) 43-60 (describing "FR shuffling"); and Osbourn, J. et al., Methods 36 (2005) 61-68 and Klimka, A. et al., Br. J. Cancer 83 (2000) 252-260 (describing the "guided selection" approach to FR shuffling).

Human framework regions that may be used for humanization include but are not limited to: framework regions selected using the "best-fit" method (see, e.g., Sims, M.J. et al., J. Immunol. 151 (1993) 2296-2308; framework regions derived from the consensus sequence of human antibodies of a particular subgroup of light or heavy chain variable regions (see, e.g., Carter, P. et al., Proc. Natl. Acad. Sci. USA 89 (1992) 4285-4289; and Presta, L.G. et al., J. Immunol. 151 (1993) 2623-2632); human mature (somatically mutated) framework regions or human germline framework regions (see, e.g., Almagro, J.C. and Fransson, J., Front. Biosci. 13 (2008) 1619-1633); and framework regions derived from screening FR libraries (see, e.g., Baca, M. et al., J. Biol. Chem. 272 (1997) 10678-10684 and Rosok, M.J. et al., J. Biol. Chem. 271 (19969 22611-22618).

### III.3. Human antibody derived binding sites

In certain embodiments, the binding site in the circular fusion polypeptide as reported herein is composed of an antibody heavy chain variable domain (VH) and an antibody light chain variable domain (VL). In certain embodiments, the variable domains are from a human antibody.

Human antibodies can be produced using various techniques known in the art. Human antibodies are described generally in van Dijk, M.A. and van de Winkel, J.G., Curr. Opin. Pharmacol. 5 (2001) 368-374 and in Lonberg, N., Curr. Opin. Immunol. 20 (2008) 450-459.

Human antibodies may be prepared by administering an immunogen to a transgenic animal that has been modified to produce intact human antibodies or intact antibodies with human variable regions in response to antigenic challenge. Such animals typically contain all or a portion of the human immunoglobulin loci, which replace the endogenous immunoglobulin loci, or which are present extrachromosomally or integrated randomly into the animal's chromosomes. In such transgenic mice, the endogenous immunoglobulin loci have generally been inactivated. For review of methods for obtaining human antibodies from transgenic animals, see Lonberg, N., Nat. Biotech. 23 (2005) 1117-1125. See also, e.g., US 6,075,181 and US 6,150,584 describing XFNOMOUSF™ technology; US 5,770,429 describing HUMAB® technology; US 7,041,870 describing K-M MOUSE® technology; US 2007/0061900, describing VELOCIMOUSE® technology; WO 2007/131676 describing an immunoreconstituted mouse). Human variable regions from intact antibodies generated by such animals may be further modified.

Human antibodies can also be made by hybridoma-based methods. Human myeloma and mouse-human heteromyeloma cell lines for the production of human monoclonal antibodies have been described (see, e.g., Kozbor, D., J. Immunol. 133 (1984) 3001-3005; Brodeur, B.R. et al., Monoclonal Antibody Production Techniques and Applications, Marcel Dekker, Inc., New York (1987), pp. 51-63; and Boerner, P. et al., J. Immunol. 147 (1991) 86-95). Human antibodies generated via human B-cell hybridoma technology are also described in Li, J. et al., Proc. Natl. Acad. Sci. USA 103 (2006) 3557-3562. Additional methods include those described, for example, in US 7,189,826 (describing production of monoclonal human IgM antibodies from hybridoma cell lines) and Ni, J., Xiandai Mianyixue 26 (2006) 265-268 (describing human-human hybridomas). Human hybridoma technology (Trioma technology) is also described in Vollmers, H.P. and Brandlein, S., Histology and Histopathology 20 (2005) 927-937 and Vollmers, H.P. and Brandlein, S., Methods and Findings in Experimental and Clinical Pharmacology 27 (2005) 185-191.

Human antibodies may also be generated by isolating Fv clone variable domain sequences selected from human-derived phage display libraries. Such variable domain sequences may then be combined with a desired human constant domain. Techniques for selecting human antibodies from antibody libraries are described below.

### III.4. Library-derived antibody binding sites

In certain embodiments, the binding site in the circular fusion polypeptide as reported herein is composed of an antibody heavy chain variable domain (VH) and an antibody light chain variable domain (VL). In certain embodiments, the variable domains are isolated by screening combinatorial libraries for antibodies with the desired activity or activities.

For example, a variety of methods are known in the art for generating phage display libraries and screening such libraries for antibodies possessing the desired binding characteristics. Such methods are reviewed, e.g., in Hoogenboom, H.R. et al., Methods in Molecular Biology 178 (2001) 1-37 and further described, e.g., in the McCafferty, J. et al., Nature 348 (1990) 552-554; Clackson, T. et al., Nature 352 (1991) 624-628; Marks, J.D. et al., J. Mol. Biol. 222 (1992) 581-597; Marks, J.D. and Bradbury, A., Methods in Molecular Biology 248 (2003) 161-175; Sidhu, S.S. et al., J. Mol. Biol. 338 (2004) 299-310; Lee, C.V. et al., J. Mol. Biol. 340 (2004) 1073-1093; Fellouse, F.A., Proc. Natl. Acad. Sci. USA 101 (2004) 12467-12472; and Lee, C.V. et al., J. Immunol. Methods 284 (2004) 119-132.

In certain phage display methods, repertoires of VH and VL genes are separately cloned by polymerase chain reaction (PCR) and recombined randomly in phage libraries, which can then be screened for antigen-binding phage as described in Winter, G. et al., Ann. Rev. Immunol. 12 (1994) 433-455. Phage typically display antibody fragments, either as single-chain Fv (scFv) fragments or as Fab fragments. Libraries from immunized sources provide high-affinity binding sites to the immunogen without the requirement of constructing hybridomas. Alternatively, the naive repertoire can be cloned (e.g., from human) to provide a single source of antibodies to a wide range of non-self- and also self-antigens without any immunization as described by Griffiths, A.D. et al., EMBO J. 12 (1993) 725-734. Finally, naive libraries can also be made synthetically by cloning non-rearranged V-gene segments from stem cells, and using PCR primers containing random sequence to encode the highly variable CDR3 regions and to accomplish rearrangement *in vitro,* as described by Hoogenboom, H.R. and Winter, G., J. Mol. Biol. 227 (1992) 381-388. Patent publications describing human antibody phage libraries include, for example: US 5,750,373, and US 2005/0079574, US 2005/0119455, US 2005/0266000, US 2007/0117126, US 2007/0160598, US 2007/0237764, US 2007/0292936, and US 2009/0002360.

Antibodies or antibody fragments isolated from human antibody libraries are considered human antibodies or human antibody fragments herein.

### IV. Hetero-multi(di)merization domains

For assuring the correct association of the individual circular fusion polypeptides to form hetero-multicircular fusion polypeptides different technologies can be used. One of them is the so called "knob-in-hole" engineering (see, e.g., US 5,731,168). Multicircular fusion polypeptides may also be made by engineering electrostatic steering effects for making Fc-heterodimeric molecules (WO 2009/089004); crosslinking two or more circular fusion polypeptides (see, e.g., US 4,676,980, and Brennan, M. et al., Science 229 (1985) 81-83); using leucine zippers to produce bicircular fusion polypeptides (see, e.g., Kostelny, S.A. et al., J. Immunol. 148 (1992) 1547-1553).

Several approaches for CH3-modifications in order to support heterodimerization have been described, for example in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954, WO 2013/096291, which are herein included by reference.

Typically, in the approaches known in the art, the CH3 domain of the first heavy chain and the CH3 domain of the second heavy chain are both engineered in a complementary manner so that the heavy chain comprising one engineered CH3 domain can no longer homodimerize with another heavy chain of the same structure (e.g. a CH3-engineered first heavy chain can no longer homodimerize with another CH3-engineered first heavy chain; and a CH3-engineered second heavy chain can no longer homodimerize with another CH3-engineered second heavy chain). Thereby the heavy chain comprising one engineered CH3 domain is forced to heterodimerize with another heavy chain comprising the CH3 domain, which is engineered in a complementary manner. For this embodiment, the CH3 domain of the first heavy chain and the CH3 domain of the second heavy chain are engineered in a complementary manner by amino acid substitutions, such that the first heavy chain and the second heavy chain are forced to heterodimerize, whereas the first heavy chain and the second heavy chain can no longer homodimerize (e.g. for steric reasons).

The different approaches for supporting heavy chain heterodimerization known in the art, that were cited and included above, are contemplated as different alternatives used in providing a multispecific antibody as reported herein, which comprises a "non-crossed Fab region" derived from a first antibody, which specifically binds to a first antigen, and a "crossed Fab region" derived from a second antibody, which specifically binds to a second antigen, in combination with the particular amino acid substitutions described above.

The CH3 domains of the multicircular fusion polypeptide (Contorsbody) as reported herein can be altered by the "knob-into-holes" technology which is described in detail with several examples in e.g. WO 96/027011, Ridgway, J.B., et al., Protein Eng. 9 (1996) 617-621; and Merchant, A.M., et al., Nat. Biotechnol. 16 (1998) 677-681. In this method the interaction surfaces of the two CH3 domains are altered to increase the heterodimerization of both heavy chains containing these two CH3 domains. Each of the two CH3 domains (of the two heavy chains) can be the "knob", while the other is the "hole". The introduction of a disulfide bridge further stabilizes the heterodimers (Merchant, A.M., et al., Nature Biotech. 16 (1998) 677-681; Atwell, S., et al., J. Mol. Biol. 270 (1997) 26-35) and increases the yield.

In one preferred embodiment the multicircular fusion polypeptide as reported herein comprises a T366W mutation in the CH3 domain of the "knobs chain" (i.e. first circular fusion polypeptide) and T366S, L368A, Y407V mutations in the CH3 domain of the "hole-chain" (i.e. second circular fusion polypeptide) (numbering according to Kabat EU index). An additional interchain disulfide bridge between the CH3 domains can also be used (Merchant, A.M., et al., Nature Biotech. 16 (1998) 677-681) e.g. by introducing a Y349C mutation into the CH3 domain of the "knobs chain" and a E356C mutation or a S354C mutation into the CH3 domain of the "hole chain". Thus in a another preferred embodiment, the multicircular fusion polypeptide as reported herein comprises the Y349C and T366W mutations in one of the two CH3 domains and the E356C, T366S, L368A and Y407V mutations in the other of the two CH3 domains or the multicircular fusion polypeptide as reported herein comprises the Y349C and T366W mutations in one of the two CH3 domains and the S354C, T366S, L368A and Y407V mutations in the other of the two CH3 domains (the additional Y349C mutation in one CH3 domain and the additional E356C or S354C mutation in the other CH3 domain forming a interchain disulfide bridge) (numbering according to Kabat EU index).

But also other knobs-in-holes technologies as described by EP 1 870 459A1, can be used alternatively or additionally. In one embodiment the multicircular fusion polypeptide as reported herein comprises the R409D and K370E mutations in the CH3 domain of the "knobs chain" and the D399K and E357K mutations in the CH3 domain of the "hole-chain" (numbering according to Kabat EU index).

In one embodiment the multicircular fusion polypeptide as reported herein comprises a T366W mutation in the CH3 domain of the "knobs chain" and the T366S, L368A and Y407V mutations in the CH3 domain of the "hole chain" and additionally the R409D and K370E mutations in the CH3 domain of the "knobs chain" and the D399K and E357K mutations in the CH3 domain of the "hole chain" (numbering according to the Kabat EU index).

In one embodiment the multicircular fusion polypeptide as reported herein comprises the Y349C and T366W mutations in one of the two CH3 domains and the S354C, T366S, L368A and Y407V mutations in the other of the two CH3 domains, or the multicircular fusion polypeptide as reported herein comprises the Y349C and T366W mutations in one of the two CH3 domains and the S354C, T366S, L368A and Y407V mutations in the other of the two CH3 domains and additionally the R409D and K370E mutations in the CH3 domain of the "knobs chain" and the D399K and E357K mutations in the CH3 domain of the "hole chain" (numbering according to the Kabat EU index).

Apart from the "knob-into-hole technology" other techniques for modifying the CH3 domains of the heavy chains of a multicircular fusion polypeptide to enforce heterodimerization are known in the art. These technologies, especially the ones described in WO 96/27011, WO 98/050431, EP 1870459, WO 2007/110205, WO 2007/147901, WO 2009/089004, WO 2010/129304, WO 2011/90754, WO 2011/143545, WO 2012/058768, WO 2013/157954 and WO 2013/096291 are contemplated herein as alternatives to the "knob-into-hole technology" in combination with a multicircular fusion polypeptide as reported herein.

In one embodiment of a multicircular fusion polypeptide as reported herein the approach described in EP 1870459 is used to support heterodimerization of the first heavy chain and the second heavy chain of the multicircular fusion polypeptide. This approach is based on the introduction of charged amino acids with opposite charges at specific amino acid positions in the CH3/CH3-domain-interface between both, the first and the second heavy chain.

Accordingly, this embodiment relates to a multicircular fusion polypeptide as reported herein, wherein in the tertiary structure of the antibody the CH3 domain of the first heavy chain and the CH3 domain of the second heavy chain form an interface that is located between the respective antibody CH3 domains, wherein the respective amino acid sequences of the CH3 domain of the first heavy chain and the CH3 domain of the second heavy chain each comprise a set of amino acids that is located within said interface in the tertiary structure of the circular fusion polypeptide, wherein from the set of amino acids that is located in the interface in the CH3 domain of one heavy chain a first amino acid is substituted by a positively charged amino acid and from the set of amino acids that is located in the interface in the CH3 domain of the other heavy chain a second amino acid is substituted by a negatively charged amino acid. The multicircular fusion polypeptide according to this embodiment is herein also referred to as "CH3(+/-)-engineered multicircular fusion polypeptide" (wherein the abbreviation "+/-" stands for the oppositely charged amino acids that were introduced in the respective CH3 domains).

In one embodiment of said CH3(+/-)-engineered multicircular fusion polypeptide as reported herein the positively charged amino acid is selected from K, R and H, and the negatively charged amino acid is selected from E or D.

In one embodiment of said CH3(+/-)-engineered multicircular fusion polypeptide as reported herein the positively charged amino acid is selected from K and R, and the negatively charged amino acid is selected from E or D.

In one embodiment of said CH3(+/-)-engineered multicircular fusion polypeptide as reported herein the positively charged amino acid is K, and the negatively charged amino acid is E.

In one embodiment of said CH3(+/-)-engineered multicircular fusion polypeptide as reported herein in the CH3 domain of one heavy chain the amino acid R at position 409 is substituted by D and the amino acid K at position is substituted by E, and in the CH3 domain of the other heavy chain the amino acid D at position 399 is substituted by K and the amino acid E at position 357 is substituted by K (numbering according to Kabat EU index).

In one embodiment of a multicircular fusion polypeptide as reported herein the approach described in WO 2013/157953 is used to support heterodimerization of the first heavy chain and the second heavy chain of the multicircular fusion polypeptide. In one embodiment of said multicircular fusion polypeptide as reported herein, in the CH3 domain of one heavy chain the amino acid T at position 366 is substituted by K, and in the CH3 domain of the other heavy chain the amino acid L at position 351 is substituted by D (numbering according to Kabat EU index). In another embodiment of said multicircular fusion polypeptide as reported herein, in the CH3 domain of one heavy chain the amino acid T at position 366 is substituted by K and the amino acid L at position 351 is substituted by K, and in the CH3 domain of the other heavy chain the amino acid L at position 351 is substituted by D (numbering according to Kabat EU index).

In another embodiment of said multicircular fusion polypeptide as reported herein, in the CH3 domain of one heavy chain the amino acid T at position 366 is substituted by K and the amino acid L at position 351 is substituted by K, and in the CH3 domain of the other heavy chain the amino acid L at position 351 is substituted by D (numbering according to Kabat EU index). Additionally at least one of the following substitutions is comprised in the CH3 domain of the other heavy chain: the amino acid Y at position 349 is substituted by E, the amino acid Y at position 349 is substituted by D and the amino acid L at position 368 is substituted by E (numbering according to Kabat EU index). In one embodiment the amino acid L at position 368 is substituted by E (numbering according to Kabat EU index).

In one embodiment of a multicircular fusion polypeptide as reported herein the approach described in WO 2012/058768 is used to support heterodimerization of the first circular fusion polypeptide and the second circular fusion polypeptide of the multicircular fusion polypeptide. In one embodiment of said multicircular fusion polypeptide as reported herein, in the CH3 domain of one heavy chain the amino acid L at position 351 is substituted by Y and the amino acid Y at position 407 is substituted by A, and in the CH3 domain of the other heavy chain the amino acid T at position 366 is substituted by A and the amino acid K at position 409 is substituted by F (numbering according to Kabat EU index). In another embodiment, in addition to the aforementioned substitutions, in the CH3 domain of the other heavy chain at least one of the amino acids at positions 411 (originally T), 399 (originally D), 400 (originally S), 405 (originally F), 390 (originally N) and 392 (originally K) is substituted (numbering according to Kabat EU index). Preferred substitutions are:
- substituting the amino acid T at position 411 by an amino acid selected from N, R, Q, K, D, E and W (numbering according to Kabat EU index),
- substituting the amino acid D at position 399 by an amino acid selected from R, W, Y, and K (numbering according to Kabat EU index),
- substituting the amino acid S at position 400 by an amino acid selected from E, D, R and K (numbering according to Kabat EU index),
- substituting the amino acid F at position 405 by an amino acid selected from I, M, T, S, V and W (numbering according to Kabat EU index;
- substituting the amino acid N at position 390 by an amino acid selected from R, K and D (numbering according to Kabat EU index; and
- substituting the amino acid K at position 392 by an amino acid selected from V, M, R, L, F and E (numbering according to Kabat EU index).

In another embodiment of said multicircular fusion polypeptide as reported herein (engineered according to WO 2012/058768), in the CH3 domain of one heavy chain the amino acid L at position 351 is substituted by Y and the amino acid Y at position 407 is substituted by A, and in the CH3 domain of the other heavy chain the amino acid T at position 366 is substituted by V and the amino acid K at position 409 is substituted by F (numbering according to Kabat EU index). In another embodiment of said multicircular fusion polypeptide as reported herein, in the CH3 domain of one heavy chain the amino acid Y at position 407 is substituted by A, and in the CH3 domain of the other heavy chain the amino acid T at position 366 is substituted by A and the amino acid K at position 409 is substituted by F (numbering according to Kabat EU index). In said last aforementioned embodiment, in the CH3 domain of said other heavy chain the amino acid K at position 392 is substituted by E, the amino acid T at position 411 is substituted by E, the amino acid D at position 399 is substituted by R and the amino acid S at position 400 is substituted by R (numbering according to Kabat EU index).

In one embodiment of a multicircular fusion polypeptide as reported herein the approach described in WO 2011/143545 is used to support heterodimerization of the first circular fusion polypeptide and the second circular fusion polypeptide of the multicircular fusion polypeptide. In one embodiment of said multicircular fusion polypeptide as reported herein, amino acid modifications in the CH3 domains of both heavy chains are introduced at positions 368 and/or 409 (numbering according to Kabat EU index).

In one embodiment of a multicircular fusion polypeptide as reported herein the approach described in WO 2011/090762 is used to support heterodimerization of the first circular fusion polypeptide and the second circular fusion polypeptide of the multicircular fusion polypeptide. WO 2011/090762 relates to amino acid modifications according to the "knob-into-hole" technology. In one embodiment of said CH3(KiH)-engineered multicircular fusion polypeptide as reported herein, in the CH3 domain of one heavy chain the amino acid T at position 366 is substituted by W, and in the CH3 domain of the other heavy chain the amino acid Y at position 407 is substituted by A (numbering according to Kabat EU index). In another embodiment of said CH3(KiH)-engineered multicircular fusion polypeptide as reported herein, in the CH3 domain of one heavy chain the amino acid T at position 366 is substituted by Y, and in the CH3 domain of the other heavy chain the amino acid Y at position 407 is substituted by T (numbering according to Kabat EU index).

In one embodiment of a multicircular fusion polypeptide as reported herein, which is of IgG2 isotype, the approach described in WO 2011/090762 is used to support heterodimerization of the first heavy chain and the second heavy chain of the multicircular fusion polypeptide.

In one embodiment of a multicircular fusion polypeptide as reported herein, the approach described in WO 2009/089004 is used to support heterodimerization of the first circular fusion polypeptide and the second circular fusion polypeptide of the multicircular fusion polypeptide. In one embodiment of said multicircular fusion polypeptide as reported herein, in the CH3 domain of one heavy chain the amino acid K or N at position 392 is substituted by a negatively charged amino acid (in one preferred embodiment by E or D, in one preferred embodiment by D), and in the CH3 domain of the other heavy chain the amino acid D at position 399 the amino acid E or D at position 356 or the amino acid E at position 357 is substituted by a positively charged amino acid (in one preferred embodiment K or R, in one preferred embodiment by K, in one preferred embodiment the amino acids at positions 399 or 356 are substituted by K) (numbering according to Kabat EU index). In one further embodiment, in addition to the aforementioned substitutions, in the CH3 domain of the one heavy chain the amino acid K or R at position 409 is substituted by a negatively charged amino acid (in one preferred embodiment by E or D, in one preferred embodiment by D) (numbering according to Kabat EU index). In one even further embodiment, in addition to or alternatively to the aforementioned substitutions, in the CH3 domain of the one heavy chain the amino acid K at position 439 and/or the amino acid K at position 370 is substituted independently from each other by a negatively charged amino acid (in one preferred embodiment by E or D, in one preferred embodiment by D) (numbering according to Kabat EU index).

In one embodiment of a multicircular fusion polypeptide as reported herein, the approach described in WO 2007/147901 is used to support heterodimerization of the first circular fusion polypeptide and the second circular fusion polypeptide of the multicircular fusion polypeptide. In one embodiment of said multicircular fusion polypeptide as reported herein, in the CH3 domain of one heavy chain the amino acid K at position 253 is substituted by E, the amino acid D at position 282 is substituted by K and the amino acid K at position 322 is substituted by D, and in the CH3 domain of the other heavy chain the amino acid D at position 239 is substituted by K, the amino acid E at position 240 is substituted by K and the amino acid K at position 292 is substituted by D (numbering according to Kabat EU index).

In one embodiment of a multicircular fusion polypeptide as reported herein, the approach described in WO 2007/110205 is used to support heterodimerization of the first circular fusion polypeptide and the second circular fusion polypeptide of the multicircular fusion polypeptide.

In one embodiment of all aspects and embodiments as reported herein the multicircular fusion polypeptide is a bicircular fusion polypeptide or a tricircular fusion polypeptide. In one preferred embodiment the multicircular fusion polypeptide is a bispecific bicircular fusion polypeptide.

In one embodiment of all aspects as reported herein, the multicircular fusion polypeptide has a constant domain structure of an IgG type antibody. In one further embodiment of all aspects as reported herein, the multicircular fusion polypeptide is characterized in that said multicircular fusion polypeptide comprises an Fc-region of human subclass IgG1, or of human subclass IgG1 with the mutations L234A and L235A and optionally P329G. In one further embodiment of all aspects as reported herein, the multicircular fusion polypeptide is characterized in that said multicircular fusion polypeptide comprises an Fc-region of human subclass IgG2. In one further embodiment of all aspects as reported herein, the multicircular fusion polypeptide is characterized in that said multicircular fusion polypeptide comprises an Fc-region of human subclass IgG3. In one further embodiment of all aspects as reported herein, the multicircular fusion polypeptide is characterized in that said multicircular fusion polypeptide comprises an Fc-region of human subclass IgG4 or, of human subclass IgG4 with the additional mutation S228P and L235E and optionally P329G.

### V. Variants

In certain embodiments, amino acid sequence variants of the circular fusion polypeptide provided herein are contemplated. For example, it may be desirable to improve the binding affinity and/or other biological properties of the circular fusion polypeptide. Amino acid sequence variants of a circular fusion polypeptide may be prepared by introducing appropriate modifications into the nucleotide sequence encoding the circular fusion polypeptide, or by peptide synthesis. Such modifications include, for example, deletions from, and/or insertions into and/or substitutions of residues within the amino acid sequences of the circular fusion polypeptide. Any combination of deletion, insertion, and substitution can be made to arrive at the final construct, provided that the final construct possesses the desired characteristics, e.g., antigen-binding.

### a) Substitution, Insertion, and Deletion Variants

In certain embodiments, circular fusion polypeptide variants having one or more amino acid substitutions are provided. Sites of interest for substitutional mutagenesis include the HVRs and FRs. Conservative substitutions are shown in the following Table under the heading of "preferred substitutions". More substantial changes are provided in the following Table under the heading of "exemplary substitutions," and as further described below in reference to amino acid side chain classes. Amino acid substitutions may be introduced into a circular fusion polypeptide of interest and the products screened for a desired activity, e.g., retained/improved antigen binding, decreased immunogenicity, or improved ADCC or CDC.

| **Original Residue** | **Exemplary Substitutions** | **Preferred Substitutions** |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

One type of substitutional variant involves substituting one or more hypervariable region residues of a parent circular fusion polypeptide (e.g. comprising a binding site derived from a humanized or human antibody). Generally, the resulting variant(s) selected for further study will have modifications (e.g., improvements) in certain biological properties (e.g., increased affinity, reduced immunogenicity) relative to the parent circular fusion polypeptide and/or will have substantially retained certain biological properties of the parent circular fusion polypeptide. An exemplary substitutional variant is an affinity matured circular fusion polypeptide, which may be conveniently generated, e.g., using phage display-based affinity maturation techniques such as those described herein. Briefly, one or more HVR residues are mutated and the variant circular fusion polypeptide displayed on phage and screened for a particular biological activity (e.g. binding affinity).

Alterations (e.g., substitutions) may be made in HVRs, e.g., to improve circular fusion polypeptide affinity. Such alterations may be made in HVR "hotspots," i.e., residues encoded by codons that undergo mutation at high frequency during the somatic maturation process (see, e.g., Chowdhury, P.S., Methods Mol. Biol. 207 (2008) 179-196), and/or residues that contact antigen, with the resulting variant VH or VL being tested for binding affinity. Affinity maturation by constructing and reselecting from secondary libraries has been described, e.g., in Hoogenboom, H.R. et al. in Methods in Molecular Biology 178 (2002) 1-37. In some embodiments of affinity maturation, diversity is introduced into the variable genes chosen for maturation by any of a variety of methods (e.g., error-prone PCR, chain shuffling, or oligonucleotide-directed mutagenesis). A secondary library is then created. The library is then screened to identify any circular fusion polypeptide variants with the desired affinity. Another method to introduce diversity involves HVR-directed approaches, in which several HVR residues (e.g., 4-6 residues at a time) are randomized. HVR residues involved in antigen binding may be specifically identified, e.g., using alanine scanning mutagenesis or modeling. CDR-H3 and CDR-L3 in particular are often targeted.

In certain embodiments, substitutions, insertions, or deletions may occur within one or more HVRs so long as such alterations do not substantially reduce the ability of the circular fusion polypeptide to bind the intended target. For example, conservative alterations (e.g., conservative substitutions as provided herein) that do not substantially reduce binding affinity may be made in HVRs. Such alterations may, for example, be outside of antigen contacting residues in the HVRs. In certain embodiments of the variant VH and VL sequences provided above, each HVR either is unaltered, or contains no more than one, two or three amino acid substitutions.

A useful method for identification of residues or regions of a circular fusion polypeptide that may be targeted for mutagenesis is called "alanine scanning mutagenesis" as described by Cunningham, B.C. and Wells, J.A., Science 244 (1989) 1081-1085. In this method, a residue or group of target residues (e.g., charged residues such as arg, asp, his, lys, and glu) are identified and replaced by a neutral or negatively charged amino acid (e.g., alanine or polyalanine) to determine whether the interaction of the circular fusion polypeptide with its target is affected. Further substitutions may be introduced at the amino acid locations demonstrating functional sensitivity to the initial substitutions. Alternatively, or additionally, a crystal structure of a target-circular fusion polypeptide complex to identify contact points between the circular fusion polypeptide and its target. Such contact residues and neighboring residues may be targeted or eliminated as candidates for substitution. Variants may be screened to determine whether they contain the desired properties.

Amino acid sequence insertions include amino- and/or carboxyl-terminal fusions ranging in length from one residue to polypeptides containing a hundred or more residues, as well as intrasequence insertions of single or multiple amino acid residues. Examples of terminal insertions include a circular fusion polypeptide with an N-terminal methionyl residue. Other insertional variants of the circular fusion polypeptide molecule include the fusion to the N- or C-terminus of the circular fusion polypeptide to an enzyme (e.g. for ADEPT) or a polypeptide which increases the serum half-life of the circular fusion polypeptide.

### b) Glycosylation variants

In certain embodiments, a circular fusion polypeptide provided herein is altered to increase or decrease the extent to which the circular fusion polypeptide is glycosylated. Addition or deletion of glycosylation sites to a circular fusion polypeptide may be conveniently accomplished by altering the amino acid sequence such that one or more glycosylation sites is created or removed.

Where the circular fusion polypeptide comprises an Fc-region, the carbohydrate attached thereto may be altered. Native Fc-region comprising circular fusion polypeptides produced by mammalian cells typically comprise a branched, biantennary oligosaccharide that is generally attached by an N-linkage to Asn297 of the CH2 domain of the Fc-region (see, e.g., Wright, A. and Morrison, S.L., TIBTECH 15 (1997) 26-32). The oligosaccharide may include various carbohydrates, e.g., mannose, N-acetyl glucosamine (GlcNAc), galactose, and sialic acid, as well as a fucose attached to a GlcNAc in the "stem" of the biantennary oligosaccharide structure. In some embodiments, modifications of the oligosaccharide in a circular fusion polypeptide of the invention may be made in order to create circular fusion polypeptide variants with certain improved properties.

In one embodiment, circular fusion polypeptide variants are provided having a carbohydrate structure that lacks fucose attached (directly or indirectly) to an Fc-region. For example, the amount of fucose in such circular fusion polypeptide may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc-region (EU numbering of Fc-region residues); however, Asn297 may also be located about ± 3 amino acids upstream or downstream of position 297, i.e., between positions 294 and 300, due to minor sequence variations in antibodies. Such fucosylation variants may have improved ADCC function (see, e.g., US 2003/0157108; US 2004/0093621). Examples of publications related to "defucosylated" or "fucose-deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO 2005/053742; WO 2002/031140; Okazaki, A. et al., J. Mol. Biol. 336 (2004) 1239-1249; Yamane-Ohnuki, N. et al., Biotech. Bioeng. 87 (2004) 614-622. Examples of cell lines capable of producing defucosylated circular fusion polypeptides include Lecl3 CHO cells deficient in protein fucosylation (Ripka, J. et al., Arch. Biochem. Biophys. 249 (1986) 533-545; US 2003/0157108; and WO 2004/056312, especially at Example 11), and knockout cell lines, such as alpha-1,6-fucosyltransferase gene, *FUT8,* knockout CHO cells (see, e.g., Yamane-Ohnuki, N. et al., Biotech. Bioeng. 87 (2004) 614-622; Kanda, Y. et al., Biotechnol. Bioeng. 94 (2006) 680-688; WO 2003/085107).

Circular fusion polypeptide variants are further provided with bisected oligosaccharides, e.g., in which a biantennary oligosaccharide attached to the Fc-region of the circular fusion polypeptide is bisected by GlcNAc. Such circular fusion polypeptide variants may have reduced fucosylation and/or improved ADCC function. Examples of such antibody variants are described, e.g., in WO 2003/011878; US 6,602,684; and US 2005/0123546. Circular fusion polypeptide variants with at least one galactose residue in the oligosaccharide attached to the Fc-region are also provided. Such circular fusion polypeptide variants may have improved CDC function. Such antibody variants are described, e.g., in WO 1997/30087; WO 1998/58964; and WO 1999/22764.

### c) Fc-region variants

In certain embodiments, one or more amino acid modifications may be introduced into the Fc-region of a circular fusion polypeptide provided herein, thereby generating an Fc-region variant. The Fc-region variant may comprise a human Fc-region sequence (*e.g.,* a human IgG1, IgG2, IgG3 or IgG4 Fc-region) comprising an amino acid modification (e.g. a substitution) at one or more amino acid positions.

In certain embodiments, the invention contemplates a circular fusion polypeptide variant that possesses some but not all effector functions, which make it a desirable candidate for applications in which the half-life *in vivo* is important yet certain effector functions (such as complement and ADCC) are unnecessary or deleterious. *In vitro* and/or *in vivo* cytotoxicity assays can be conducted to confirm the reduction/depletion of CDC and/or ADCC activities. For example, Fc receptor (FcR) binding assays can be conducted to ensure that the circular fusion polypeptide lacks FcyR binding (hence likely lacking ADCC activity), but retains FcRn binding ability. The primary cells for mediating ADCC, NK cells, express Fc(RIII only, whereas monocytes express FcyRI, FcγRII and FcγRIII. FcR expression on hematopoietic cells is summarized in Table 3 on page 464 of Ravetch, J.V. and Kinet, J.P., Annu. Rev. Immunol. 9 (1991) 457-492. Non-limiting examples of *in vitro* assays to assess ADCC activity of a molecule of interest is described in US 5,500,362 (see, e.g. Hellstrom, I. et al., Proc. Natl. Acad. Sci. USA 83 (1986) 7059-7063; and Hellstrom, I. et al., Proc. Natl. Acad. Sci. USA 82 (1985) 1499-1502); US 5,821,337 (see Bruggemann, M. et al., J. Exp. Med. 166 (1987) 1351-1361). Alternatively, non-radioactive assays methods may be employed (see, for example, ACTI™ non-radioactive cytotoxicity assay for flow cytometry (CellTechnology, Inc. Mountain View, CA; and CytoTox 96® non-radioactive cytotoxicity assay (Promega, Madison, WI). Useful effector cells for such assays include peripheral blood mononuclear cells (PBMC) and Natural Killer (NK) cells. Alternatively, or additionally, ADCC activity of the molecule of interest may be assessed *in vivo, e.g.,* in an animal model such as that disclosed in Clynes, R. et al., Proc. Natl. Acad. Sci. USA 95 (1998) 652-656. C1q binding assays may also be carried out to confirm that the antibody is unable to bind C1q and hence lacks CDC activity (see, e.g., C1q and C3c binding ELISA in WO 2006/029879 and WO 2005/100402). To assess complement activation, a CDC assay may be performed (see, for example, Gazzano-Santoro, H. et al., J. Immunol. Methods 202 (1996) 163-171; Cragg, M.S. et al., Blood 101 (2003) 1045-1052; and Cragg, M.S. and M.J. Glennie, Blood 103 (2004) 2738-2743). FcRn binding and *in vivo* clearance/half-life determinations can also be performed using methods known in the art (see, e.g., Petkova, S.B. et al., Int. Immunol. 18 (2006: 1759-1769).

Fc-region comprising circular fusion polypeptides with reduced effector function include those with substitution of one or more of Fc-region residues 238, 265, 269, 270, 297, 327 and 329 (US 6,737,056). Such Fc-region mutants include Fc-region mutants with substitutions at two or more of amino acid positions 265, 269, 270, 297 and 327, including the so-called "DANA" Fc-region mutant with substitution of residues 265 and 297 to alanine (US 7,332,581).

Certain Fc-region comprising circular fusion polypeptide variants with improved or diminished binding to FcRs are described (see, e.g., US 6,737,056; WO 2004/056312, and Shields, R.L. et al., J. Biol. Chem. 276 (2001) 6591-6604).

In certain embodiments, a circular fusion polypeptide variant comprises an Fc-region with one or more amino acid substitutions which improve ADCC, e.g., substitutions at positions 298, 333, and/or 334 of the Fc-region (EU numbering of residues).

In some embodiments, alterations are made in the Fc-region that result in altered (*i.e.,* either improved or diminished) C1q binding and/or Complement Dependent Cytotoxicity (CDC), e.g., as described in US 6,194,551, WO 99/51642, and Idusogie, E.E. et al., J. Immunol. 164 (2000) 4178-4184.

Antibodies with increased half-lives and improved binding to the neonatal Fc receptor (FcRn), which is responsible for the transfer of maternal IgGs to the fetus (Guyer, R.L. et al., J. Immunol. 117 (1976) 587-593, and Kim, J.K. et al., J. Immunol. 24 (1994) 2429-2434), are described in US 2005/0014934. Those antibodies comprise an Fc-region with one or more substitutions therein which improve binding of the Fc-region to FcRn. Such Fc variants include those with substitutions at one or more of Fc-region residues: 238, 256, 265, 272, 286, 303, 305, 307, 311, 312, 317, 340, 356, 360, 362, 376, 378, 380, 382, 413, 424 or 434, e.g., substitution of Fc-region residue 434 (US 7,371,826).

See also Duncan, A.R. and Winter, G., Nature 322 (1988) 738-740; US 5,648,260; US 5,624,821; and WO 94/29351 concerning other examples of Fc-region variants.

In one embodiment of all aspects the circular fusion polypeptide comprises (all positions according to EU index of Kabat)
i) a homodimeric Fc-region of the human IgG1 subclass optionally with the mutations P329G, L234A and L235A, or
ii) a homodimeric Fc-region of the human IgG4 subclass optionally with the mutations P329G, S228P and L235E, or
iii) a homodimeric Fc-region of the human IgG1 subclass optionally with the mutations P329G, L234A, L235A, I253A, H310A, and H435A, or optionally with the mutations P329G, L234A, L235A, H310A, H433A, and Y436A, or
iv) a heterodimeric Fc-region whereof
   a) one Fc-region polypeptide comprises the mutation T366W, and the other Fc-region polypeptide comprises the mutations T366S, L368A and Y407V, or
   b) one Fc-region polypeptide comprises the mutations T366W and Y349C, and the other Fc-region polypeptide comprises the mutations T366S, L368A, Y407V, and S354C, or
   c) one Fc-region polypeptide comprises the mutations T366W and S354C, and the other Fc-region polypeptide comprises the mutations T366S, L368A, Y407V and Y349C, or
v) a heterodimeric Fc-region of the human IgGI subclass whereof both Fc-region polypeptides comprise the mutations P329G, L234A and L235A and
   a) one Fc-region polypeptide comprises the mutation T366W, and the other Fc-region polypeptide comprises the mutations T366S, L368A and Y407V, or
   b) one Fc-region polypeptide comprises the mutations T366W and Y349C, and the other Fc-region polypeptide comprises the mutations T366S, L368A, Y407V, and S354C, or
   c) one Fc-region polypeptide comprises the mutations T366W and S354C, and the other Fc-region polypeptide comprises the mutations T366S, L368A, Y407V and Y349C,
   or
vi) a heterodimeric Fc-region of the human IgG4 subclass whereof both Fc-region polypeptides comprise the mutations P329G, S228P and L235E and
   a) one Fc-region polypeptide comprises the mutation T366W, and the other Fc-region polypeptide comprises the mutations T366S, L368A and Y407V, or
   b) one Fc-region polypeptide comprises the mutations T366W and Y349C, and the other Fc-region polypeptide comprises the mutations T366S, L368A, Y407V, and S354C, or
   c) one Fc-region polypeptide comprises the mutations T366W and S354C, and the other Fc-region polypeptide comprises the mutations T366S, L368A, Y407V and Y349C,
   or
vii) a combination of one of i), ii), and iii) with one of vi), v) and vi).

In one embodiment of all aspects as reported herein, a circular fusion polypeptide comprising a CH3 domain, comprises an additional C-terminal glycine-lysine dipeptide (G446 and K447, numbering according to Kabat EU index). In one embodiment of all aspects as reported herein, a circular fusion polypeptide comprising a CH3 domain comprises an additional C-terminal glycine residue (G446, numbering according to Kabat EU index).

The circular fusion polypeptide as reported herein comprises in one embodiment an Fc-region characterized by being of human subclass IgG1 with mutations PVA236, L234A/L235A, and/or GLPSS331 (numbering according to EU index of Kabat), or of subclass IgG4. In a further embodiment, the circular fusion polypeptide is characterized by comprising an Fc-region being of any IgG class, in one embodiment being of the IgGI or IgG4 subclass, containing at least one mutation in E233, L234, L235, G236, D270, N297, E318, K320, K322, A327, A330, P331 and/or P329 (numbering according to EU index of Kabat). It is further in one embodiment that the circular fusion polypeptide comprises an Fc-region of the human IgG4 subclass which contains the mutation S228P, or the mutations S228P and L235E (Angal, S., et al., Mol. Immunol. 30 (1993) 105-108) (numbering according to EU index of Kabat).

The C-terminus of the Fc-region polypeptides comprised in the circular fusion polypeptide as reported herein can be a complete C-terminus ending with the amino acid residues PGK. The C-terminus can be a shortened C-terminus in which one or two of the C-terminal amino acid residues have been removed. In one preferred embodiment the C-terminus is a shortened C-terminus ending with the amino acid residues PG.

### d) Cysteine engineered circular fusion polypeptides

In certain embodiments, it may be desirable to create cysteine engineered circular fusion polypeptides, in which one or more residues of are substituted with cysteine residues. In particular embodiments, the substituted residues occur at accessible sites of the circular fusion polypeptide. By substituting those residues with cysteine, reactive thiol groups are thereby positioned at accessible sites of the circular fusion polypeptide and may be used to conjugate the circular fusion polypeptide to other moieties, such as drug moieties or linker-drug moieties, to create an immunoconjugate, as described further herein. In certain embodiments, any one or more of the following residues of a circular fusion polypeptide as reported herein, especially of a Contorsbody, may be substituted with cysteine: V205 (Kabat numbering) of the light chain; A118 (EU numbering) of the heavy chain; and S400 (EU numbering) of the heavy chain Fc-region. Cysteine engineered circular fusion polypeptide may be generated alike antibodies as described, e.g., in US 7,521,541.

### e) Circular Fusion Polypeptide Derivatives

In certain embodiments, a circular fusion polypeptide provided herein may be further modified to contain additional non-proteinaceous moieties that are known in the art and readily available. The moieties suitable for derivatization of the circular fusion polypeptide include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the circular fusion polypeptide may vary, and if more than one polymer is attached, they can be the same or different molecules. In general, the number and/or type of polymers used for derivatization can be determined based on considerations including, but not limited to, the particular properties or functions of the circular fusion polypeptide to be improved, whether the circular fusion polypeptide derivative will be used in a therapy under defined conditions, etc.

In another embodiment, conjugates of a circular fusion polypeptide and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed.

### f) Blood-brain-barrier shuttle conjugates

In certain embodiments, a circular fusion polypeptide provided herein may be further modified to contain one or more blood-brain-barrier shuttle modules that are known in the art and readily available.

The blood-brain-barrier shuttle module is characterized by having a binding specificity for a blood-brain-barrier receptor. This binding specificity can be obtained either by fusing a blood-brain-barrier shuttle module to the circular fusion polypeptide as reported herein or it can be obtained by introducing the binding specificity to the blood-brain-barrier receptor as one of the binding specificities of a multispecific (mono- or multi)circular fusion polypeptide and, thus, comprises the binding specificity for a therapeutic target and the binding specificity to the blood-brain-barrier receptor.

One or more blood-brain-barrier shuttle modules can be fused to any terminus of the light or heavy chain of the circular fusion polypeptide as reported herein. In one preferred embodiment the blood-brain-barrier shuttle module is fused to the C-terminus of the circular fusion polypeptide. In one preferred embodiment the blood-brain-barrier shuttle module is fused to the N-terminus of the circular fusion polypeptide.

The one or more blood-brain-barrier shuttle modules can be fused to the respective circular fusion polypeptide either directly or via peptidic linker. In one preferred embodiment the peptidic linker has the amino acid sequence GGGGSGGGGS (SEQ ID NO: 64), or GGGGSGGGGSGGGGS (SEQ ID NO: 65) or (G4S)₆ (SEQ ID NO: 70).

The blood-brain-barrier shuttle module can be an antibody scFv fragment or a Fab. In one embodiment the blood-brain-barrier shuttle module is a scFv comprising in N- to C-terminal order a light chain variable domain-a light chain constant domain-a peptidic linker-a heavy chain variable domain-the heavy chain constant domain 1.

In one embodiment the blood-brain-barrier shuttle module is scFv fragment or a Fab of a humanized variant of the anti-transferrin receptor-antibody 8D3 with a (G4S)₆ peptidic linker (SEQ ID NO: 70).

The term humanized variant thereof denotes a molecule that has been obtained by grafting the HVRs of the murine 8D3 antibody on a human framework with the optional introduction of one to three mutations independently of each other in each of the framework regions (FRs) and/or the hypervariable regions (HVRs).

The anti-transferrin receptor antibody 8D3 (see e.g. Boado, R.J., et al., Biotechnol. Bioeng. 102 (2009) 1251-1258) is a murine antibody wherein the heavy chain variable domain has the amino acid sequence of SEQ ID NO: 71 and wherein the light chain variable domain has the amino acid sequence of SEQ ID NO: 72 (variant 1) or of SEQ ID NO: 73 (variant 2).

In one embodiment the blood-brain-barrier shuttle module is scFv fragment or a Fab of a humanized variant of the anti-transferrin receptor-antibody 299 (see WO2017/055541).

The term humanized variant thereof denotes a molecule that has been obtained by grafting the HVRs of the 299 antibody on a human framework with the optional introduction of one to three mutations independently of each other in each of the framework regions (FRs) and/or the hypervariable regions (HVRs).

The anti-transferrin receptor antibody 299 is a rabbit antibody wherein the heavy chain variable domain has the amino acid sequence of SEQ ID NO: 74 and the light chain variable domain has the amino acid sequence of SEQ ID NO: 75.

In one embodiment the blood-brain-barrier shuttle module comprises a transferrin receptor binding specificity comprises (a) a HVR-H1 comprising the amino acid sequence of SEQ ID NO: 76; (b) a HVR-H2 comprising the amino acid sequence of SEQ ID NO: 77; (c) a HVR-H3 comprising the amino acid sequence of SEQ ID NO: 78, 79 or 80; (d) a HVR-L1 comprising the amino acid sequence of SEQ ID NO: 81; (e) a HVR-L2 comprising the amino acid sequence of SEQ ID NO: 82; and (f) a HVR-L3 comprising the amino acid sequence of SEQ ID NO: 83.

In one embodiment the blood-brain-barrier shuttle module comprises at least one pair of the heavy chain variable domain of SEQ ID NO: 84 and the light chain variable domain of SEQ ID NO: 85 forming a binding site for the transferrin receptor.

In one embodiment the blood-brain-barrier shuttle module comprises at least one pair of the heavy chain variable domain of SEQ ID NO: 86 and the light chain variable domain of SEQ ID NO: 87 forming a binding site for the transferrin receptor.

In one embodiment the blood-brain-barrier shuttle module comprises at least one pair of the heavy chain variable domain of SEQ ID NO: 88 and the light chain variable domain of SEQ ID NO: 87 forming a binding site for the transferrin receptor.

In one embodiment the blood-brain-barrier shuttle module is scFv fragment or a Fab of a humanized variant of the anti-transferrin receptor-antibody 494 (see EP 15187820).

The term humanized variant thereof denotes a molecule that has been obtained by grafting the HVRs of the 494 antibody on a human framework with the optional introduction of one to three mutations independently of each other in each of the framework regions (FRs) and/or the hypervariable regions (HVRs).

The anti-transferrin receptor antibody 494 is a murine antibody wherein the heavy chain variable domain has the amino acid sequence of SEQ ID NO: 89 and the light chain variable domain has the amino acid sequence of SEQ ID NO: 90.

In one embodiment the blood-brain-barrier shuttle module is scFv fragment or a Fab of an anti-transferrin receptor antibody that specifically bind to human transferrin receptor (huTfR) and cynomolgus transferrin receptor (cyTfR). In certain embodiments, the anti-transferrin receptor antibody
- binds to human transferrin receptor (huTfR) and cynomolgus transferrin receptor (cyTfR), and/or
- has an off-rate for the human transferrin receptor that is equal to or less than (i.e. at most) that of the anti-transferrin receptor antibody 128.1 for the cynomolgus transferrin receptor, whereby the off-rates are determined by surface plasmon resonance, and whereby the anti-transferrin receptor antibody 128.1 has a heavy chain variable domain of SEQ ID NO: 91 and a light chain variable domain of SEQ ID NO: 92, and/or
- binds with an off-rate for the human transferrin receptor that is between and including 0.1 1/s and 0.005 1/s.

One aspect as reported herein is an anti-transferrin receptor antibody that specifically binds to human transferrin receptor and cynomolgus transferrin receptor, which comprises
i) a humanized heavy chain variable domain derived from the heavy chain variable domain of SEQ ID NO: 74, and
ii) a humanized light chain variable domain derived from the light chain variable domain of SEQ ID NO: 75,
wherein the antibody has an off-rate for the human transferrin receptor that is equal to or less than (i.e. at most) the off-rate of the anti-transferrin receptor antibody 128.1 for the cynomolgus transferrin receptor,
whereby the off-rates are determined by surface plasmon resonance, and
whereby the anti-transferrin receptor antibody 128.1 has a heavy chain variable domain of SEQ ID NO: 91 and a light chain variable domain of SEQ ID NO: 92.

In one embodiment the antibody has in the light chain variable domain at position 80 a proline amino acid residue (P) (numbering according to Kabat).

In one embodiment the antibody has in the light chain variable domain at position 91 an asparagine amino acid residue (N) (numbering according to Kabat).

In one embodiment the antibody has in the light chain variable domain at position 93 an alanine amino acid residue (A) (numbering according to Kabat).

In one embodiment the antibody has in the heavy chain variable domain at position 100g a serine amino acid residue (S) (numbering according to Kabat).

In one embodiment the antibody has in the heavy chain variable domain at position 100g a glutamine amino acid residue (Q) (numbering according to Kabat).

In one embodiment the antibody has in the heavy chain variable domain at position 65 a serine amino acid residue (S) (numbering according to Kabat).

In one embodiment the antibody has in the heavy chain variable domain at position 105 a glutamine amino acid residue (Q) (numbering according to Kabat).

In one embodiment the antibody the antibody has in the light chain variable domain at position 80 a proline amino acid residue (P), in the light chain variable domain at position 91 an asparagine amino acid residue (N), in the light chain variable domain at position 93 an alanine amino acid residue (A), in the heavy chain variable domain at position 100g a serine amino acid residue (S), in the heavy chain variable domain at position 65 a serine amino acid residue (S), and in the heavy chain variable domain at position 105 a glutamine amino acid residue (Q) (numbering according to Kabat).

In one embodiment the antibody the antibody has in the light chain variable domain at position 80 a proline amino acid residue (P), in the light chain variable domain at position 91 an asparagine amino acid residue (N), in the light chain variable domain at position 93 an alanine amino acid residue (A), in the heavy chain variable domain at position 100g a glutamine amino acid residue (Q), in the heavy chain variable domain at position 65 a serine amino acid residue (S), and in the heavy chain variable domain at position 105 a glutamine amino acid residue (Q) (numbering according to Kabat).

One aspect as reported herein is an anti-transferrin receptor antibody that specifically bind to human transferrin receptor (huTfR) comprising
i) a heavy chain variable domain (VH) sequence having at least 90% sequence identity to the amino acid sequence of SEQ ID NO: 88, and
ii) a light chain variable domain (VL) having at least 90% sequence identity to the amino acid sequence of SEQ ID NO: 87,
wherein the antibody has about the same off-rate as an antibody comprising a heavy chain variable domain (VH) sequence of SEQ ID NO: 88 and a light chain variable domain (VL) sequence of SEQ ID NO: 87.

In one aspect, herein is provided a circular fusion polypeptide as reported herein conjugated via a peptidic linker to a monovalent binding entity which binds to a blood-brain-barrier receptor.

In one aspect, herein is provided a dicircular fusion polypeptide as reported herein wherein one circular fusion polypeptide is conjugated via a peptidic linker to a monovalent binding entity which binds to a blood-brain-barrier receptor.

In one aspect, herein is provided a dicircular fusion polypeptide as reported herein wherein both circular fusion polypeptide are each individually conjugated via a peptidic linker to a monovalent binding entity which binds to a blood-brain-barrier receptor. This conjugate comprises two monovalent binding entities binding to a blood-brain-barrier receptor.

In one embodiment, the monovalent binding entity which binds to the blood-brain-barrier receptor is selected from the group consisting of proteins, polypeptides and peptides.

In one embodiment, the monovalent binding entity which binds to the blood-brain-barrier receptor comprises a molecule selected from the group consisting of a blood-brain-barrier receptor ligand, a scFv, an Fv, a scFab, a VHH, in one preferred embodiment a scFv or a scFab.

In one embodiment, the blood-brain-barrier receptor is selected from the group consisting of transferrin receptor, insulin receptor, insulin-like growth factor receptor, low density lipoprotein receptor-related protein 8, low density lipoprotein receptor-related protein 1 and heparin-binding epidermal growth factor-like growth factor. In one preferred embodiment the blood-brain-barrier receptor is the transferrin receptor.

In one embodiment, the monovalent binding entity which binds to the blood-brain-barrier receptor comprises one scFab or one scFv directed to the transferrin receptor, more particular a scFab or scFv recognizing an epitope in the transferrin receptor comprised within the amino acid sequence of SEQ ID NO: 93, 94 or 95.

In one embodiment, the monovalent binding entity which binds to the blood-brain-barrier receptor is coupled to the C-terminal end of the circular fusion polypeptide by the linker.

In one embodiment, the peptidic linker is an amino acid sequence with a length of at least 15 amino acids, more preferably with a length of 18 to 25 amino acids.

In one preferred embodiment, the conjugate comprises a circular fusion polypeptide as reported herein (specifically binding to a brain target) as brain effector entity, a linker of the sequence GGSGGGGSGGGGSGGGGS (SEQ ID NO: 68) and one scFab as monovalent binding entity which binds to the human transferrin receptor as blood brain receptor, wherein the scFab is coupled by the linker to the C-terminal end of the circular fusion polypeptide, and wherein the scFab recognizes an epitope in the human transferrin receptor comprised within the amino acid sequence of SEQ ID NO: 93, 94 or 95.

In one preferred embodiment, the conjugate comprises a dicircular fusion polypeptide as reported herein (specifically binding to a brain target) as brain effector entity, two linker of the sequence GGSGGGGSGGGGSGGGGS (SEQ ID NO: 68) and two scFab as monovalent binding entities which bind to the human transferrin receptor as blood brain receptor, wherein each of the scFabs is coupled by one linker to the C-terminal end of a different circular fusion polypeptide, and wherein the scFab recognizes an epitope in the human transferrin receptor comprised within the amino acid sequence of SEQ ID NO: 93, 94 or 95.

In one embodiment, the first circular fusion polypeptide comprises a first dimerization module and the second circular fusion polypeptide comprises a second dimerization module allowing homo- or/and heterodimerization of the two circular fusion polypeptides.

In one embodiment, the heterodimerization module of the first circular fusion polypeptide is a knob heavy chain Fc-region and the heterodimerization module of the second circular fusion polypeptide is a hole heavy chain Fc-region (according to the knobs-into-holes strategy; see e.g. WO 96/027011; Ridgway, J.B., et al., Prot. Eng. 9 (1996) 617-621; Merchant, A.M., et al., Nat. Biotechnol. 16 (1998) 677-681). The introduction of a disulfide bridge further stabilizes the heterodimers (Merchant, A.M., et al., Nat. Biotech. 16 (1998) 677-681; Atwell, S., et al., J. Mol. Biol. 270 (1997) 26-35) and increases the yield.

In one embodiment, the homodimerization module of the first circular fusion polypeptide is a heavy chain Fc-region and the homodimerization module of the second circular fusion polypeptide is a heavy chain Fc-region, wherein both Fc-regions are modified by the amino acid substitutions S364G, L368F, D399K and K409D (wherein the amino acid positions are numbered according to the EU Index of Kabat). The modification/mutation maintains attractive interactions between identical chains but lead to repulsion of different chains.

In one embodiment, the heterodimerization module of the first circular fusion polypeptide is a knob heavy chain Fc-region and the heterodimerization module of the second circular fusion polypeptide is a hole heavy chain Fc-region (according to the knobs-into-holes strategy), wherein one Fc-region is modified by the amino acid substitutions S364G, L368F, D399K and K409D (wherein the amino acid positions are numbered according to the EU Index of Kabat). The modification/mutation reduces attractive interactions between hole chains and promotes heterodimerization.

The "EU numbering system" or "EU index" is generally used when referring to a residue in an immunoglobulin heavy chain constant region (e.g., the EU index reported in Kabat et al., Sequences of Proteins of Immunological Interest, 5th ed., Public Health Service, National Institutes of Health, Bethesda, MD (1991) expressly incorporated herein by reference).

The circular fusion polypeptide conjugate as reported herein can be used to transport the circular fusion polypeptide across the blood brain barrier.

In one embodiment, the circular fusion polypeptide that is coupled at its C-terminal end to the scFab as monovalent binding entity which binds to the human transferrin receptor has the following structure in N- to C-terminal direction:
- circular fusion polypeptide,
- peptidic linker coupling the C-terminal end of the circular fusion polypeptide to the N-terminal end of the VL domain of the scFab, in one preferred embodiment the peptidic linker has the amino acid sequence GGSGGGGSGGGGSGGGGS (SEQ ID NO: 68),
- variable light chain domain (VL) and C-kappa light chain domain of the scFab,
- peptidic linker coupling the C-terminal end of the C-kappa light chain domain of the scFab to the N-terminal end of the VH domain of the scFab, in one preferred embodiment the peptidic linker has the amino acid sequence (G₄S)₄GG (SEQ ID NO: 69),
- variable heavy chain domain (VH) of the scFab antibody and IgG CH1 heavy chain domain.

In one embodiment, the circular fusion polypeptide that is coupled at its C-terminal end to the scFv as monovalent binding entity which binds to the human transferrin receptor has the following structure in N- to C-terminal direction:
- circular fusion polypeptide,
- peptidic linker coupling the C-terminal end of the circular fusion polypeptide to the N-terminal end of the VL domain of the scFv antibody fragment, in one preferred embodiment the peptidic linker is a peptide with the amino acid sequence GGSGGGGSGGGGSGGGGS (SEQ ID NO: 68),
- variable light chain domain (VL),
- peptidic linker coupling the C-terminal end of the variable light chain domain to the N-terminal end of the VH domain of the scFv, in one preferred embodiment the peptidic linker is a peptide with the amino acid sequence (G₄S)₄GG (SEQ ID NO: 69),
- variable heavy chain domain (VH) of the scFv antibody fragment.

### One blood-brain-barrier shuttle module

In one aspect the circular fusion polypeptide or the multicircular fusion polypeptide comprises exactly one blood-brain-barrier binding specificity or shuttle module, thus is at least bispecific, wherein the blood-brain-barrier binding specificity or shuttle module comprises
- humanized variants of the variable domains of the anti-human transferrin receptor antibody 8D3 of SEQ ID NO: 71 and 72 or 73, or
- the pair of the heavy chain variable domain of SEQ ID NO: 88 and the light chain variable domain of SEQ ID NO: 87, or
- humanized variants of the variable domains of the anti-human transferrin receptor antibody 494 of SEQ ID NO: 89 and 90,
whereby the blood-brain-barrier binding specificity or shuttle module transports the (multi)circular fusion polypeptide across the blood-brain-barrier

### One or two blood-brain-barrier shuttle modules

In one aspect the circular fusion polypeptide or the multicircular fusion polypeptide comprises one or two blood-brain-barrier binding specificities or shuttle module(s), thus is at least bispecific, wherein the blood-brain-barrier shuttle binding site or module is/are derived from an antibody which binds with low affinity to a blood-brain-barrier receptor (BBB-R, BBB-R binding specificity), whereby the blood-brain-barrier binding specificity or shuttle module derived from an antibody which binds with low affinity to a blood-brain-barrier receptor transports the (multi)circular fusion polypeptide across the blood-brain-barrier.

In one embodiment, the BBB-R is selected from the group consisting of transferrin receptor (TfR), insulin receptor, insulin-like growth factor receptor (IGF receptor), low density lipoprotein receptor-related protein 8 (LRP8), low density lipoprotein receptor-related protein 1 (LRP1), and heparin-binding epidermal growth factor-like growth factor (HB-EGF). In another such aspect, the BBB-R is a human BBB-R. In one such aspect, the BBB-R is TfR. In another such aspect, the BBB-R is TfR and the antibody does not inhibit TfR activity. In another such aspect, the BBB-R is TfR and the antibody does not inhibit the binding of TfR to transferrin.

In one embodiment, the antibody does not impair the binding of the BBB-R to one or more of its native ligands. In one such embodiment, the antibody specifically binds to human transferrin receptor (hTfR) in such a manner that it does not inhibit binding of the hTfR to human transferrin.

In one embodiment, the BBB-R binding specificity has an IC₅₀ for the BBB-R from about 1 nM to about 100 µM. In one embodiment, the IC₅₀ is from about 5 nM to about 100 µM. In one embodiment, the IC₅₀ is from about 50 nM to about 100 µM. In one embodiment, the IC₅₀ is from about 100 nM to about 100 µM. In one embodiment, the BBB-R binding specificity has an affinity for the BBB-R from about 5 nM to about 10 µM. In one embodiment, the BBB-R binding specificity, when conjugated to or comprised in the circular fusion polypeptide, has an affinity for the BBB-R from about 30 nM to about 1 µM. In one embodiment, the BBB-R binding specificity, when conjugated to or comprised in the circular fusion polypeptide, has an affinity for the BBB-R from about 50 nM to about 1 µM. In one embodiment, the affinity of the BBB-R binding specificity or the circular fusion polypeptide conjugate for the BBB-R is measured using scatchard analysis. In one embodiment, the affinity of the BBB-R binding specificity or of the circular fusion polypeptide conjugate for the BBB-R is measured using BIACORE analysis. In one embodiment, the affinity of the BBB-R binding specificity or of the circular fusion polypeptide conjugate for the BBB-R is measured using a competition ELISA.

### Use of the blood-brain-barrier shuttle containing conjugates

In another embodiment, herein is provided a method of increasing exposure of the CNS to a circular fusion polypeptide, wherein the circular fusion polypeptide is coupled to an antibody or antibody fragment which binds with low affinity to a BBB-R, thereby increasing the exposure of the CNS to the circular fusion polypeptide.

The term "coupled" includes cases wherein the anti-BBB-R antibody binding specificity is introduced as second binding specificity in an at least bispecific circular fusion polypeptide. The term "coupled" also includes cases wherein the anti-BBB-R antibody binding specificity is conjugated as independent binding specificity in a circular fusion polypeptide.

In one embodiment, the increase in CNS exposure to the circular fusion polypeptide is measured relative to the CNS exposure of a circular fusion polypeptide coupled with a typical antibody not having lowered affinity for the BBB-R. In one embodiment, the increase in CNS exposure to the circular fusion polypeptide is measured as a ratio of the amount of the circular fusion polypeptide found in the CNS relative to the amount found in the serum after administration. In one embodiment, the increase in CNS exposure results in a ratio of greater than 0.1 %. In one embodiment, the increase in CNS exposure to the circular fusion polypeptide is measured relative to the CNS exposure of the circular fusion polypeptide in the absence of a coupled anti-BBB-R antibody. In one embodiment, the increase in CNS exposure to the circular fusion polypeptide is measured by imaging. In one embodiment, the increase in CNS exposure to the circular fusion polypeptide is measured by an indirect readout such as a modification of one or more physiological symptoms.

A method of increasing retention in the CNS of a circular fusion polypeptide administered to a subject, wherein the circular fusion polypeptide is coupled to an antibody or antibody fragment, which binds with low affinity to a BBB-R, such that the retention in the CNS of the circular fusion polypeptide is increased.

In another embodiment, herein is provided a method of optimizing the pharmacokinetics and/or pharmacodynamics of a circular fusion polypeptide to be efficacious in the CNS of a subject, wherein the circular fusion polypeptide is coupled to an antibody or antibody fragment, which binds with low affinity to a BBB-R, whereby the antibody or antibody fragment is selected such that its affinity for the BBB-R after coupling to the circular fusion polypeptide results in an amount of transport of the antibody or antibody fragment conjugated to the circular fusion polypeptide across the BBB that optimizes the pharmacokinetics and/or pharmacodynamics of the circular fusion polypeptide in the CNS.

In another embodiment herein is provided a method of treating a neurological disorder in a mammal comprising treating the mammal with an antibody or antibody fragment, which binds a BBB-R and which is coupled to a circular fusion polypeptide, wherein the antibody has been selected to have a low affinity for the BBB-R and thereby improves CNS uptake of the antibody and coupled circular fusion polypeptide. In one embodiment, the treating results in lessening or elimination of disorder symptoms. In another aspect, the treating results in amelioration of the neurological disorder.

In one embodiment of all previous aspects, the anti-BBB-R antibody has an IC₅₀ for the BBB-R from about 1 nM to about 100 µM. In another such embodiment, the IC₅₀ is from about 5 nM to about 100 µM. In another such embodiment, the IC₅₀ is from about 50 nM to about 100 µM. In another such embodiment, the IC₅₀ is from about 100 nM to about 100 µM. In another embodiment, the antibody has an affinity for the BBB-R from about 5 nM to about 10 µM. In another embodiment, the antibody, when coupled to the circular fusion polypeptide, has an affinity for the BBB-R from about 30 nM to about 1 µM. In another embodiment, the antibody, when coupled to the circular fusion polypeptide, has an affinity for the BBB-R from about 50 nM to about 1 µM. In one embodiment, the affinity of the anti-BBB-R antibody or the circular fusion polypeptide conjugate for the BBB-R is measured using scatchard analysis. In another embodiment, the affinity of the anti-BBB-R antibody or the circular fusion polypeptide conjugate for the BBB-R is measured using BIACORE analysis. In another embodiment, the affinity of the anti-BBB-R antibody or the circular fusion polypeptide conjugate for the BBB-R is measured using a competition ELISA.

In another embodiment, the circular fusion polypeptide conjugate is labeled. In another embodiment, the anti-BBB-R antibody or fragment does not impair the binding of the BBB-R to one or more of its native ligands. In another embodiment, the anti-BBB-R antibody specifically binds to hTfR in such a manner that it does not inhibit binding of the hTfR to human transferrin. In another embodiment, the circular fusion polypeptide conjugate is administered to a mammal. In another embodiment, the mammal is a human. In another embodiment, the mammal has a neurological disorder. In another embodiment, the neurological disorder is selected from the group consisting of Alzheimer's disease (AD), stroke, dementia, muscular dystrophy (MD), multiple sclerosis (MS), amyotrophic lateral sclerosis (ALS), cystic fibrosis, Angelman's syndrome, Liddle syndrome, Parkinson's disease, Pick's disease, Paget's disease, cancer, and traumatic brain injury.

### Non-covalent complexes as blood-brain barrier shuttles

One part of the non-covalent complex is a blood brain barrier-shuttle module (BBB-shuttle module) that is a bispecific antibody with a first binding specificity for a hapten and a second binding specificity for a blood-brain-barrier receptor (BBBR). Such a BBB-shuttle module recognizes a transcytoseable cell surface target on the blood brain barrier (such as TfR, LRPs or other targets, BBB-R) and simultaneously binds to a haptenylated circular fusion polypeptide.

In more detail, the circular fusion polypeptide is conjugated to a hapten and complexed by the hapten-binding site of the blood brain barrier shuttle. This complex is defined and stable and specifically delivers the haptenylated circular fusion polypeptide over the blood brain barrier. Since the haptenylated circular fusion polypeptide is complexed in a non-covalent manner by the blood-brain-barrier shuttle, the haptenylated circular fusion polypeptide is on the one hand bound to its delivery vehicle (= blood-brain-barrier shuttle = bispecific antibody) during its time in the circulation but can also on the other hand be efficiently released after transcytosis. The conjugation with the hapten can be effected without interfering with the activity of the circular fusion polypeptide. The blood-brain-barrier shuttle does not contain an unusual covalent addition and therefore obviates any risk of immunogenicity. Complexes of haptenylated circular fusion polypeptide with the bispecific antibody containing the hapten-specific binding sites confer benign biophysical behavior to the circular fusion polypeptide. Furthermore, such complexes are capable to target the load to cells or tissues which display the antigen that is recognized by the bispecific antibody's second binding specificity.

The circular fusion polypeptide retains its functionality despite being haptenylated, as well as while being complexed by the blood-brain-barrier shuttle (= bispecific antibody). In addition, the blood-brain-barrier receptor binding site of the bispecific antibody retains its binding specificity and affinity in the presence of complexed haptenylated circular fusion polypeptide. The complexes of haptenylated circular fusion polypeptide with the bispecific antibody as reported herein can be used to target the circular fusion polypeptide specifically to cells that express the blood-brain-barrier receptor. Since the haptenylated circular fusion polypeptide is coupled in a non-covalent manner to the bispecific antibody the circular fusion polypeptide can be released after internalization or transcytosis.

### VI. Recombinant Methods and Compositions

Circular fusion polypeptide like antibodies may be produced using recombinant methods and compositions, e.g., as described in US 4,816,567. In one embodiment, isolated nucleic acid encoding a circular fusion polypeptide described herein is provided. Such nucleic acid may encode an amino acid sequence comprising one circular fusion polypeptide and optionally also an amino acid sequence comprising a second circular fusion polypeptide (e.g., a first circular fusion polypeptide and a second circular fusion polypeptide of a dicircular fusion polypeptide). In a further embodiment, one or more vectors (e.g., expression vectors) comprising such nucleic acid are provided. In a further embodiment, a host cell comprising such nucleic acid is provided. In one such embodiment, a host cell comprises (e.g., has been transformed with): (1) a vector comprising a nucleic acid that encodes an amino acid sequence comprising a circular fusion polypeptide (homomeric or homomultimeric circular fusion polypeptide) and optionally a second amino acid sequence comprising the second circular fusion polypeptide in case of a heterodimeric dicircular fusion polypeptide and optionally further nucleic acids that encode amino acid sequences of further circular fusion polypeptides in case of a multicircular fusion polypeptide, or (2) in case of a heterodimeric dicircular fusion polypeptide a first vector comprising a nucleic acid that encodes an amino acid sequence comprising the first circular fusion polypeptide and a second vector comprising a nucleic acid that encodes an amino acid sequence comprising the second circular fusion polypeptide. In one embodiment, the host cell is eukaryotic, e.g. a Chinese Hamster Ovary (CHO) cell or lymphoid cell (e.g., Y0, NS0, Sp20 cell). In one embodiment, a method of making a circular fusion polypeptide is provided, wherein the method comprises culturing a host cell comprising a nucleic acid encoding the circular fusion polypeptide, as provided above, under conditions suitable for expression of the circular fusion polypeptide, and optionally recovering the circular fusion polypeptide from the host cell (or host cell culture medium).

For recombinant production of a circular fusion polypeptide, nucleic acid encoding an antibody, e.g., as described above, is isolated and inserted into one or more vectors for further cloning and/or expression in a host cell. Such nucleic acid may be readily produced using conventional procedures.

Suitable host cells for cloning or expression of circular fusion polypeptide-encoding vectors include prokaryotic or eukaryotic cells described herein. For example, circular fusion polypeptides may be produced in bacteria, in particular when glycosylation and Fc effector function are not needed. For expression of antibody fragments and polypeptides in bacteria, see, e.g., US 5,648,237, US 5,789,199, and US 5,840,523 (see also Charlton, K.A., In: Methods in Molecular Biology, Vol. 248, Lo, B.K.C. (ed.), Humana Press, Totowa, NJ (2003), pp. 245-254, describing expression of antibody fragments in *E. coli.*)*.* After expression, the circular fusion polypeptide may be isolated from the bacterial cell paste in a soluble fraction and can be further purified.

In addition to prokaryotes, eukaryotic microbes such as filamentous fungi or yeast are suitable cloning or expression hosts for circular fusion polypeptide-encoding vectors, including fungi and yeast strains whose glycosylation pathways have been "humanized", resulting in the production of a circular fusion polypeptide with a partially or fully human glycosylation pattern (see Gerngross, T.U., Nat. Biotech. 22 (2004) 1409-1414; Li, H. et al., Nat. Biotech. 24 (2006) 210-215).

Suitable host cells for the expression of glycosylated circular fusion polypeptides are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts (see, e.g., US 5,959,177, US 6,040,498, US 6,420,548, US 7,125,978, and US 6,417,429 (describing PLANTIBODIES™ technology for producing antibodies in transgenic plants)).

Vertebrate cells may also be used as hosts. For example, mammalian cell lines that are adapted to grow in suspension may be useful. Other examples of useful mammalian host cell lines are monkey kidney CV1 line transformed by SV40 (COS-7); human embryonic kidney line (293 or 293 cells as described, e.g., in Graham, F.L. et al., J. Gen Virol. 36 (1977) 59-74); baby hamster kidney cells (BHK); mouse sertoli cells (TM4 cells as described, e.g., in Mather, J.P., Biol. Reprod. 23 (1980) 243-252); monkey kidney cells (CV1); African green monkey kidney cells (VERO-76); human cervical carcinoma cells (HELA); canine kidney cells (MDCK; buffalo rat liver cells (BRL 3A); human lung cells (W138); human liver cells (Hep G2); mouse mammary tumor (MMT 060562); TRI cells, as described, e.g., in Mather, J.P. et al., Annals N.Y. Acad. Sci. 383 (1982) 44-68; MRC 5 cells; and FS4 cells. Other useful mammalian host cell lines include Chinese hamster ovary (CHO) cells, including DHFR" CHO cells (Urlaub, G. et al., Proc. Natl. Acad. Sci. USA 77 (1980) 4216-4220); and myeloma cell lines such as Y0, NS0 and Sp2/0. For a review of certain mammalian host cell lines suitable for antibody production, see, e.g., Yazaki, P. and Wu, A.M., Methods in Molecular Biology, Vol. 248, Lo, B.K.C. (ed.), Humana Press, Totowa, NJ (2004), pp. 255-268.

### VII. Assays

Circular fusion polypeptides provided herein may be identified, screened for, or characterized for their physical/chemical properties and/or biological activities by various assays known in the art for determining binding of a polypeptide to its target.

### VIII. Immunoconjugates

The invention also provides immunoconjugates comprising a circular fusion polypeptide as reported herein conjugated to one or more cytotoxic agents, such as chemotherapeutic agents or drugs, growth inhibitory agents, toxins (e.g., protein toxins, enzymatically active toxins of bacterial, fungal, plant, or animal origin, or fragments thereof), or radioactive isotopes.

In one embodiment, an immunoconjugate is an circular fusion polypeptide-drug conjugate in which a circular fusion polypeptide is conjugated to one or more drugs, including but not limited to a maytansinoid (see US 5,208,020, US 5,416,064 and EP 0 425 235 B1); an auristatin such as monomethyl auristatin drug moieties DE and DF (MMAE and MMAF) (see US 5,635,483, US 5,780,588, and US 7,498,298); a dolastatin; a calicheamicin or derivative thereof (see US 5,712,374, US 5,714,586, US 5,739,116, US 5,767,285, US 5,770,701, US 5,770,710, US 5,773,001, and US 5,877,296; Hinman, L.M. et al., Cancer Res. 53 (1993) 3336-3342; and Lode, H.N. et al., Cancer Res. 58 (1998) 2925-2928); an anthracycline such as daunomycin or doxorubicin (see Kratz, F. et al., Curr. Med. Chem. 13 (2006) 477-523; Jeffrey, S.C. et al., Bioorg. Med. Chem. Lett. 16 (2006) 358-362; Torgov, M.Y. et al., Bioconjug. Chem. 16 (2005) 717-721; Nagy, A. et al., Proc. Natl. Acad. Sci. USA 97 (2000) 829-834; Dubowchik, G.M. et al., Bioorg. & Med. Chem. Letters 12 (2002) 1529-1532; King, H.D. et al., J. Med. Chem. 45 (20029 4336-4343; and US 6,630,579); methotrexate; vindesine; a taxane such as docetaxel, paclitaxel, larotaxel, tesetaxel, and ortataxel; a trichothecene; and CC1065.

In another embodiment, an immunoconjugate comprises a circular fusion polypeptide as reported herein conjugated to an enzymatically active toxin or fragment thereof, including but not limited to diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes.

In another embodiment, an immunoconjugate comprises a circular fusion polypeptide as reported herein conjugated to a radioactive atom to form a radioconjugate. A variety of radioactive isotopes are available for the production of radioconjugates. Examples include At²¹¹, I¹³¹, I¹²⁵, Y⁹⁰, Re¹⁸⁶, Re¹⁸⁸, Sm¹⁵³, Bi²¹², P³², Pb²¹² and radioactive isotopes of Lu. When the radioconjugate is used for detection, it may comprise a radioactive atom for scintigraphic studies, for example TC^{99m} or I¹²³, or a spin label for nuclear magnetic resonance (NMR) imaging (also known as magnetic resonance imaging, MRI), such as iodine-123 again, iodine-131, indium-111, fluorine-19, carbon-13, nitrogen-15, oxygen-17, gadolinium, manganese or iron. Conjugates of a circular fusion polypeptide and cytotoxic agent may be made using a variety of bifunctional protein coupling agents such as N-succinimidyl-3-(2-pyridyldithio) propionate (SPDP), succinimidyl-4-(N-maleimidomethyl) cyclohexane-1-carboxylate (SMCC), iminothiolane (IT), bifunctional derivatives of imidoesters (such as dimethyl adipimidate HC1), active esters (such as disuccinimidyl suberate), aldehydes (such as glutaraldehyde), bis-azido compounds (such as bis (p-azidobenzoyl) hexanediamine), bis-diazonium derivatives (such as bis-(p-diazoniumbenzoyl)-ethylenediamine), diisocyanates (such as toluene 2,6-diisocyanate), and bis-active fluorine compounds (such as 1,5-difluoro-2,4-dinitrobenzene). For example, a ricin immunotoxin can be prepared as described in Vitetta, E.S. et al., Science 238 (1987) 1098-1104. Carbon-14-labeled 1-isothiocyanatobenzyl-3-methyldiethylene triamine pentaacetic acid (MX-DTPA) is an exemplary chelating agent for conjugation of radionucleotide to the circular fusion polypeptide (see WO 94/11026). The linker may be a "cleavable linker" facilitating release of a cytotoxic drug in the cell. For example, an acid-labile linker, peptidase-sensitive linker, photolabile linker, dimethyl linker or disulfide-containing linker (Chari, R.V. et al., Cancer Res. 52 (1992) 127-131; US 5,208,020) may be used.

The immunoconjugates herein expressly contemplate, but are not limited to such conjugates prepared with cross-linker reagents including, but not limited to, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, and sulfo-SMPB, and SVSB (succinimidyl-(4-vinylsulfone)benzoate) which are commercially available (e.g., from Pierce Biotechnology, Inc., Rockford, IL., U.S.A).

### IX. Methods and Compositions for Diagnostics and Detection

In certain embodiments, any of the circular fusion polypeptides provided herein is useful for detecting the presence of its target in a biological sample. The term "detecting" as used herein encompasses quantitative or qualitative detection. In certain embodiments, a biological sample comprises a blood, serum, plasma, cell or tissue.

In one embodiment, a circular fusion polypeptide for use in a method of diagnosis or detection is provided. In a further aspect, a method of detecting the presence of the target of the circular fusion polypeptide in a biological sample is provided. In certain embodiments, the method comprises contacting the biological sample with the circular fusion polypeptide as described herein under conditions permissive for binding of the circular fusion polypeptide to its target, and detecting whether a complex is formed between the circular fusion polypeptide and its target. Such method may be an *in vitro* or *in vivo* method. In one embodiment, a circular fusion polypeptide is used to select subjects eligible for therapy with said circular fusion polypeptide.

In certain embodiments, labeled circular fusion polypeptides are provided. Labels include, but are not limited to, labels or moieties that are detected directly (such as fluorescent, chromophoric, electron-dense, chemiluminescent, and radioactive labels), as well as moieties, such as enzymes or ligands, that are detected indirectly, e.g., through an enzymatic reaction or molecular interaction. Exemplary labels include, but are not limited to, the radioisotopes ³²P, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (US 4,737,456), luciferin, 2,3-dihydrophthalazinediones, horseradish peroxidase (HRP), alkaline phosphatase, β-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, coupled with an enzyme that employs hydrogen peroxide to oxidize a dye precursor such as HRP, lactoperoxidase, or microperoxidase, biotin/avidin, spin labels, bacteriophage labels, stable free radicals, and the like.

### X. Pharmaceutical Formulations

Pharmaceutical formulations of a circular fusion polypeptide as described herein are prepared by mixing such circular fusion polypeptide having the desired degree of purity with one or more optional pharmaceutically acceptable carriers (Remington's Pharmaceutical Sciences, 16th edition, Osol, A. (ed.) (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyl dimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as poly(vinylpyrrolidone); amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g. Zn-protein complexes); and/or non-ionic surfactants such as polyethylene glycol (PEG). Exemplary pharmaceutically acceptable carriers herein further include interstitial drug dispersion agents such as soluble neutral-active hyaluronidase glycoproteins (sHASEGP), for example, human soluble PH-20 hyaluronidase glycoproteins, such as rhuPH20 (HYLENEX®, Baxter International, Inc.). Certain exemplary sHASEGPs and methods of use, including rhuPH20, are described in US 2005/0260186 and US 2006/0104968. In one aspect, a sHASEGP is combined with one or more additional glycosaminoglycanases such as chondroitinases.

Exemplary lyophilized antibody formulations are described in US 6,267,958. Aqueous antibody formulations include those described in US 6,171,586 and WO 2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methyl methacrylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences, 16th edition, Osol, A. (ed.) (1980).

Sustained-release preparations may be prepared. Suitable examples of sustained-release preparations include semi-permeable matrices of solid hydrophobic polymers containing the circular fusion polypeptide, which matrices are in the form of shaped articles, e.g. films, or microcapsules.

The formulations to be used for *in vivo* administration are generally sterile. Sterility may be readily accomplished, e.g., by filtration through sterile filtration membranes.

### XI. Therapeutic Methods and Compositions

Any of the circular fusion polypeptides provided herein may be used in therapeutic methods.

In one aspect, a circular fusion polypeptide for use as a medicament is provided. In further aspects, a circular fusion polypeptide for use in treating a disease is provided. In certain embodiments, a circular fusion polypeptide for use in a method of treatment is provided. In certain embodiments, the invention provides a circular fusion polypeptide for use in a method of treating an individual having a disease comprising administering to the individual an effective amount of the circular fusion polypeptide. In one such embodiment, the method further comprises administering to the individual an effective amount of at least one additional therapeutic agent. An "individual" according to any of the above embodiments is preferably a human.

In a further aspect, the invention provides for the use of a circular fusion polypeptide in the manufacture or preparation of a medicament. In one embodiment, the medicament is for treatment of a disease. In a further embodiment, the medicament is for use in a method of treating a disease comprising administering to an individual having said disease an effective amount of the medicament. In one such embodiment, the method further comprises administering to the individual an effective amount of at least one additional therapeutic agent. An "individual" according to any of the above embodiments may be a human.

In a further aspect, the invention provides a method for treating a disease. In one embodiment, the method comprises administering to an individual having such disease an effective amount of a circular fusion polypeptide. In one such embodiment, the method further comprises administering to the individual an effective amount of at least one additional therapeutic agent. An "individual" according to any of the above embodiments may be a human.

In a further aspect, the invention provides pharmaceutical formulations comprising any of the circular fusion polypeptides provided herein, e.g., for use in any of the above therapeutic methods. In one embodiment, a pharmaceutical formulation comprises any of the circular fusion polypeptides provided herein and a pharmaceutically acceptable carrier. In another embodiment, a pharmaceutical formulation comprises any of the circular fusion polypeptides provided herein and at least one additional therapeutic agent.

Circular fusion polypeptides as reported herein can be used either alone or in combination with other agents in a therapy. For instance, a circular fusion polypeptide of the invention may be co-administered with at least one additional therapeutic agent.

Such combination therapies noted above encompass combined administration (where two or more therapeutic agents are included in the same or separate formulations), and separate administration, in which case, administration of the circular fusion polypeptide of the invention can occur prior to, simultaneously, and/or following, administration of the additional therapeutic agent or agents. In one embodiment, administration of the circular fusion polypeptide and administration of an additional therapeutic agent occur within about one month, or within about one, two or three weeks, or within about one, two, three, four, five, or six days, of each other. Suited circular fusion polypeptides of the invention can also be used in combination with radiation therapy.

A circular fusion polypeptide of the invention (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Circular fusion polypeptides of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The circular fusion polypeptide need not be, but is optionally formulated with one or more agents currently used to prevent or treat the disorder in question. The effective amount of such other agents depends on the amount of circular fusion polypeptide present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a circular fusion polypeptide of the invention (when used alone or in combination with one or more other additional therapeutic agents) will depend on the type of disease to be treated, the type of circular fusion polypeptide, the severity and course of the disease, whether the circular fusion polypeptide is administered for preventive or therapeutic purposes, previous therapy, the patient's clinical history and response to the circular fusion polypeptide, and the discretion of the attending physician. The circular fusion polypeptide is suitably administered to the patient at one time or over a series of treatments. Depending on the type and severity of the disease, about 1 µg/kg to 15 mg/kg (e.g. 0.5mg/kg - 10 mg/kg) of circular fusion polypeptide can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the circular fusion polypeptide would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or *e.g.* about six doses of the circular fusion polypeptide). An initial higher loading dose, followed by one or more lower doses may be administered. However, other dosage regimens may be useful. The progress of this therapy is easily monitored by conventional techniques and assays.

It is understood that any of the above formulations or therapeutic methods may be carried out using an immunoconjugate of the invention in place of or in addition to a circular fusion polypeptide.

### XII. Articles of Manufacture

In another aspect of the invention, an article of manufacture containing materials useful for the treatment, prevention and/or diagnosis of a disorder is provided. The article of manufacture comprises a container and a label or package insert on or associated with the container. Suitable containers include, for example, bottles, vials, syringes, IV solution bags, etc. The containers may be formed from a variety of materials such as glass or plastic. The container holds a composition which is by itself or combined with another composition effective for treating, preventing and/or diagnosing the condition and may have a sterile access port (for example the container may be an intravenous solution bag or a vial having a stopper pierceable by a hypodermic injection needle). At least one active agent in the composition is a circular fusion polypeptide of the invention. The label or package insert indicates that the composition is used for treating the condition of choice. Moreover, the article of manufacture may comprise (a) a first container with a composition contained therein, wherein the composition comprises a circular fusion polypeptide of the invention; and (b) a second container with a composition contained therein, wherein the composition comprises a further cytotoxic or otherwise therapeutic agent. The article of manufacture in this embodiment of the invention may further comprise a package insert indicating that the compositions can be used to treat a particular condition. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

All documents cited herein (scientific, book or patents) are incorporated by reference.

The following examples and figures are provided to aid the understanding of the present invention, the true scope of which is set forth in the appended claims. It is understood that modifications can be made in the procedures set forth without departing from the spirit of the invention.

### Description of the Figures

- **Figure 1**: Scheme of the general structure of the circular fusion polypeptide as reported herein.

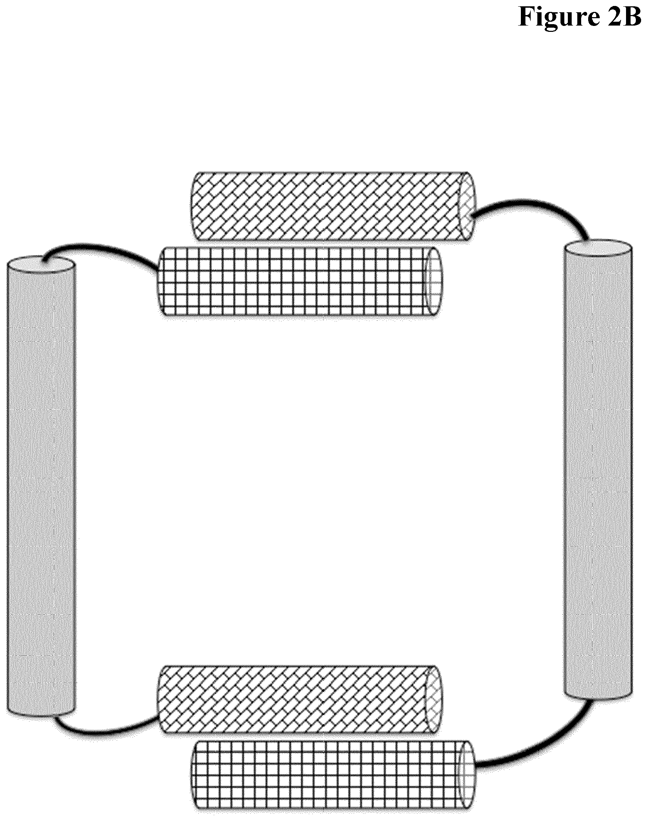

- **Figure 2**: Schemes of the general structure of dicircular fusion polypeptides as reported herein.

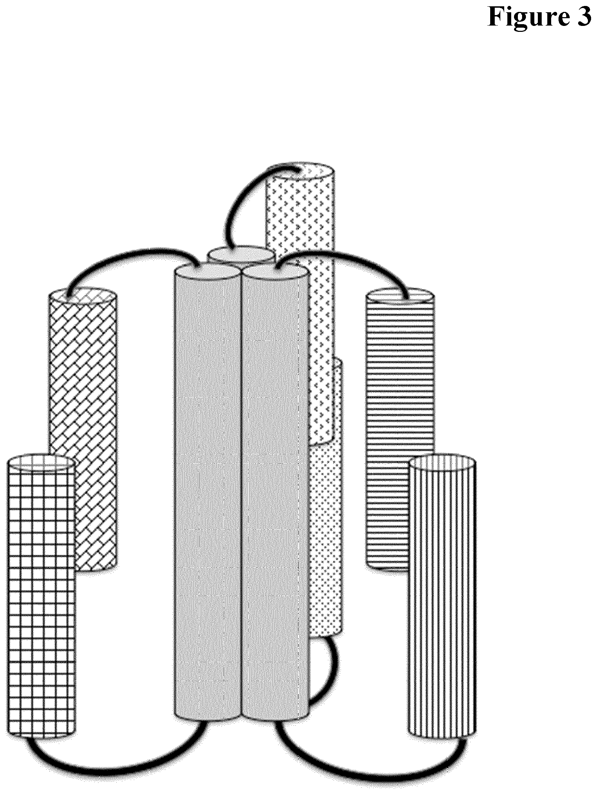

- **Figure 3**: Scheme of the general structure of tricircular fusion polypeptides as reported herein.

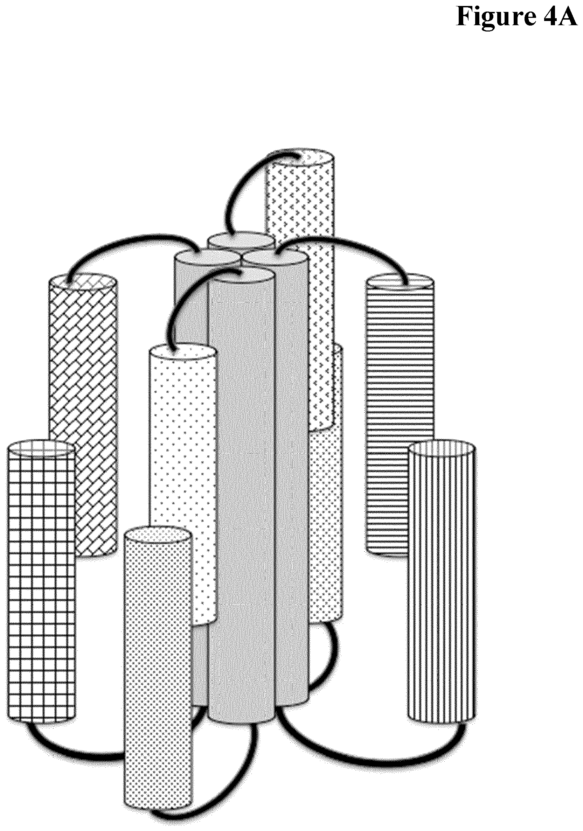

- **Figure 4**: Scheme of the general structure of tetracircular fusion polypeptides as reported herein.
- **Figure 5**: Orientation and spatial distance between the binding sites of a normal antibody of the IgG type and an exemplary dicircular fusion polypeptides as reported herein.
- **Figure 6**: Product quality analysis of the circular fusion polypeptide as reported herein by mass spectrometry.
- **Figure 7**: Differential effects of trastuzumab and the dicircular and the tetracircular anti-Her2 Contorsbody, respectively, with respect to proliferation.
- **Figure 8**: Binding of an exemplary dicircular fusion polypeptide as reported herein to the FcRn.
- **Figure 9**: ADCC kinetic of the anti-Her2 Contorsbodies and trastuzumab.
- **Figures 10+11**: Scheme of the relative positions and orientations of the VH domain in the "VH-in" orientation (close to the Fc-region) and the "VH-out" orientation (more apart from the Fc-region).
- **Figure 12**: Exemplary chains of anti-cMET circular fusion polypeptides with the respective orientation used for the production of bispecific bicircular fusion polypeptides as reported herein.
- **Figure 13**: Dicircular fusion polypeptide as reported herein wherein one circular fusion polypeptide comprises a VH/VL-pair as binding site and the other circular fusion polypeptide comprises a peptide-loaded MHC-I complex as binding site.
- **Figure 14**: Effect based on MHC-I mediated killer cell recruiting and cell removal of an MHC-I IgG-type antibody fusion and the bicircular fusion polypeptide as reported herein and shown in Figure 13.
- **Figure 15**: UV extinction chromatogram (280 nm) of the different Contorsbody forms.

### XIII. EXAMPLES

The following are examples of methods and compositions of the invention. It is understood that various other embodiments may be practiced, given the general description provided above.

### Materials and Methods

### Recombinant DNA techniques

Standard methods were used to manipulate DNA as described in Sambrook, J. et al., Molecular cloning: A laboratory manual; Cold Spring Harbor Laboratory Press, Cold Spring Harbor, New York, 1989. The molecular biological reagents were used according to the manufacturer's instructions.

### Gene and oligonucleotide synthesis

Desired gene segments were prepared by chemical synthesis at Geneart GmbH (Regensburg, Germany). The synthesized gene fragments were cloned into an E. coli plasmid for propagation/amplification. The DNA sequences of subcloned gene fragments were verified by DNA sequencing. Alternatively, short synthetic DNA fragments were assembled by annealing chemically synthesized oligonucleotides or via PCR. The respective oligonucleotides were prepared by metabion GmbH (Planegg-Martinsried, Germany).

### Reagents

All commercial chemicals, antibodies and kits were used as provided according to the manufacturer's protocol if not stated otherwise.

### Example 1

### Construction of the expression plasmids for the circular fusion polypeptides

For the expression of a circular fusion polypeptide as reported herein a transcription unit comprising the following functional elements was used:
- the immediate early enhancer and promoter from the human cytomegalovirus (P-CMV) including intron A,
- a human heavy chain immunoglobulin 5'-untranslated region (5'UTR),
- a murine immunoglobulin heavy chain signal sequence,
- a nucleic acid encoding the respective circular fusion polypeptide, and
- the bovine growth hormone polyadenylation sequence (BGH pA).

Beside the expression unit/cassette including the desired gene to be expressed the basic/standard mammalian expression plasmid contains
- an origin of replication from the vector pUC18 which allows replication of this plasmid in E. coli, and
- a beta-lactamase gene which confers ampicillin resistance in E. coli.

### Example 2

### Expression of the circular fusion polypeptides

Transient expression of circular fusion polypeptides was performed in suspension-adapted HEK293F (FreeStyle 293-F cells; Invitrogen) cells with Transfection Reagent 293-free (Novagen).

Cells have been passaged, by dilution, at least four times (volume 30 ml) after thawing in a 125 ml shake flask (Incubate/Shake at 37 °C, 7 % CO₂, 85 % humidity, 135 rpm).

The cells were expanded to 3x10⁵ cells/ml in 250 ml volume. Three days later, cells have been split and new seeded with a density of 7*10⁵ cells/ml in a 250 ml volume in a 1 liter shake flask. Transfection will be 24 hours later at a cell density around 1.4 - 2.0x10⁶ cells/ml.

Before transfection 250 µg plasmid-DNA were diluted in a final volume of 10 ml with pre-heated (water bath; 37 °C) Opti-MEM (Gibco). The solution was gently mixed and incubated at room temperature for not longer than 5 min. Then 333.3 µl 293-free transfection reagent were added to the DNA-OptiMEM-solution. Thereafter the solution was gently mixed and incubated at room temperature for 15-20 minutes. The whole volume of mixture was added to 1 L shake flask with 250 ml HEK-cell-culture-volume.

Incubate/Shake at 37 °C, 7 % CO₂, 85 % humidity, 135 rpm for 6 or 7 days.

The supernatant was harvested by a first centrifugation-step at 2,000 rpm, 4 °C, for 10 minutes. Then the supernatant was transferred into a new centrifugation-flask for a second centrifuge at 4,000 rpm, 4 °C, for 20 minutes. Thereafter the cell-free-supernatant was filtered through a 0.22 µm bottle-top-filter and stored in a freezer (-20 °C).

### Example 3

### Purification of the circular fusion polypeptides

The antibody-containing culture supernatants were filtered and purified by two chromatographic steps. The antibodies were captured by affinity chromatography using HiTrap MabSelectSuRe (GE Healthcare) equilibrated with PBS (1 mM KH₂PO₄, 10 mM Na₂HPO₄, 137 mM NaCl, 2.7 mM KCl), pH 7.4. Unbound proteins were removed by washing with equilibration buffer, and the antibody was recovered with 50 mM citrate buffer, pH 2.8, and immediately after elution neutralized to pH 6.0 with 1 M Tris-base, pH 9.0. Size exclusion chromatography on Superdex 200™ (GE Healthcare) was used as second purification step. The size exclusion chromatography was performed in 20 mM histidine buffer, 0.14 M NaCl, pH 6.0. The antibody containing solutions were concentrated with an Ultrafree -CL centrifugal filter unit equipped with a Biomax-SK membrane (Millipore, Billerica, MA) and stored at -80 °C.

### Example 4

### Binding of the anti-Her2 circular fusion polypeptide

### Surface plasmon resonance Her2 receptor binding

- **Chip Surface:**: CM5-Chip
- **T:**: 37 °C and 25 °C, respectively for assay setting 1 and 2
- **running buffer:**: PBS + 0.05% (v/v) Tween 20
- **dilution buffer:**: running buffer + 0.1 % BSA
- **analytes:**: c(HER2 ECD) = 0.41-900 nM for assay setting 1;
c(dimeric anti-Her2 Contorsbodies) = 3.7-300 nM,
c(tetrameric anti-Her2 Contorsbodies) = 1.85-150 nM,
c(trastuzumab) = 3.7-300 nM for assay setting 2
- **Ligand:**: dimeric and tetrameric anti-Her2 Contorsbody, trastuzumab bound via anti-human Fc-region antibody for assay setting 1; Her2-ECD bound via pertuzumab for assay setting 2.

The response units are directly proportional to molecular weight. The theoretical maximum of analyte binding is based on the known binding level of Her2 ECD. 100 % = 1 molecule dimeric anti-Her2 Contorsbodies or trastuzumab binds to 2 molecules HER2 ECD, respectively; 1 molecule tetrameric anti-Her2 Contorsbodies binds to 4 molecules HER2 ECD.

### Example 5

### ADCC assay- ACEA

BT-474 cells were "solubilized" with Accutase, counted in the medium and brought to a cell density of 2x10E5 cells/ml. 50µl medium were pipetted in each well on a 96-wells plates, the background effect was measured on the ACEA, and, finally, 50µl cells suspension/well (=10,000 cells/well) were added. The plates were placed in the ACEA to measure the cell index after 15 minutes. The medium was then removed by pipetting, a washing step was made with AIM-V medium, and 50µl antibody in three different concentrations was added. Natural killer cells were counted and placed in AIM-V to a cell density of 6x10E5. 50 µl (30,000 cells/well ad E/T 3:1 were added in ACEA and the cell index was measured every 5 minutes. After 24 hours, the experiment was stopped and ADCC after 2 and 4 hours was calculated.

### Example 6

### Mass spectrometric analysis of the dimeric anti-Her2 circular fusion polypeptide

PNGase F was obtained from Roche Diagnostics GmbH (14.3 U / µl; solution in sodium phosphate, EDTA and glycerol). A protease specifically cleaving in the hinge region of an IgG antibody was freshly reconstituted from a lyophilisate prior to digestion.

### Enzymatic deglycosylation of with PNGase F

50 µg Contorsbody was diluted to a final concentration of 0.6 mg/ml with 10 mM sodium phosphate buffer, pH 7.1, and deglycosylated with 1µl PNGase F at 37°C for 16 hours.

### Enzymatic cleavage

The deglycosylated sample was diluted to a final concentration of 0.5 mg / ml with 200 mM Tris buffer, pH 8.0, and subsequently digested with the IgG specific protease at 37°C for 1 hour.

### ESI-QTOF mass spectrometry

The sample was desalted by HPLC on a Sephadex G25 column (Kronlab, 5x250mm, TAC05/250G0-SR) using 40% acetonitrile with 2% formic acid (v/v). The total mass was determined via ESI-QTOF MS on a maXis 4G UHR-QTOF MS system (Bruker Daltonik) equipped with a TriVersa NanoMate source (Advion). Calibration was performed with sodium iodide (Waters ToF G2-Sample Kit 2 Part: 700008892-1). For the digested Contorsbody, data acquisition was done at 1000-4000 m/z (ISCID: 30 eV). The raw mass spectra were evaluated and transformed into individual relative molar masses. For visualization of the results, a proprietary software was used to generate deconvoluted mass spectra.

## Claims

1. A fusion polypeptide specifically binding to a target comprising a first part of a binding domain, a second part of a binding domain and a spacer domain, wherein
- the spacer domain is a polypeptide and comprises at least 25 amino acid residues,
- the first part of the binding domain is a polypeptide that is fused either directly or via a first linker to the N-terminus of the spacer domain,
- the second part of the binding domain is a polypeptide that is fused either directly or via a second linker to the C-terminus of the spacer domain,
- the first part of the binding domain and the second part of the binding domain (of the same single chain fusion polypeptide) form a functional binding site that specifically binds to a target.

2. The fusion polypeptide according to claim 1, wherein the first part of the binding domain is an antibody heavy chain variable domain and the second part of the binding domain is an antibody light chain variable domain or vice versa.

3. The fusion polypeptide according to any one of claims 1 to 2, wherein the first part of the binding domain is an antibody heavy chain Fab fragment and the second part of the binding domain is an antibody light chain Fab fragment or vice versa.

4. The fusion polypeptide according to claim 1, wherein the fusion polypeptide is free of antibody variable domains.

5. The fusion polypeptide according to any one of claims 1 to 4, wherein the first part of the binding domain and the second part of the binding domain are associated covalently by a disulfide bond with each other.

6. The fusion polypeptide according to any one of claims 1 to 5, wherein the spacer domain comprises an antibody hinge region or a (C-terminal) fragment thereof and an antibody CH2 domain or a (N-terminal) fragment thereof.

7. The fusion polypeptide according to any one of claims 1 to 6, wherein the spacer domain comprises an antibody hinge region or a fragment thereof, an antibody CH2 domain, and an antibody CH3 domain or a fragment thereof.

8. The fusion polypeptide according to any one of claims 1 to 7, wherein the first and/or the second linker is/are a peptidic linker.

9. A dimeric fusion polypeptide comprising a first fusion polypeptide according to any one of claims 1 to 8 and a second fusion polypeptide according to any one of claims 1 to 8, wherein the first and the second fusion polypeptide are identical or different and wherein the spacer domain of the first fusion polypeptide is covalently conjugated to the spacer domain of the second fusion polypeptide.

10. An isolated nucleic acid encoding the fusion polypeptide according to any one of claims 1 to 8.

11. A pair of isolated nucleic acids together encoding the dimeric fusion polypeptide according to claim 9.

12. A host cell comprising the nucleic acid according to claim 10 or the pair of nucleic acids according to claim 11.

13. A method of producing a fusion polypeptide comprising culturing the host cell of claim 12 so that the fusion polypeptide or the dimeric fusion polypeptide is produced and recovering the fusion polypeptide or the dimeric fusion polypeptide from the cell or the cultivation medium.

14. An immunoconjugate comprising the fusion polypeptide according to any one of claims 1 to 8 and a cytotoxic agent.

15. A pharmaceutical formulation comprising the fusion polypeptide according to any one of claims 1 to 8 or the dimeric fusion polypeptide according to claim 9 and a pharmaceutically acceptable carrier.

16. The fusion polypeptide according to any one of claims 1 to 8 or the dimeric fusion polypeptide according to claim 9 for use as a medicament.

17. Use of the fusion polypeptide according to any one of claims 1 to 8 or the dimeric fusion polypeptide according to claim 9 in the manufacture of a medicament.
